# EUROPEAN PATENT APPLICATION

(11) **EP 1 571 215 A2**
(43) Date of publication of application: **07.09.2005**
(21) Application number: 04026579.5
(22) Date of filing: 01.07.1998
(51) Int. Cl.: C12N 15/54, C12N 9/12, A61K 38/45, C07K 16/40, C12Q 1/68, C12Q 1/48, C12N 15/11, A61K 31/70, A01K 67/027

(54) **Vertebrate telomerase genes and proteins and uses thereof**

(30) Priority: 01.07.1997 US 51410 P; 21.07.1997 US 53018 P; 21.07.1997 US 53329 P; 04.08.1997 US 54642 P; 09.09.1997 US 58287 P
(62) Divisional of application: 98933117.8
(71) Applicant: Cambia Biosystems LLC, Bellevue, WA 98005 (US); PETER MacCALLUM CANCER INSTITUTE, Melbourne, Victoria 8006 (AU)
(72) Inventor: Kilian, Andrzej, Canberra, ACT 2602 (AU); Bowtell, David, Coburg, VIC 3058 (AU)
(74) Representative: Walker, Ross Thomson

(57) **Abstract**

Nucleic acid molecules encoding vertebrate telomerase are provided. Gene products, expression vectors and host cells suitable for expressing telomerase are also provided. Methods identifying inhibitors of telomerase activity and inhibitor compositions are disclosed.

## Description

### TECHNICAL FIELD

This invention relates generally to telomerases, and particularly to the human telomerase gene and protein and uses for diagnostics and therapy.

### BACKGROUND OF THE INVENTION

Non-circular chromosomes require a specialized mechanism for maintaining chromosome ends after each cell division because the polymerases responsible for replication of chromosomal DNA are unable to fully replicate linear DNA molecules, creating an "end replicating problem." To meet this challenge, eukaryotic cells depend upon an enzyme, telomerase, to add short, typically G-rich, relatively conserved repeats onto chromosomal ends. These repeat structures are termed telomeres.

The presence of telomeres is essential for cell viability. The absence of even a single telomere leads to cell cycle arrest in yeast, a eukaryotic cell (Sandell and Zakian, *Cell 75*:729, 1993). Telomeres shorten during replication; telomerase restores the telomeres. Thus, as expected, telomerase activity is primarily detected in actively dividing cells. As such, telomerase activity is constitutive in unicellular organisms and is regulated in more complex organisms, relatively abundant in germline and embryonic tissues and cells as well as tumor cells. In contrast, telomerase activity is difficult to detect in normal somatic human tissues. Moreover, rather than cessation of replication resulting in decreased telomerase, recent data indicate that telomerase inhibition might be one of the critical events in this transition. The seemingly direct correlation of telomerase/replication activities have prompted much speculation that inhibitors of telomerase could be a "universal" cancer therapeutic, effective for essentially all tumor types, whereas stimulators of telomerase could overcome the observed natural senescence of normal cells.

Spurred by these models, characterization of telomerase for culmination in isolation and cloning of telomerase has been a high priority. The mechanism of telomere elongation has been shown to center on the G-rich strand of the telomeric repeats. This G-rich strand, which extends to the 3' end of the chromosome, is extended by telomerase, a ribonucleoprotein, from the RNA component, which acts as a template. Various components of this complex have been isolated and cloned. The RNA component of the complex has been isolated and cloned from many different organisms, including humans (Feng et al. *Science 269*: 1236, 1995), mice and other mammalian species, *Saccharomyces cerevisiae, Tetrahymena, Euplotes,* and *Oxytricha* (see, Singer and Gottschling, *Science, 266*: 404, 1994; Lingner et al. *Genes & Develop. 8:* 1984, 1994; and Romero and Blackburn, *Cell 67*: 343, 1994). Protein components have been relatively refractory to isolation. Recently, the nucleotide sequences of several protein components have been determined (an 80 kD/95 kD dimeric protein from *Tetrahymena,* WO 96/19580; and a 67 kD protein from humans, WO 97/08314).

The present invention discloses nucleotide and amino acid sequences of telomerase, uses of these sequences for diagnostics and therapeutic uses, and further provides other related advantages.

### SUMMARY OF THE INVENTION

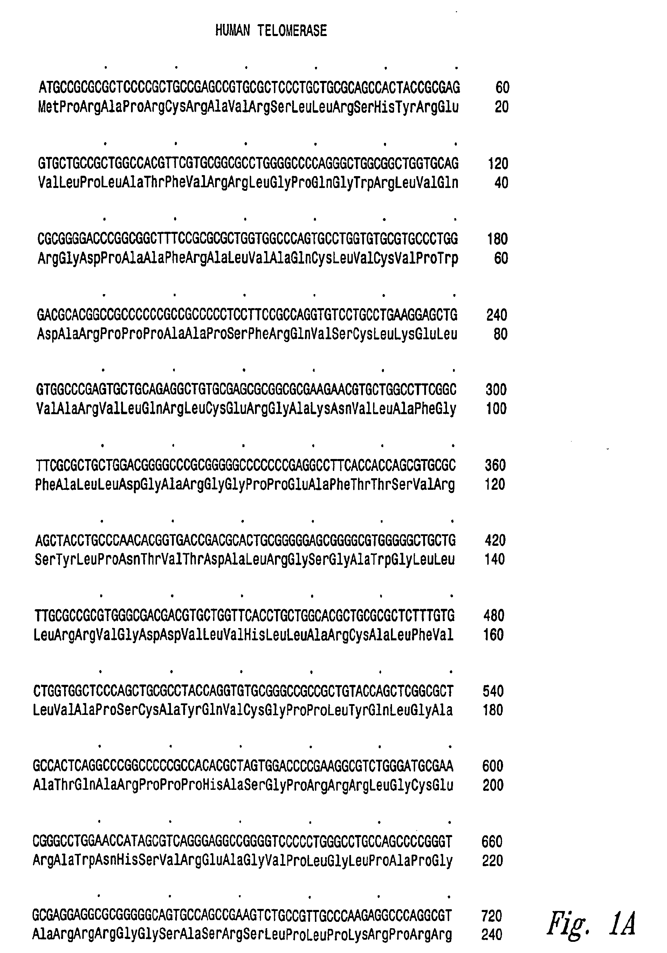

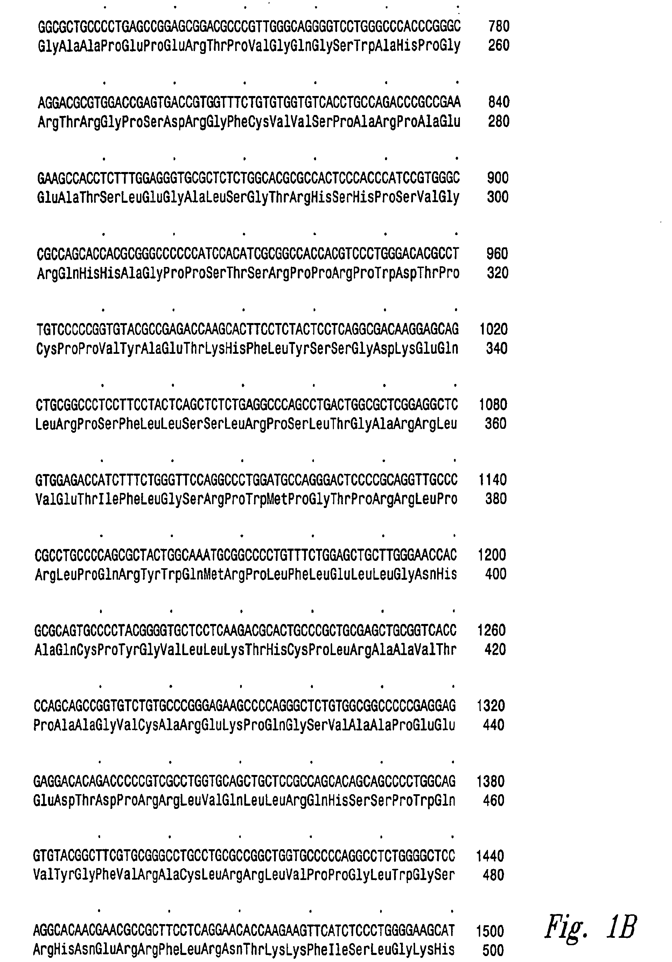

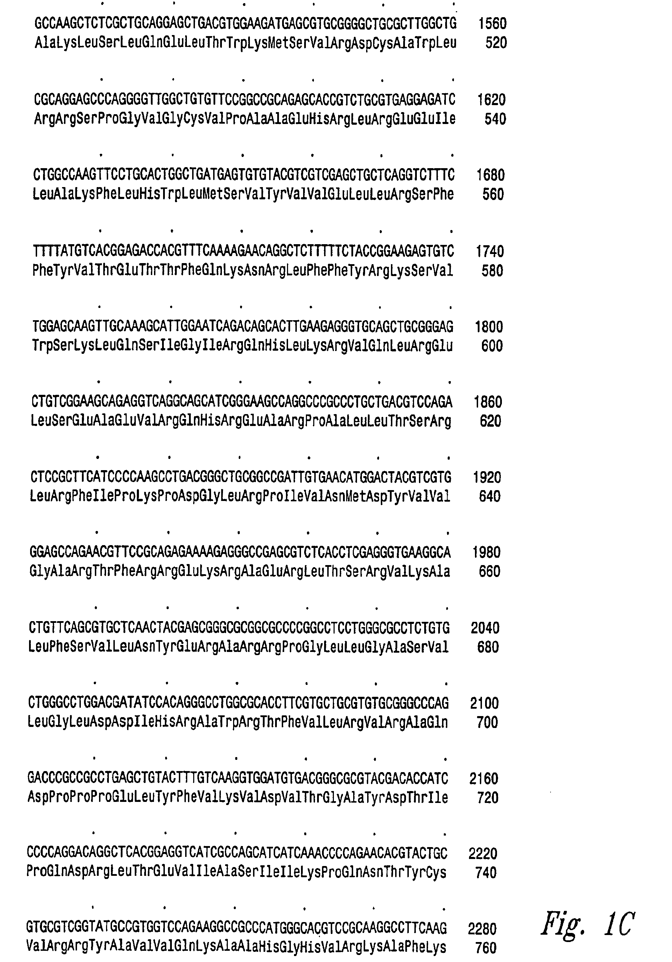

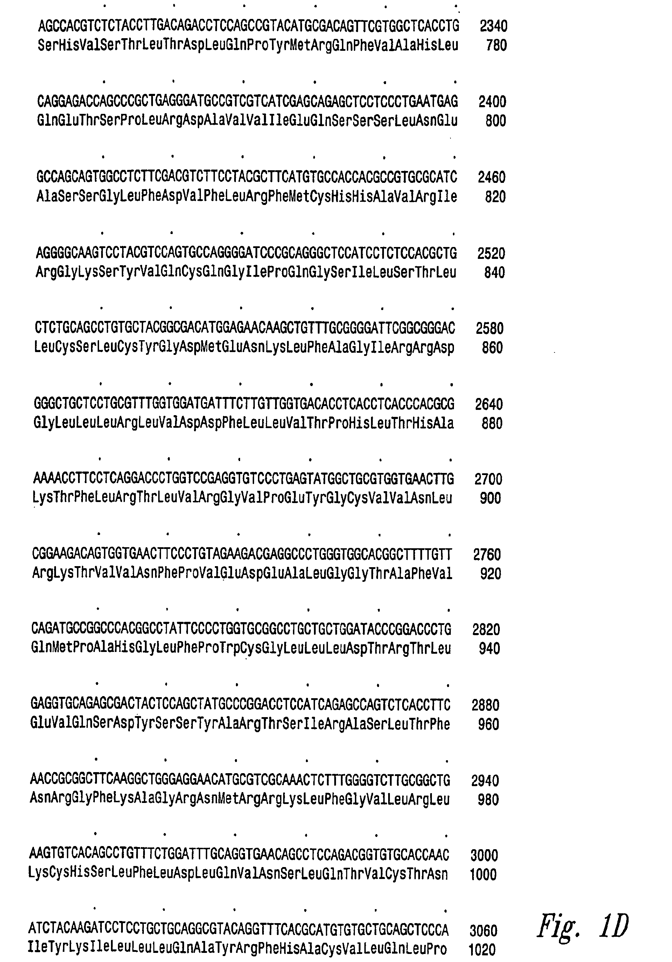

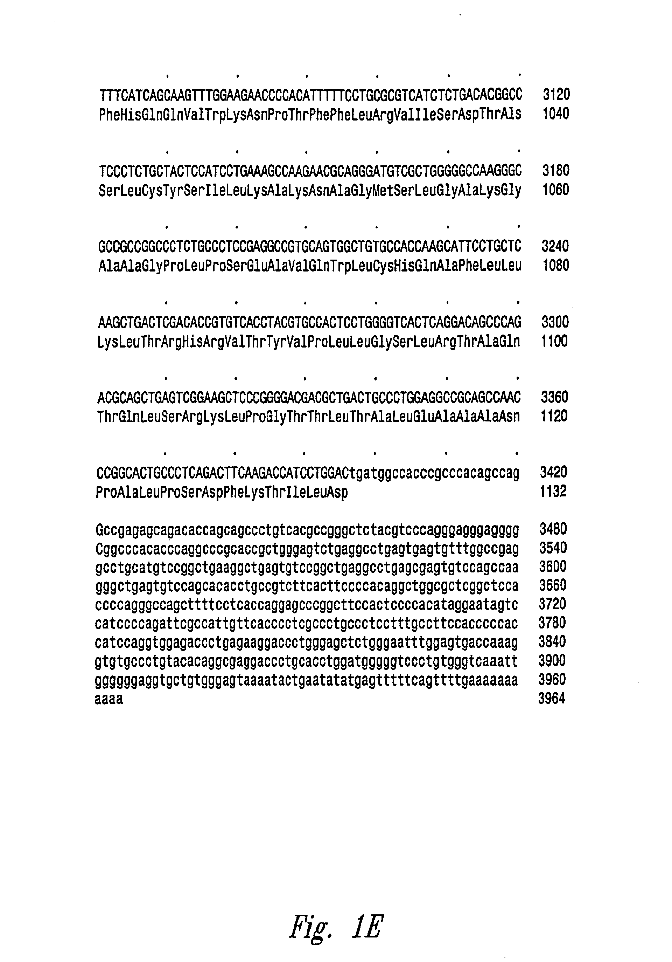

In one aspect, this invention generally provides isolated nucleic acid molecules encoding vertebrate telomerase (including variants thereof). Representative examples of vertebrates include mammals such as humans, old world monkeys (*e.g.*, macaques, chimps, and baboons), dogs, rats, and mice, as well as non-mammalian organisms such as birds. In a preferred embodiment, the nucleic acid molecule encoding a vertebrate telomerase is provided, wherein the nucleic acid molecule comprises the sequence presented in Figure 1, or hybridizes under stringent conditions to the complement of the sequence presented in Figure 1, provided that the nucleic acid molecule is not EST AA281296.

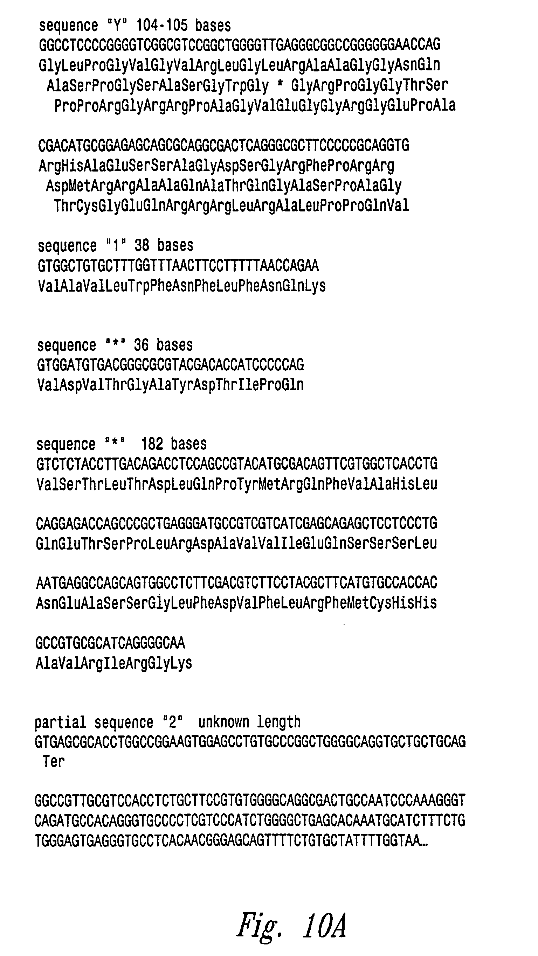

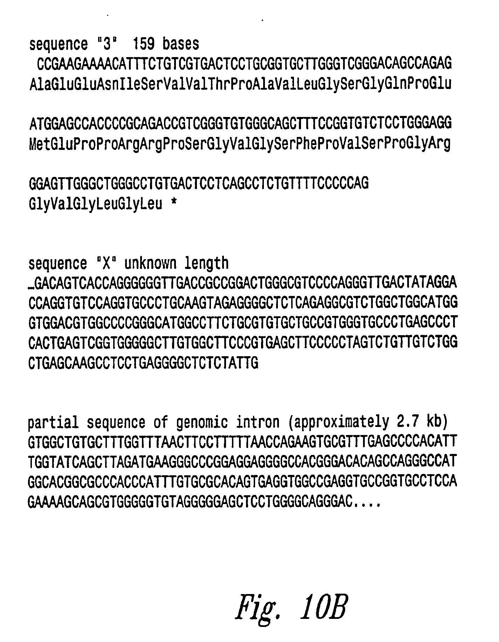

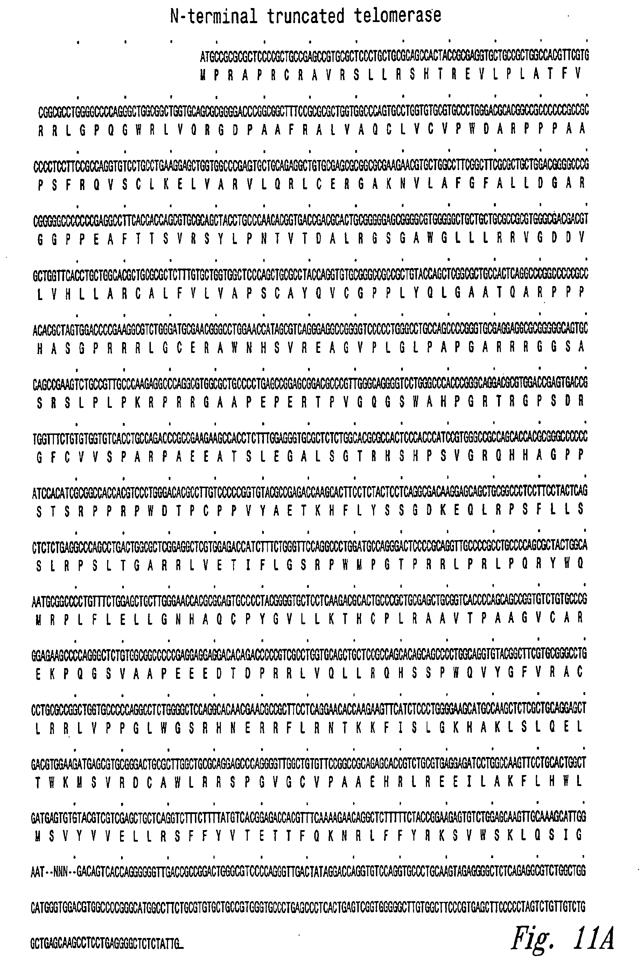

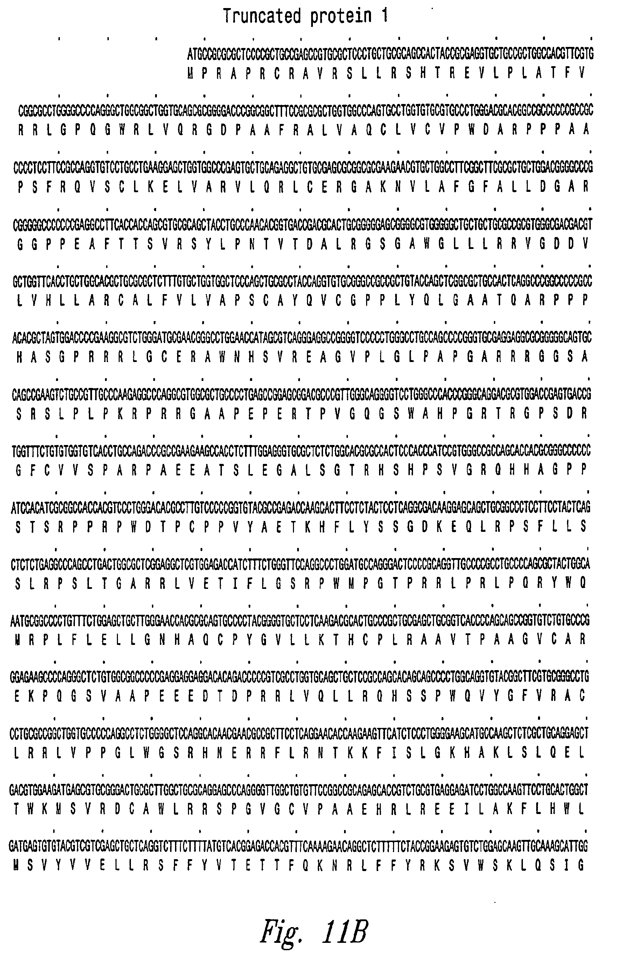

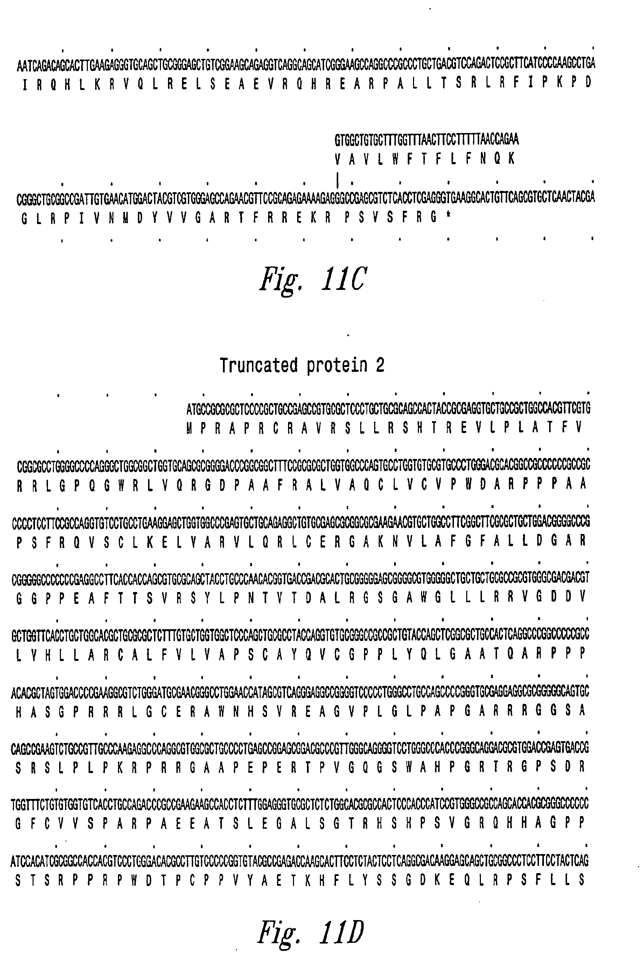

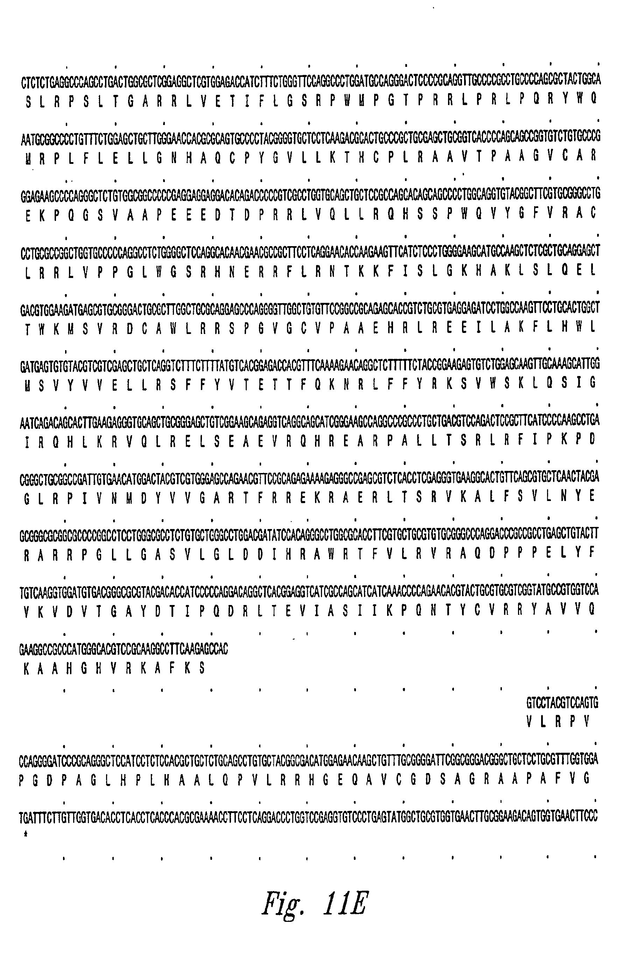

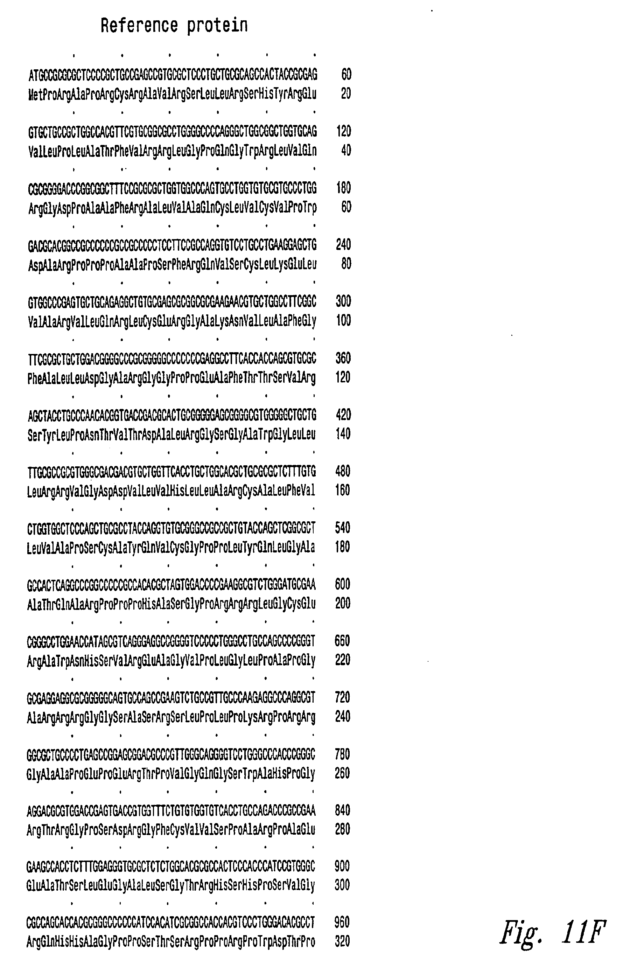

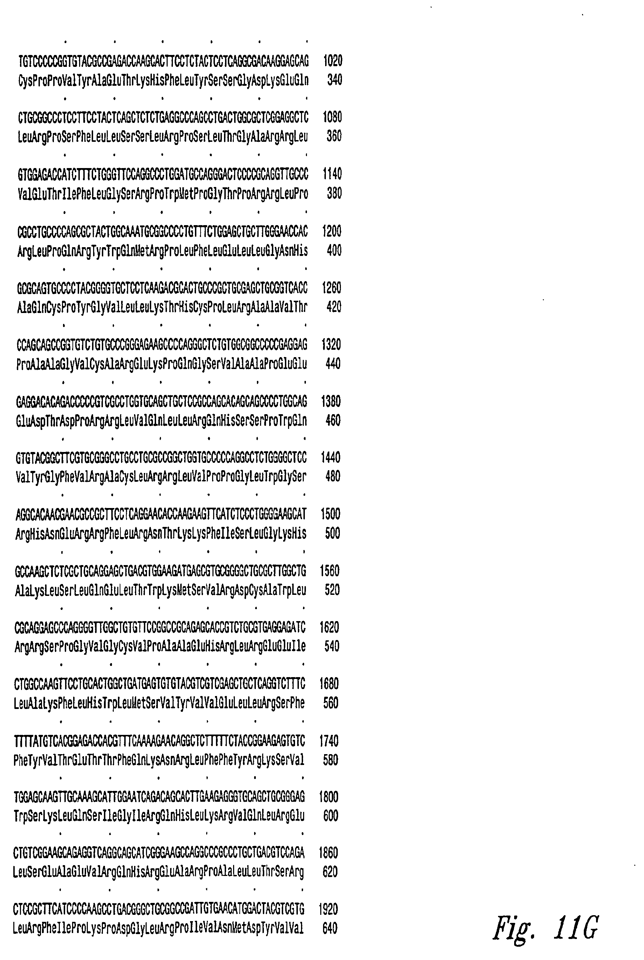

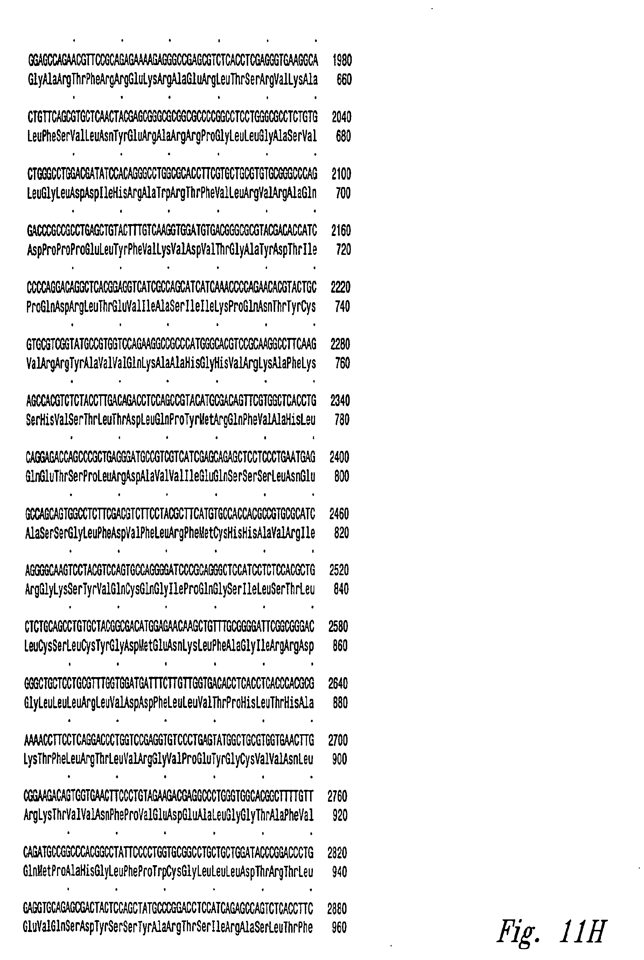

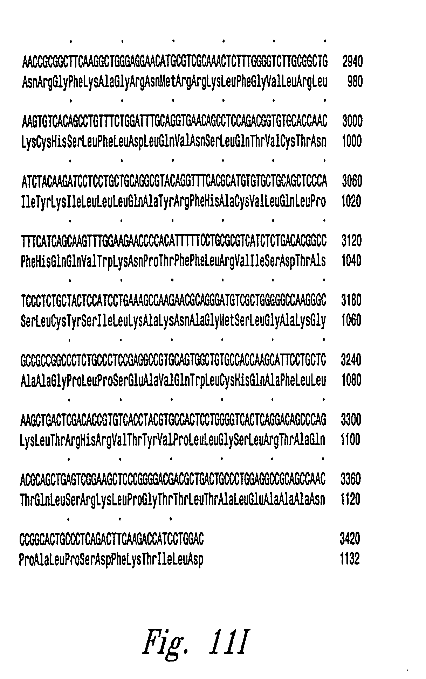

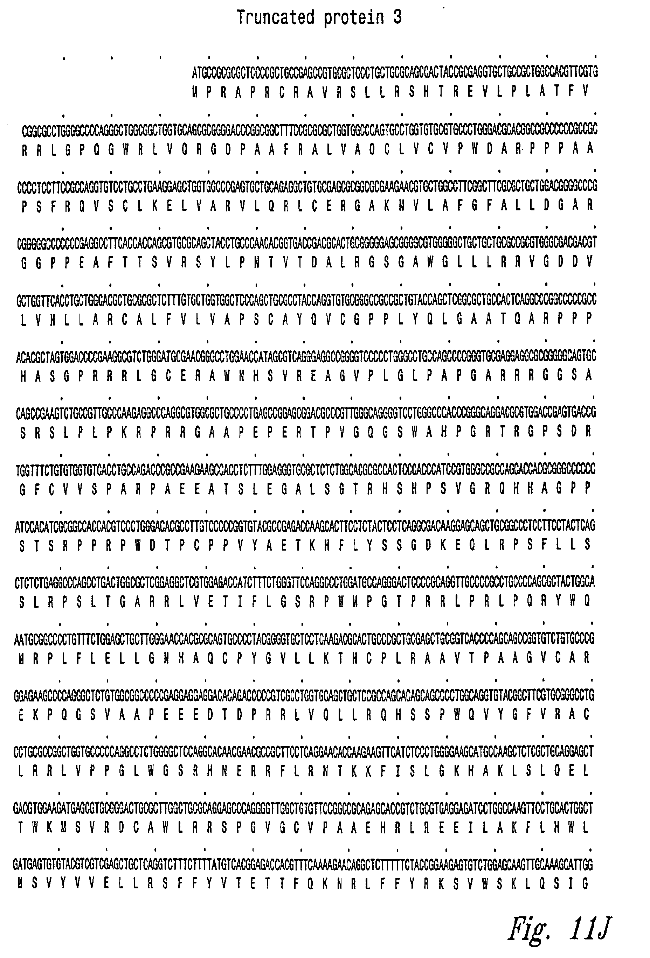

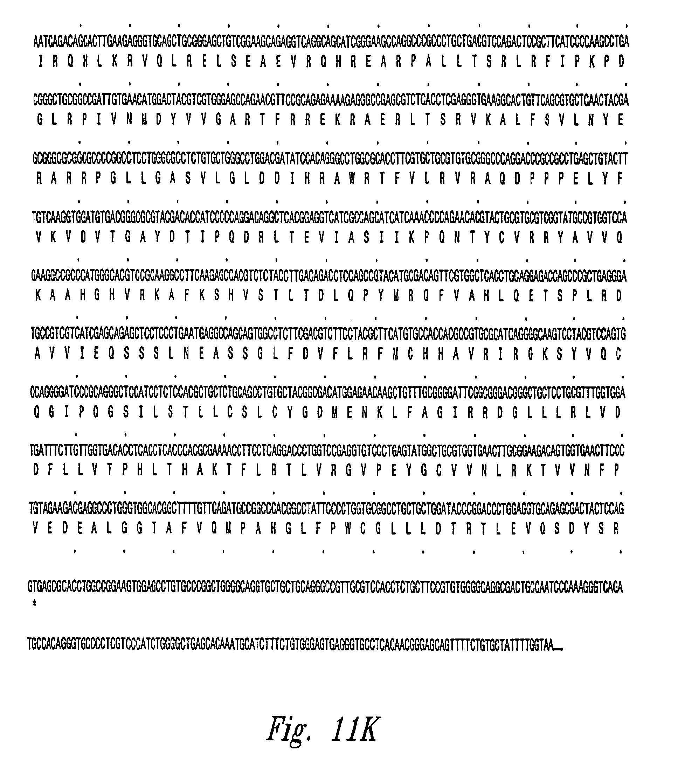

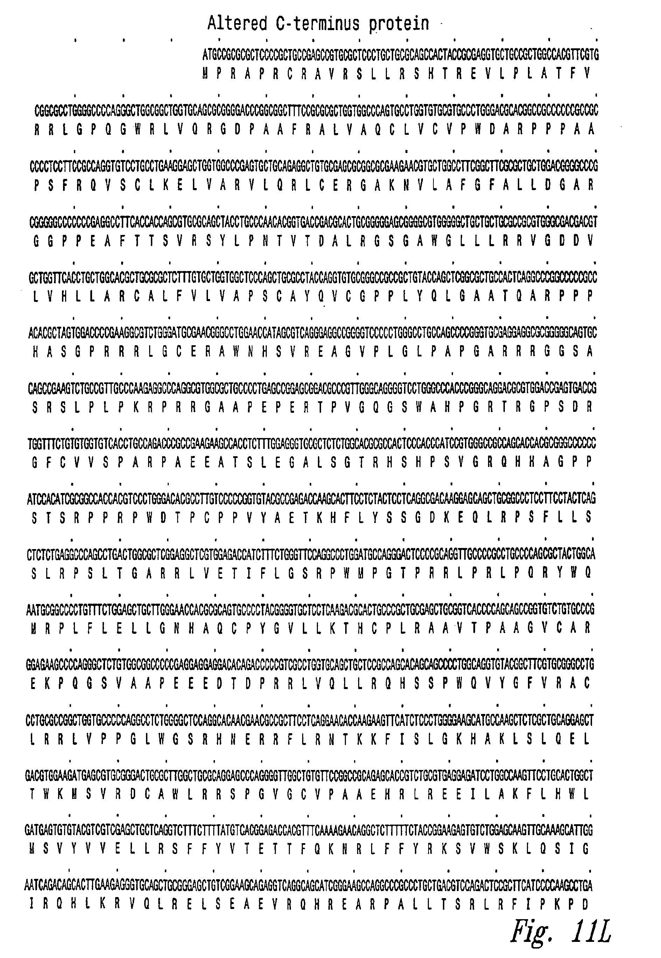

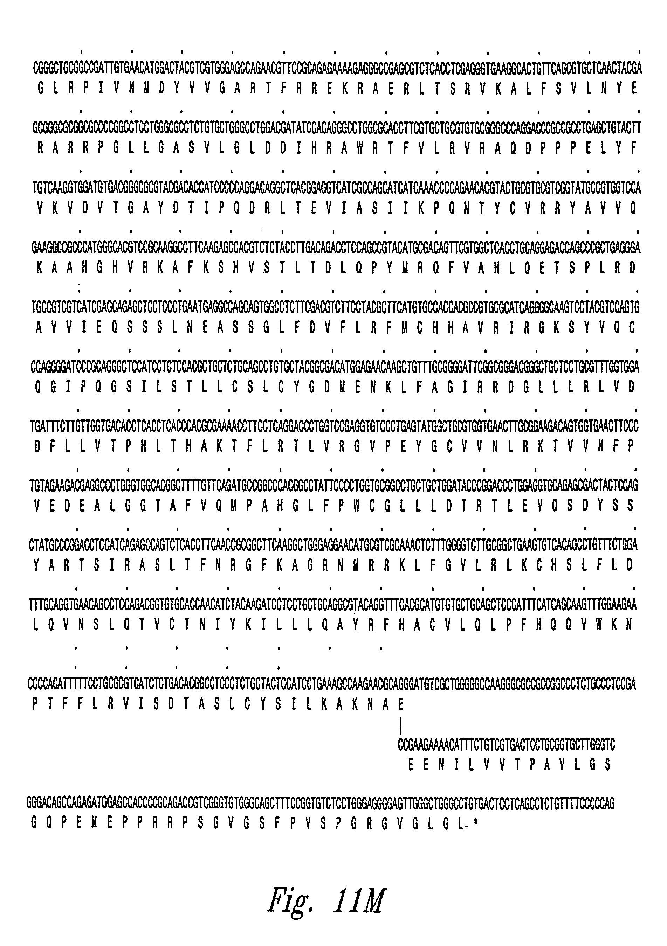

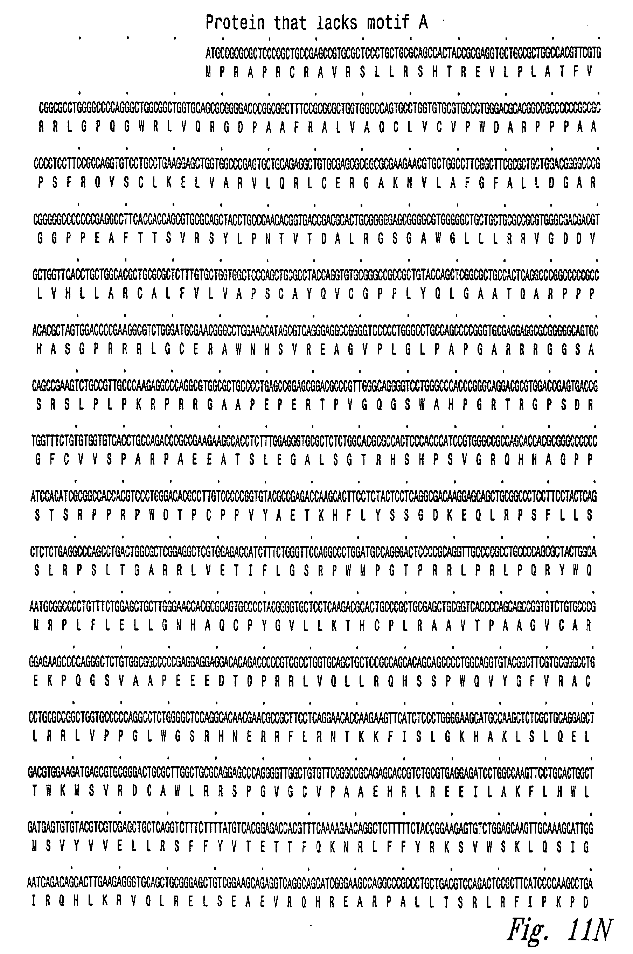

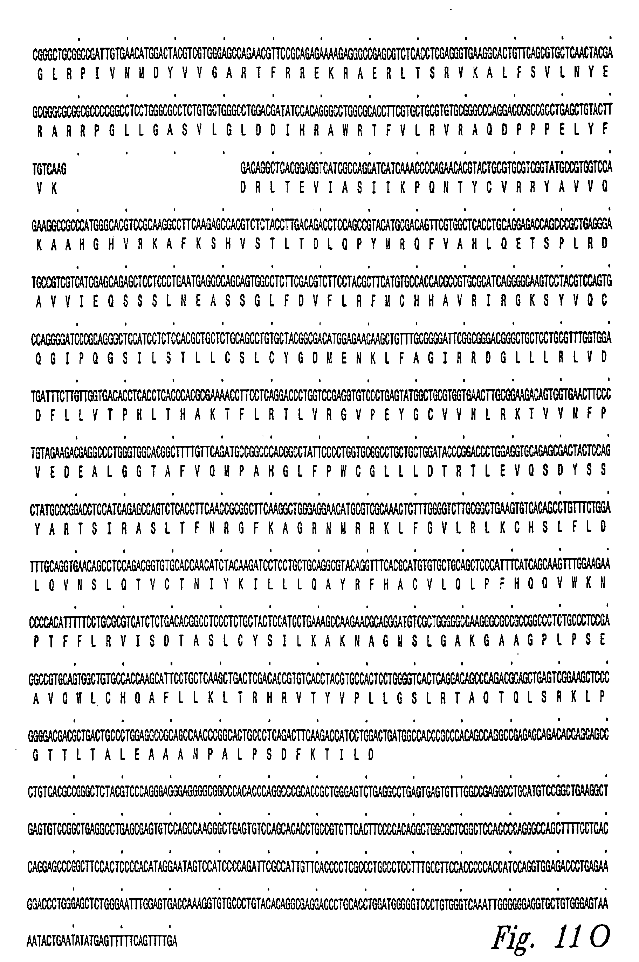

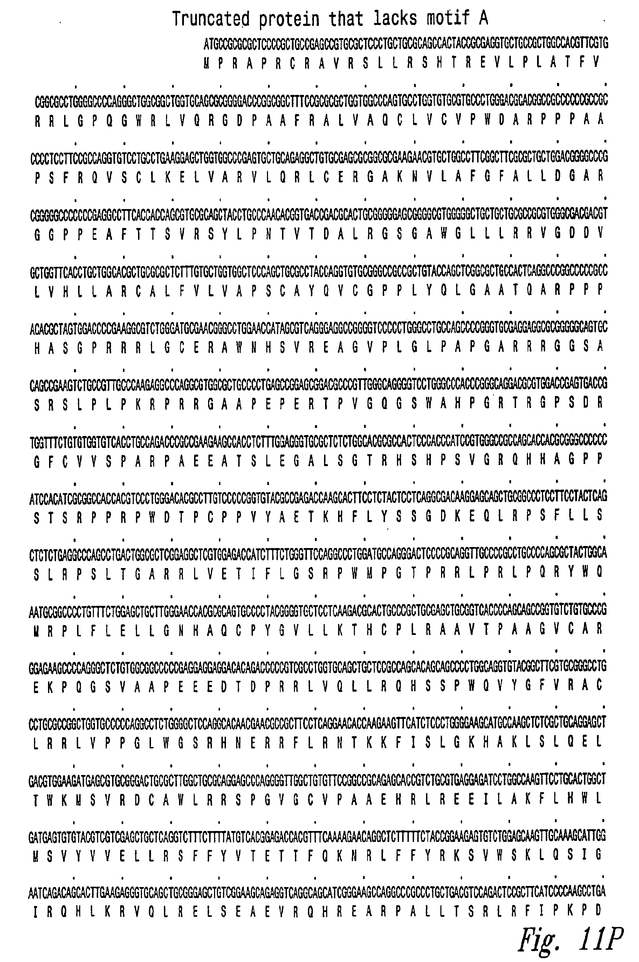

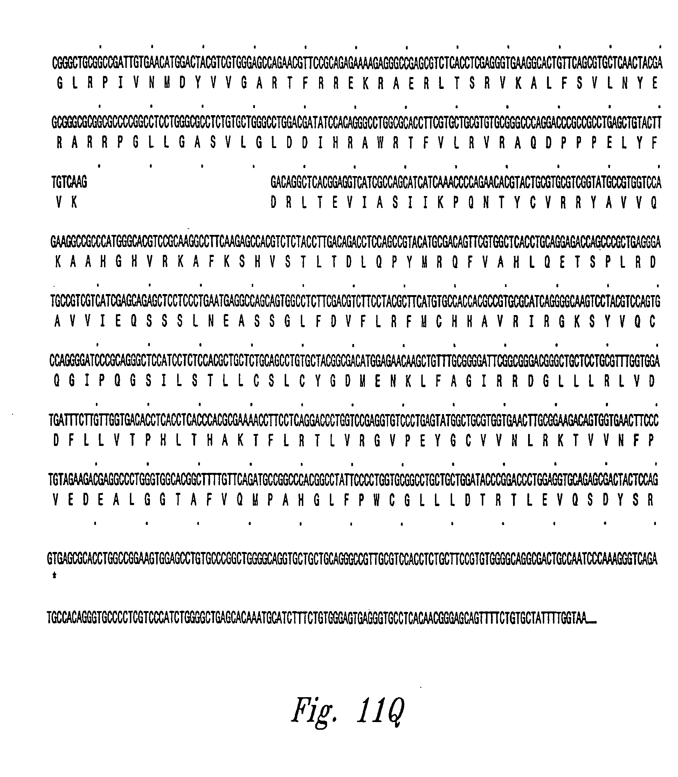

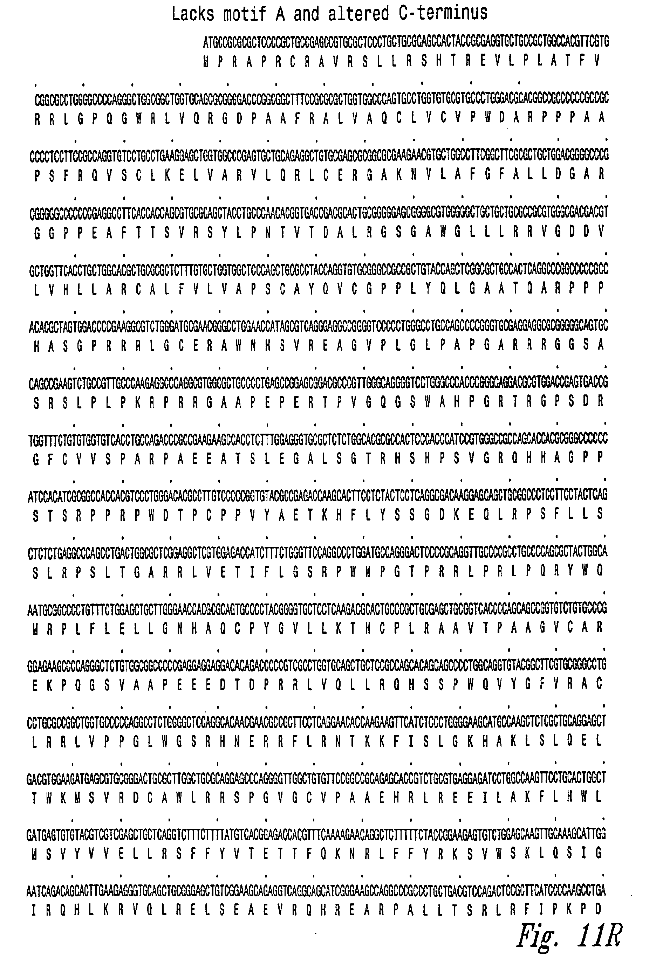

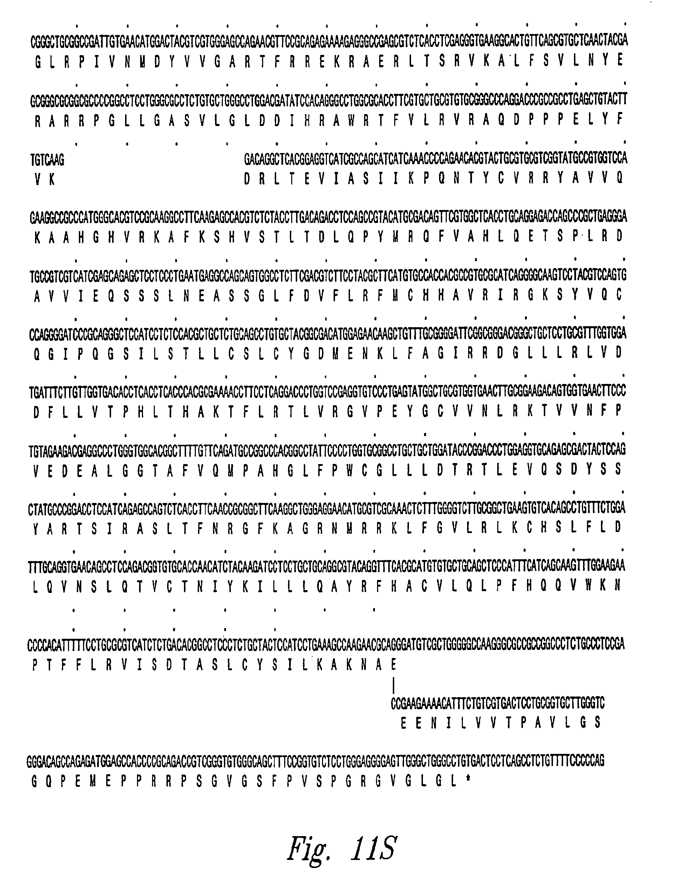

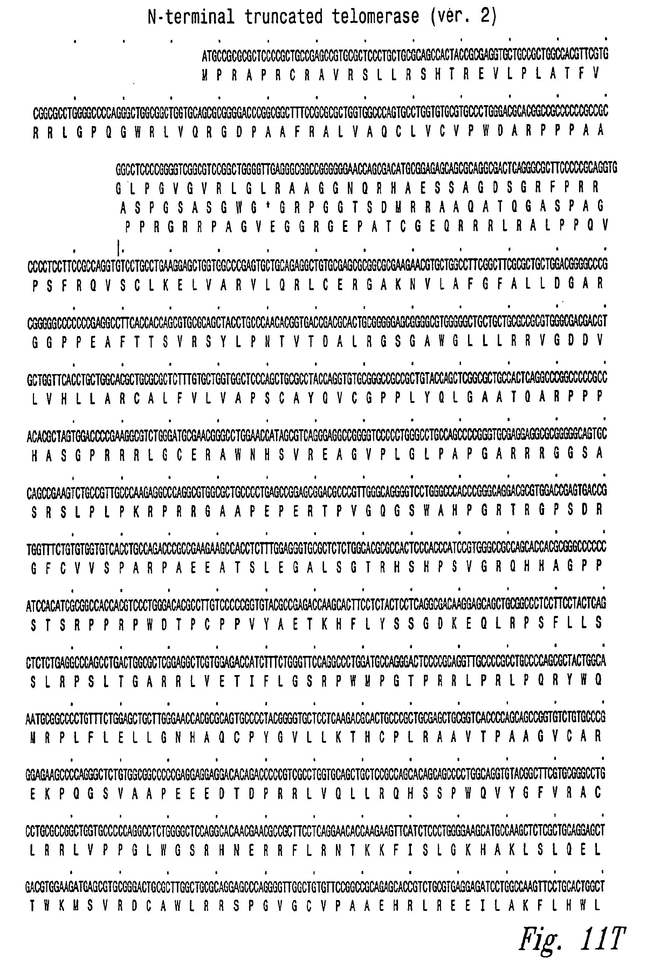

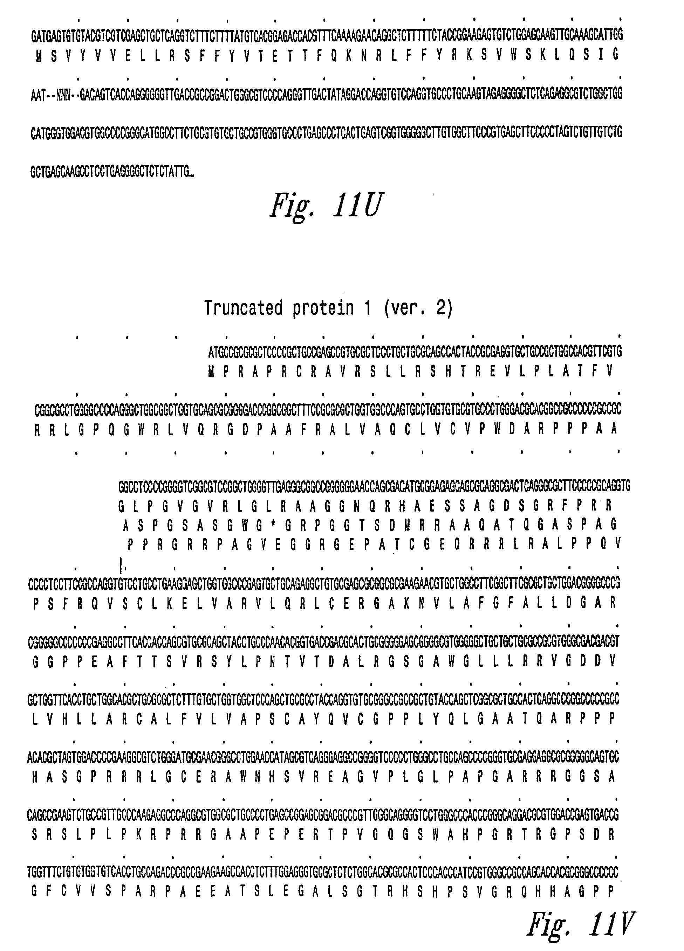

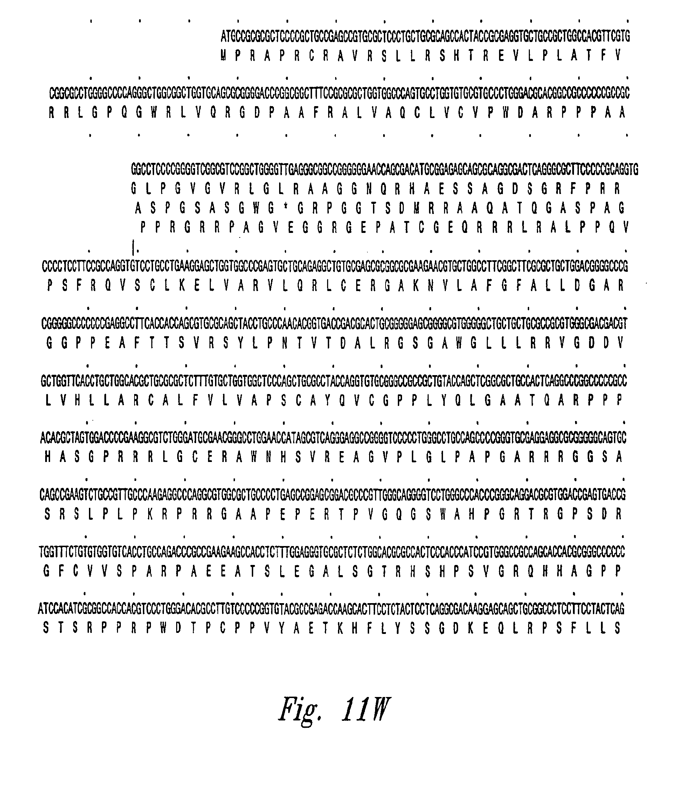

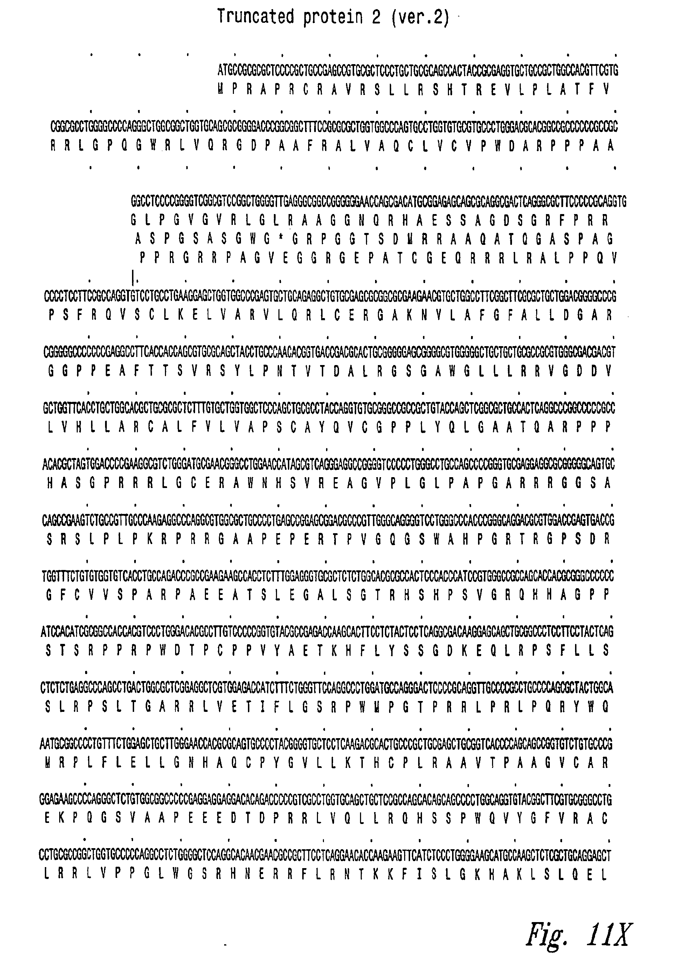

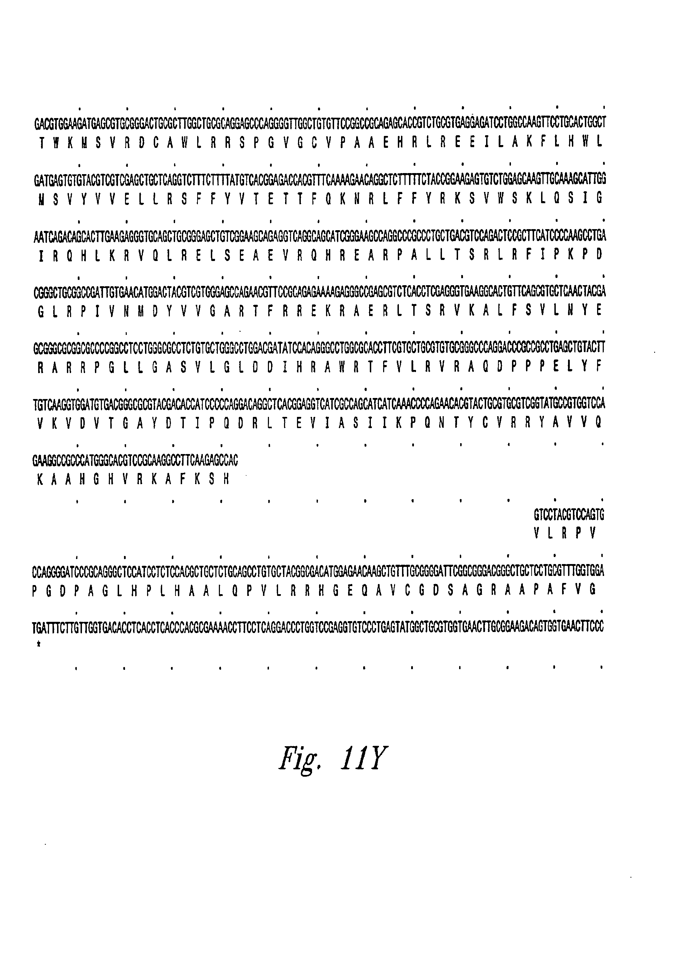

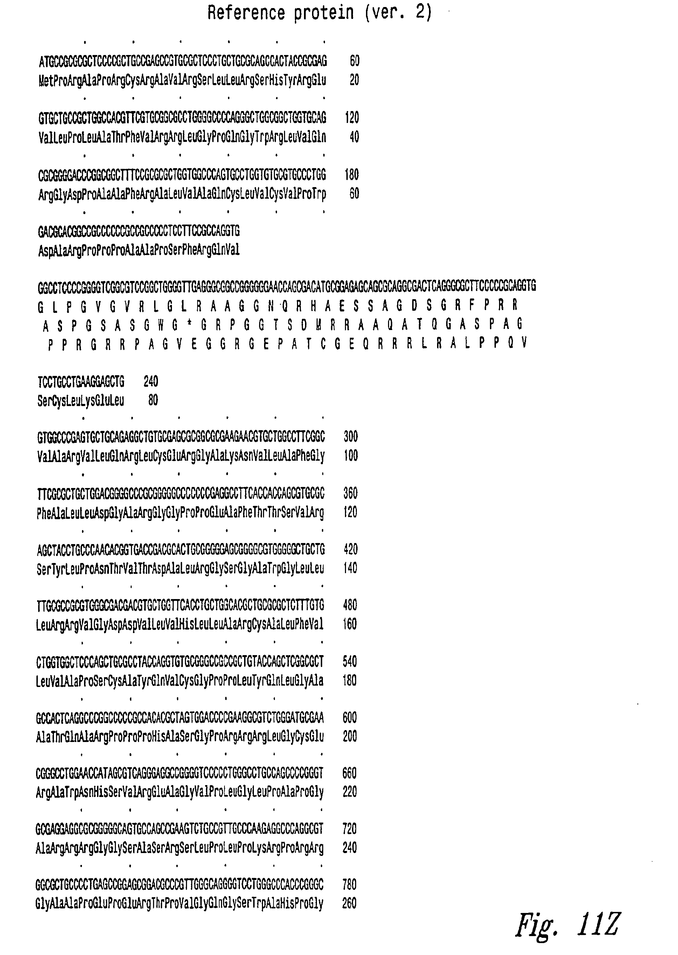

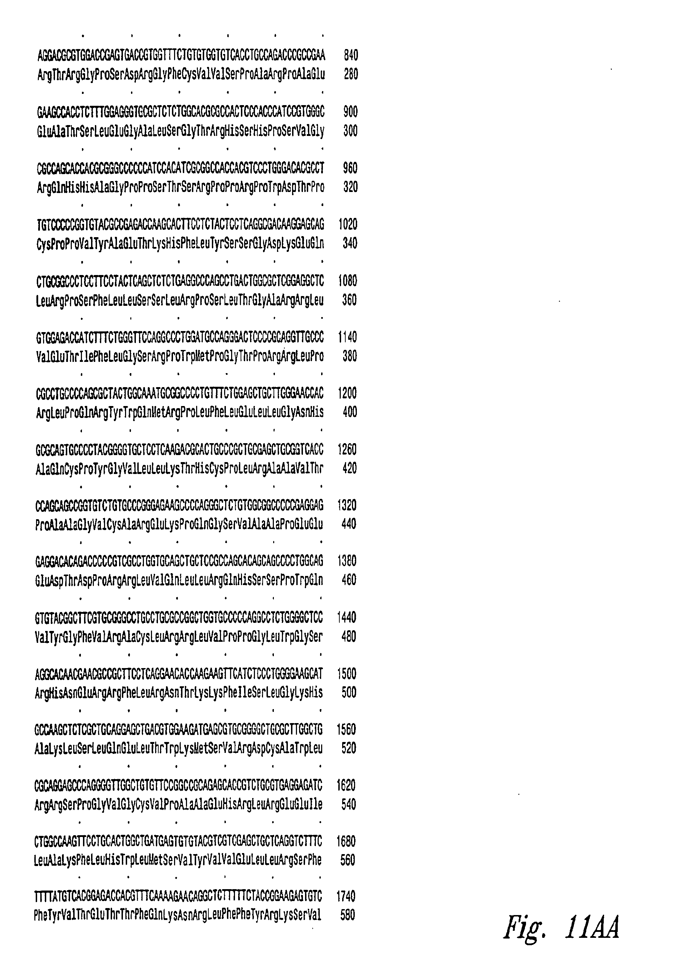

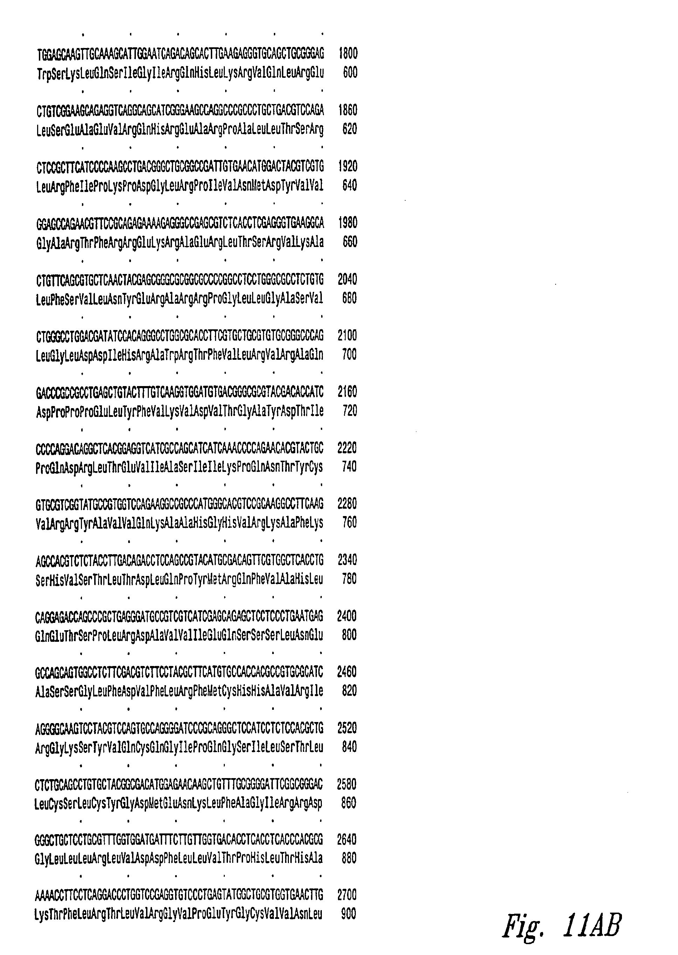

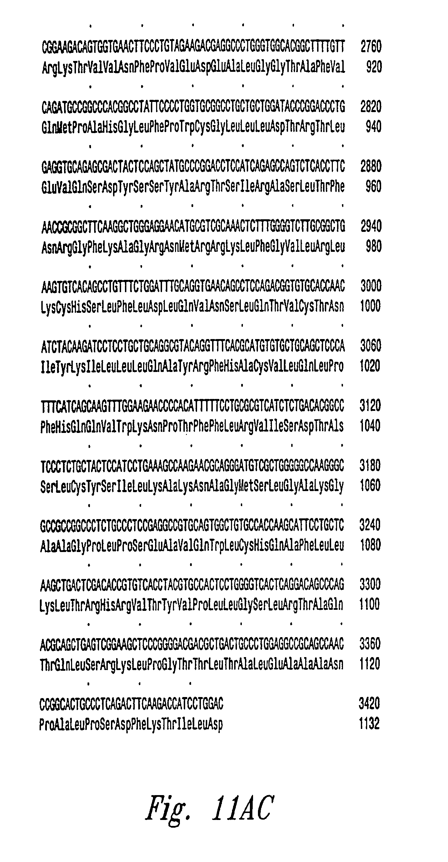

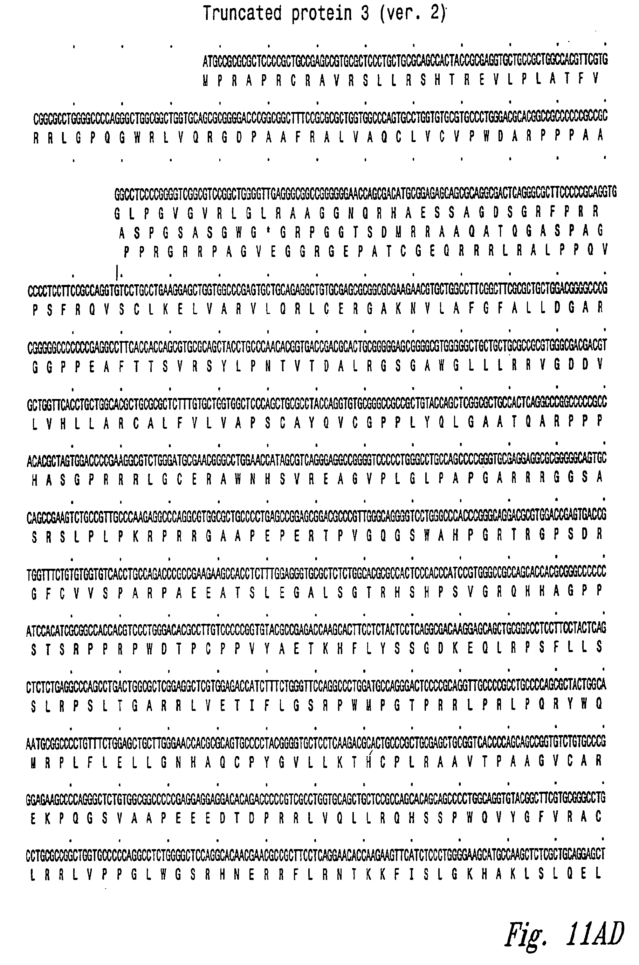

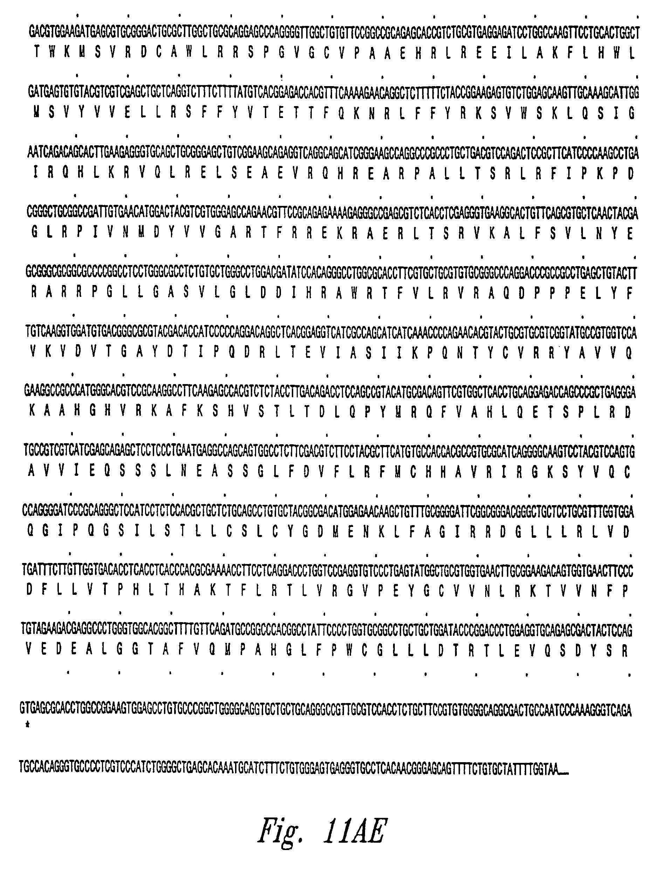

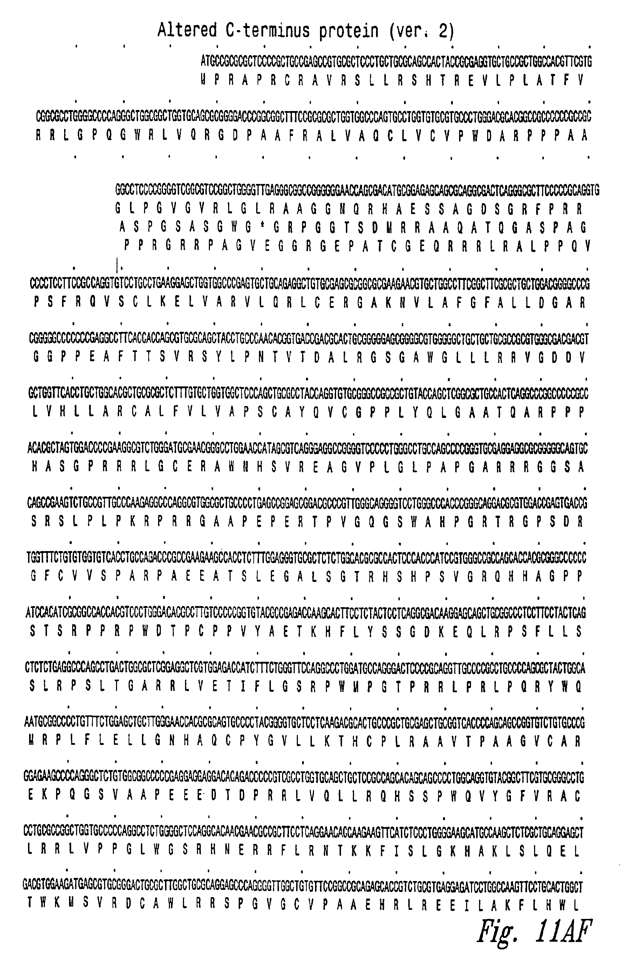

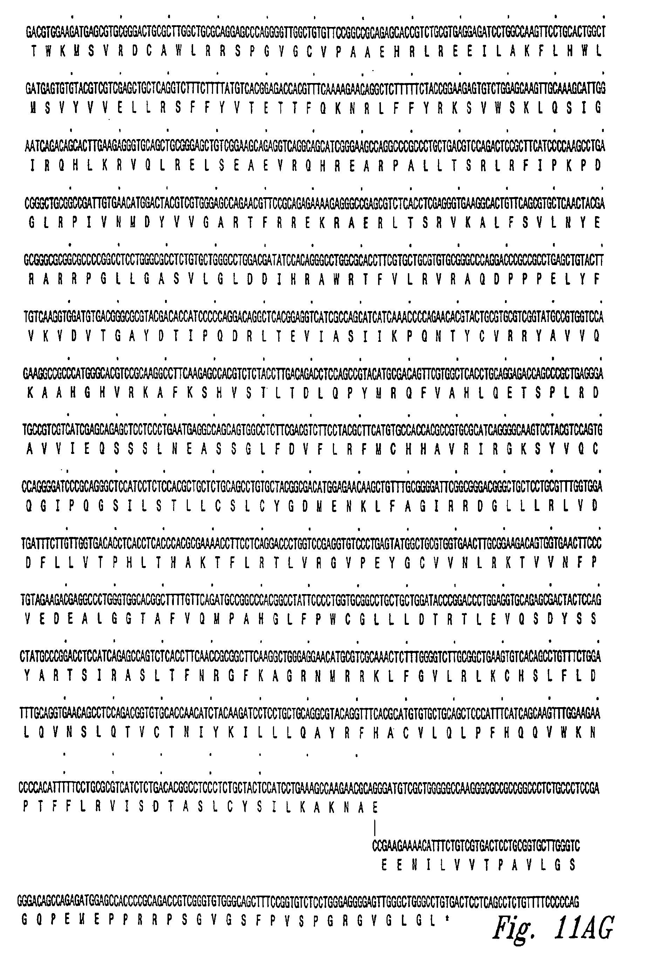

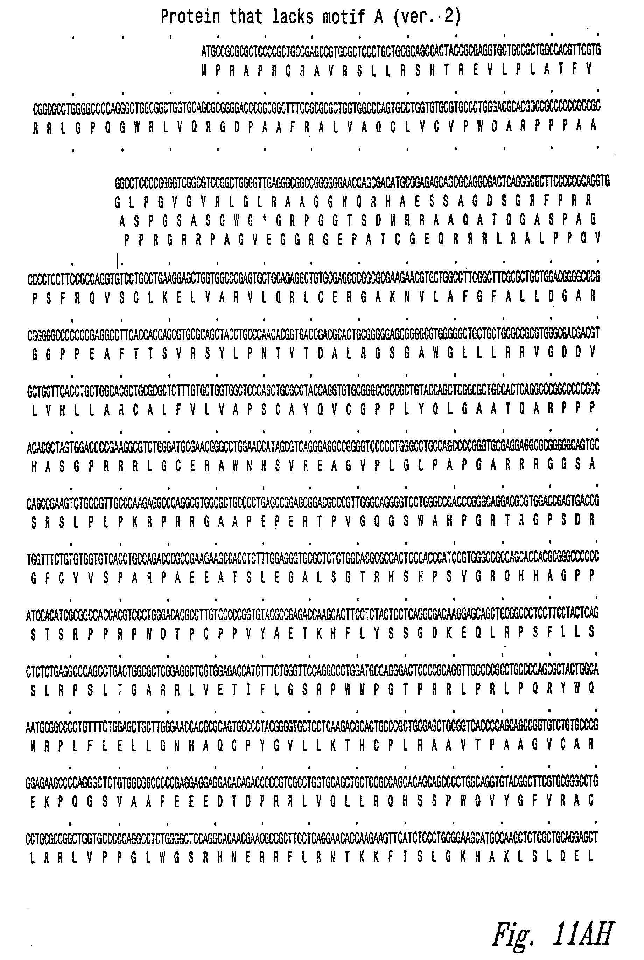

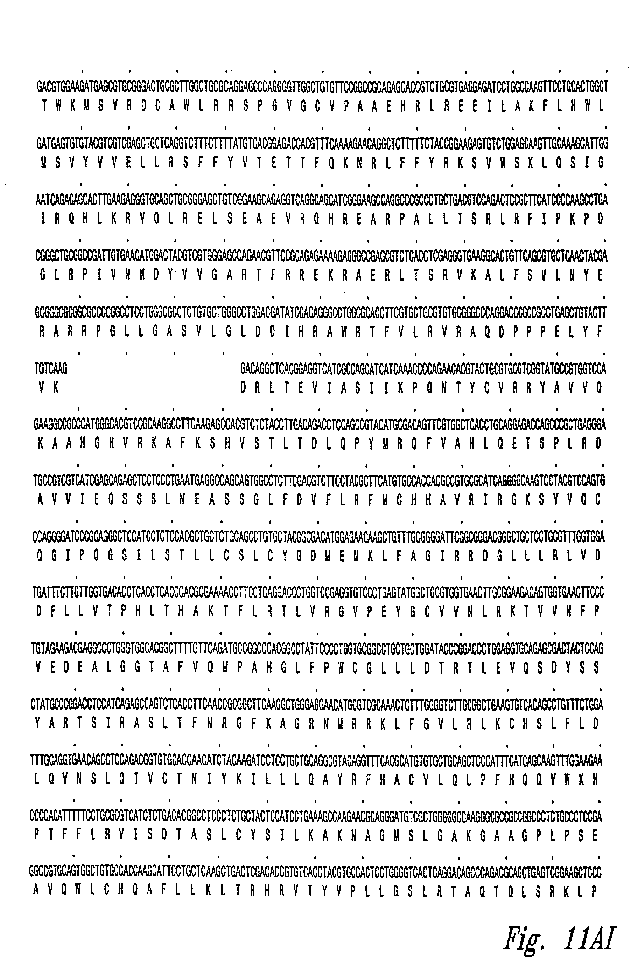

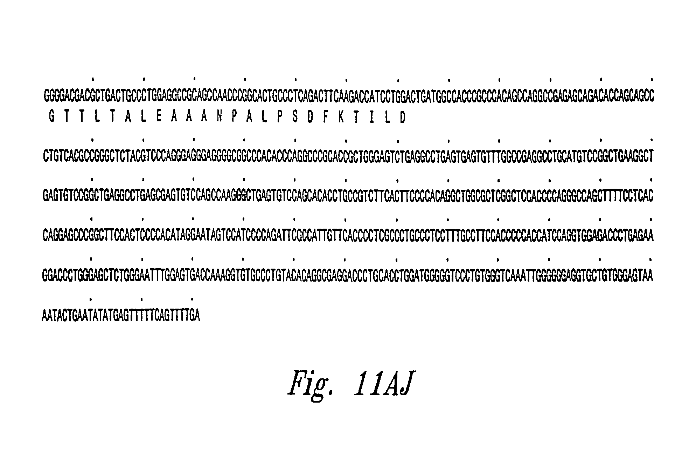

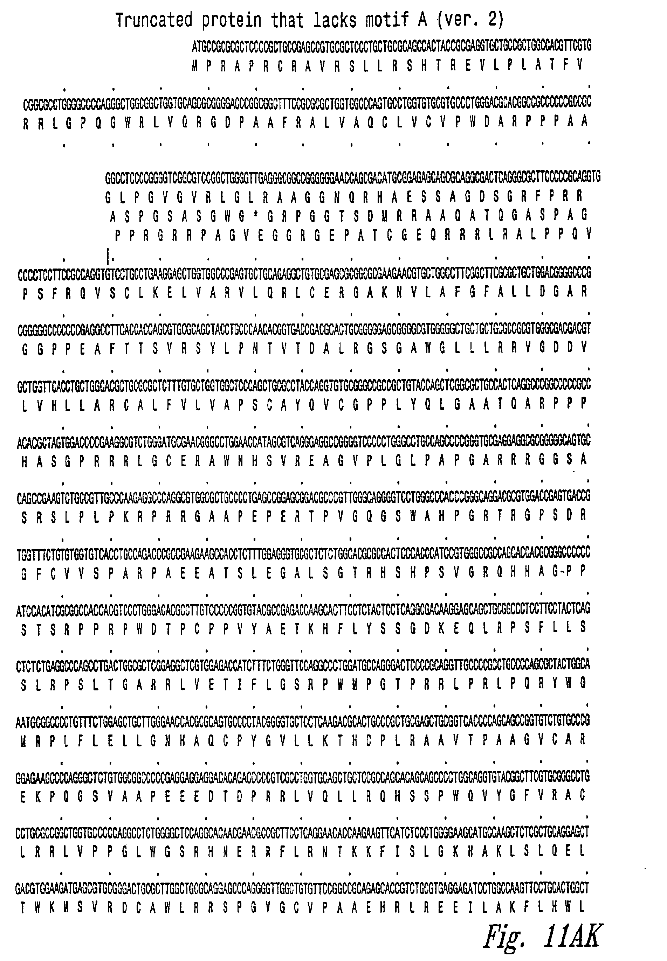

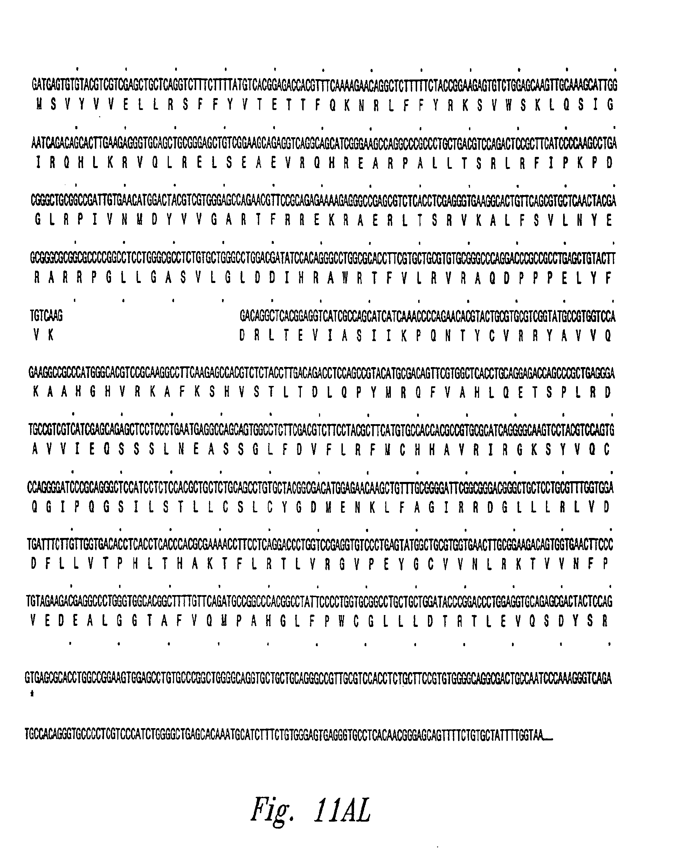

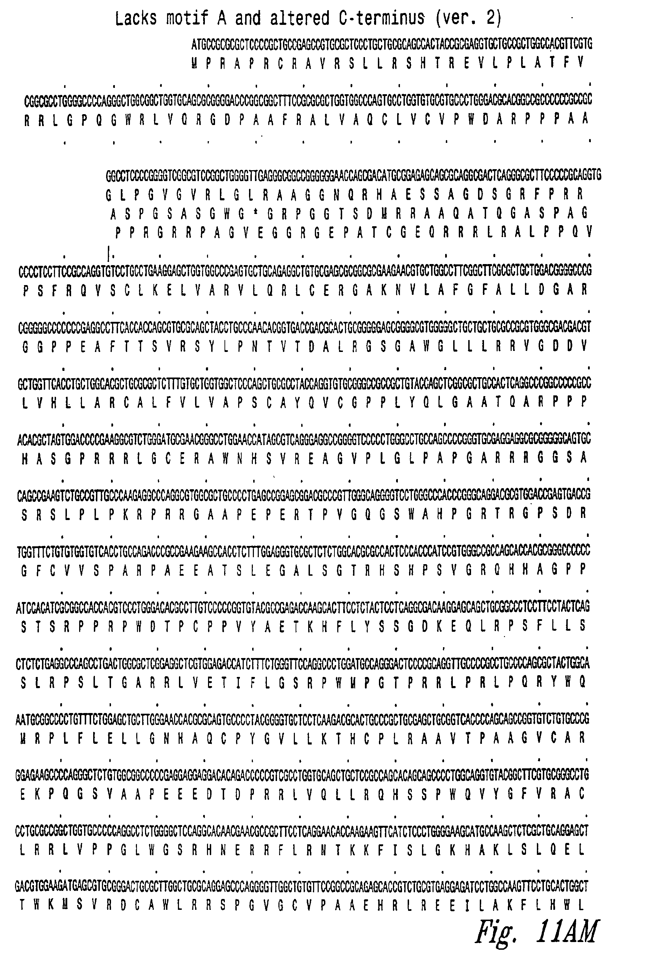

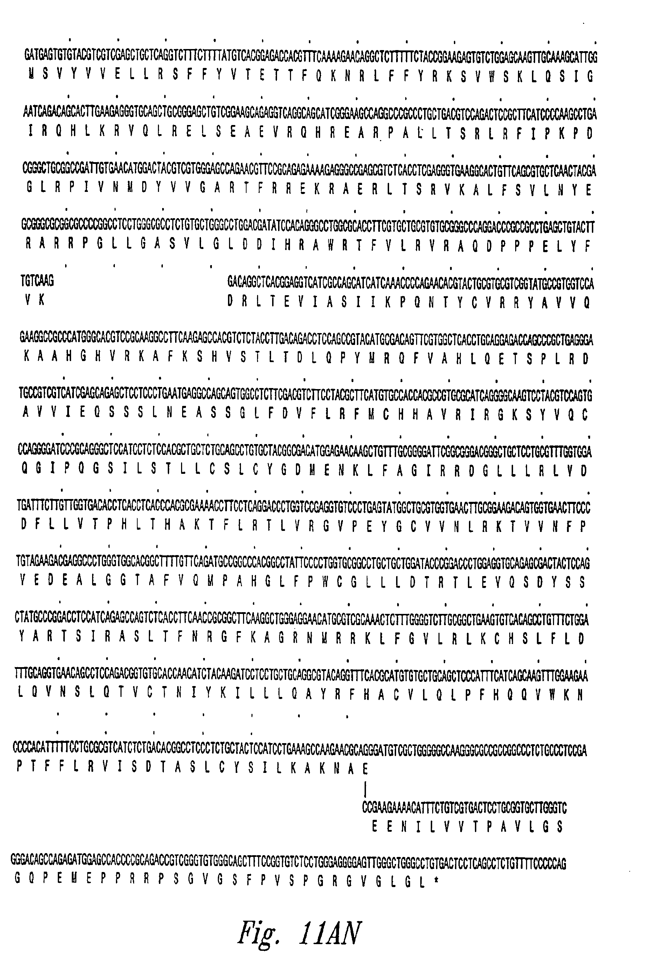

In other preferred embodiments, the nucleic acid molecule comprises any of the sequences presented in Figure I 1 or encodes any of the amino acid sequences presented in Figure 11, or hybridizes under normal stringency conditions to the complement of the sequences thereof, provided that the nucleic acid molecule is not EST AA281296. In other embodiments, the nucleic acid molecule comprises any of the sequences presented in Figure 10, or hybridizes under normal stringency conditions to the complement of the sequences thereof.

In another aspect, the invention provides an oligonucleotide comprising from 10 to 100 contiguous nucleotides from the sequence presented in Figure 1 or its complement and from 10 to 100 contiguous nucleotides from the sequences presented in Figure 10 or the complements thereof. The oligonucleotides may be labeled with a detectable label.

In yet another aspect, an expression vector is provided, comprising a heterologous promoter operably linked to a nucleic acid molecule of human telomerase. The vector may be selected from the group consisting of bacterial vectors, retroviral vectors, adenoviral vectors and yeast vectors. Host cells containing such vectors are also provided.

In another aspect, the invention provides an isolated protein comprising a human telomerase protein. The protein may comprise the amino acid sequence presented in Figure 1 or variant thereof or any of the amino acid sequences presented in Figure 11 or variant thereof. In a related aspect, the protein is a portion of a human telomerase protein, which may derive from the sequences presented in Figures 1 or 11. In preferred embodiments, the portion is from 10 to 100 amino acids long.

In other aspects, antibodies that specifically binds to human telomerase protein or portions are provided.

In a preferred aspect, an oligonucleotide (*e.g.*, a nucleic acid probe or primer) is provided that is capable of specifically hybridizing to a nucleic acid molecule encoding a human telomerase under conditions of normal stringency. Within certain embodiments, the nucleic acid molecule has a detectable label. Within certain embodiments, the nucleic acid molecule is selected such that it does not hybridize to nucleotides 1624-2012 presented in Figure 1. Within certain embodiments of the invention, the nucleic acid probe or primer may differ from a wild-type telomerase sequence by one or more nucleotides.

In a related aspect, the invention provides a pair of oligonucleotide primers capable of specifically amplifying all or a portion of a nucleic acid molecule encoding human telomerase. In specific embodiments, the nucleic acid molecule comprises the sequence presented in Figure 1, Figure 11, or complements thereof. In preferred embodiments, the pair of primers is capable of specifically amplifying sequence comprising all or a part of region 1, region α, region β, region 2, region 3 region X or region Y. In a related aspect, the invention provides an oligonucleotide that hybridizes specifically to a nucleic acid sequence in region 1, region α, region β, region 2, region 3 region X or region Y.

Methods for diagnosing cancer in a patent are also provided. These methods comprise preparing tumor cDNA and amplifying the tumor. cDNA using primers that specifically amplify human telomerase nucleic acid sequence, wherein the detection of telomerase nucleic acid sequences is indicative of a diagnosis of cancer. The amount of detected sequences may be comared to the amount of amplified telomerase sequence to a control, wherein increase telomerase nucleic acid sequences over the control is indicative of a diagnosis of cancer.

In yet another aspect, a method of determining a pattern of telomerase RNA expression in cells is provided, comprising preparing cDNA from mRNA isolated from the cells, amplifying the cDNA using primers according to claim 35, therefrom determining the pattern of telomerase RNA expression. In preferred embodiments, the method further comprises detecting the amplified product by hybridization with an oligonucleotide having all or part of the sequence of region 1, region α, region β, region 2, region 3 region X or region Y. These methods may be used to diagnose cancer in a patient, wherein the pattern is indicative of a diagnosis of cancer.

The invention also provides non-human transgenic animals whose cells contain a human telomerase gene that is operably linked to a promoter effective for the expression of the gene. In preferred embodiments, the animal is a mouse and the promoter is tissue-specific. In a related aspect, the invention provides a mouse whose cells have an endogenous telomerase gene disrupted by homologous recombination with a nonfunctional telomerase gene, wherein the mouse is unable to express endogenous telomerase.

The invention also provides inhibitors of human telomerase activity, as well as assays for identifying inhibitors of telomerase activity wherein the inhibitor binds to telomerase and is not a nucleoside analogue. The inhibitor may be an antisense nucleic acid complementary to human telomerase mRNA, a ribozyme and the like. The inhibitors may be used to treat cancer.

Also provided are methods for identifying an effector of telomerase activity, comprising the general steps of (a) adding a candidate effector to a mixture of telomerase protein, RNA component and template, wherein the telomerase protein is encoded by an isolated nucleic acid molecule as described above; (b) detecting telomerase activity, and (c) comparing the amount of activity in step (b) to the amount of activity in a control mixture without candidate effector, therefrom identifying an effector. Within further embodiments the effector is an inhibitor. With yet other embodiments the the nucleic acid molecule encodes human telomerase.

These and other aspects of the present invention will become evident upon reference to the following detailed description and attached drawings. In addition, various references are set forth below which describe in more detail certain procedures or compositions (*e.g*., plasmids, etc.), and are therefore incorporated by reference in their entirety.

### BRIEF DESCRIPTION OF THE DRAWINGS

Figures 1A-E present a DNA sequence (SEQ ID No:__) and predicted amino acid sequence (SEQ ID No:__) of human telomerase.

Figure 2 presents an alignment of *Euplotes aediculatus* p123 (SEQ ID No:__), yeast (EST2) (SEQ ID No:__) and human (HT1) telomerase protein (amino acids 29-1132) sequences. Reverse transcriptase motifs are indicated. The region of high homology among all three proteins is defined as the Telomerase region. The sequences are aligned with ClustalW.

Figure 3 is a scanned image of a Northern analysis showing that the telomerase catalytic subunit is expressed in LIM 1215 colon carcinoma cells but not in CCD primary fibroblasts. An mRNA of approximately 3.8 kb hybridizes to the hT1 probe. An additional cross-hybridizing mRNA of higher molecular weight is indicated by the top arrowhead. Cross-hybridization to ribosomal RNA present in the polyA⁺ RNA preparation is indicated. The same blot is also hybridized to a probe from the GAPDH gene as a loading control (lower panel). Marker sizes are indicated in kb.

Figure 4 is a scanned image of a Southern analysis showing that the telomerase catalytic subunit is encoded by a single gene and is not amplified in LIM 1215 cells. Genomic DNA isolated from peripheral human blood and LIM 1215 cell line is probed with a hT1 probe. The blot also contains dilutions of probe plasmid to control for the sensitivity of detection. The plasmid is diluted to approximately 10, 5 and 1 genome equivalents. H, *Hind* III; E, *Eco* RI; P, *Pst* I; X, *Xba* I; B, *Bam* HI.

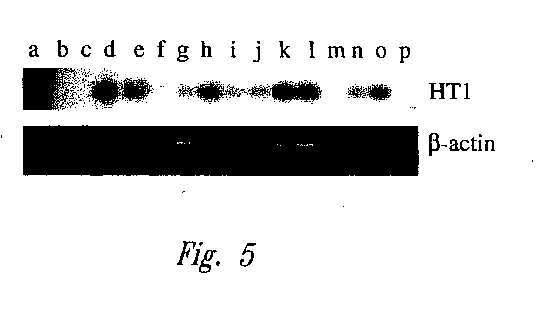

Figure 5 shows the results of amplification of cDNAs synthesized from various tissues. Amplification is performed using primers from the hT1 cDNA sequence that span an intron in the hT1 gene, and the products are blotted and probed with a radiolabeled oligonucleotide from the hT1 sequence. Amplification is also performed on the same samples with a pair of primers from the β-actin gene as a loading control. a: hT1 cDNA control; b: human genomic DNA control; c: no template control; d: normal colon RNA; e: normal testis RNA; f: normal lymphocyte RNA; g: melanoma RNA (cerebral metastasis); h: melanoma RNA (subcutaneous ankle metastasis); i: melanoma RNA (liver metastasis); j: melanoma RNA (lung metastasis); k: melanoma RNA (axillary lymph node metastasis); 1: melanoma RNA (skin metastasis); m: breast carcinoma RNA; n: breast carcinoma RNA; o: breast carcinoma RNA; p: breast carcinoma RNA.

Figure 6 presents results showing hT1 expression in pre-crisis cells and post-crisis cell lines. Upper panel: Nested amplification using primers within the original EST. Lower panel: Control RT-PCR using β-actin primers. a: BET-3K passage (p) 7 (pre-crisis); b: BET-3K p32 (post-crisis); c: BFT-3K p14 (pre-crisis); d: BFT-3K p 22 (post-crisis); e: BFT-3B p15 (pre-crisis); f: BFT-3B p29 (post-crisis); g: GM897 (ALT); h: IIICF/c (ALT); i: IIICF-T/B1 (ALT);j: No template control.

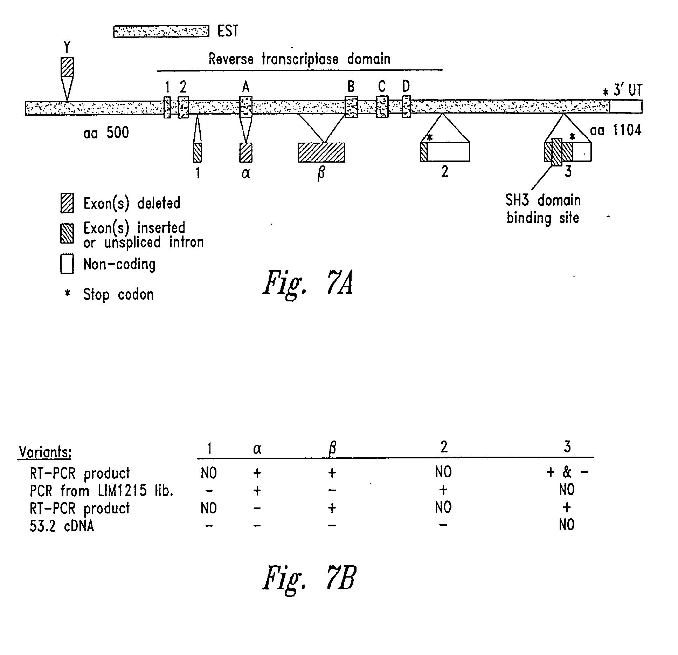

Figures 7A-C show some alternative splicing patterns of the hT1 transcript. A, Schematic representation of six splicing variants. B, Combinations of some identified RNA variants. C, Sequences of putative exon/intron junctions of RNA variants. Variants are marked as in part A. A complete DNA sequence (with protein translation) (SEQ ID No:__) of variant 3 is presented. Amino acids corresponding to a potential c-Abl/SH3 binding site are underlined. Putative exon/intron junctions are marked with and sequence coordinates are as in Figure 1. Putative spliced exons are in lower case and putative unspliced introns are in bold.

Figure 8 shows various splicing patterns of hT1 transcript in different tumor samples. Nested amplification (14 cycles) is performed using HT2026F and HT2482R primers on primary RT-PCR products generated with HT1875F and HT2781R primers. a: Lung carcinoma; b: Lymphoma; c: Lung carcinoma; d: Medulloblastoma; e: Lymphoma; f: Lymphoma; g: T47D; h: Pheochromocytoma; i: Lymphoma; j: Glioma; k: Lymphoma;1: No template control.

Figure 9 shows the results of amplification on cDNA synthesized from LIM 1215 cDNA. As shown, reverse transcriptase motif A is deleted from splicing variants containing sequence α. Primer combinations are: a, HTM2028F + HT2356R; b, HT2026F + HT2482R; c, HTM2028F + HT2482R; d. HT2026F + HT2482R.

Figures 10A-B present DNA sequences of variant regions of telomerase.

Figures 11A-W presents DNA and amino acid sequences of exemplary variant telomerase proteins.

Figure 12 is a scanned image of a telomerase activity assay.

Figures 13A-D present a schematic diagram of plasmid pAK128.4 and the DNA sequence of the plasmid.

Figures 14A-E present a schematic diagram of plasmid pAK128.7 and the DNA sequence of the plasmid.

Figures 15A-D present a schematic diagram of plasmid pAK128.14 and the DNA sequence of the plasmid.

### DETAILED DESCRIPTION OF THE INVENTION

Prior to setting forth the invention, it may be helpful to an understanding thereof to define certain terms used herein.

As used herein, "wild-type telomerase" generally refers to a polypeptide that enzymatically synthesizes nucleic acid sequences comprising simple repeat sequences (*e.g.,* CCCTAA, see Zakian, *Science 270*: 1601, 1995) to ends of chromosomes. The amino acid sequence of one representative wild-type telomerase from human has been deduced and is presented in Figure 1 (SEQ ID No. __). Within the context of this invention, it should be understood that telomerases of this invention include not only wild-type protein, but also variants (including alleles) of the wild-type protein sequence. Such variants may not necessarily exhibit enzymatic function. Briefly, such variants may result from natural polymorphisms, including RNA splice variants, generated by genetic recombination, or be synthesized by recombinant methodology, and moreover, may differ from wild-type protein by one or more amino acid substitutions, insertions, deletions, rearrangements or the like. Typically, when the result of synthesis, amino acid substitutions are conservative, *i.e*., substitution of amino acids within groups of polar, non-polar, aromatic, charged, etc. amino acids. In the region of homology to the wild-type sequence in the RTase motif regions variants will preferably have at least 90% amino acid sequence identity, and within certain embodiments, greater than 92%, 95%, or 97% identity. Outside the RTase motif region, variants will preferably have 75% amino acid identity, and within certain embodiments, at least 80%, 85%, 90%, 92%, 95% or 97% identity.

As will be appreciated by those skilled in the art, a nucleotide sequence encoding telomerase may differ from the wild-type sequence presented in the Figures; due to codon degeneracies, nucleotide polymorphisms, or amino acid differences. In other embodiments, variants should preferably hybridize to the wild-type nucleotide sequence at conditions of normal stringency, which is approximately 25-30°C below Tm of the native duplex (*e.g*., 1 M Na+ at 65°C; 5X SSPE, 0.5% SDS, 5X Denhardt's solution, at 65°C or equivalent conditions; *see generally,* Sambrook et al. *Molecular Cloning: A Laboratory Manual,* 2nd ed., Cold Spring Harbor Press, 1987; Ausubel et al., *Current Protocols in Molecular Biology,* Greene Publishing, 1987). Tm for other than short oligonucleotides can be calculated by the formula Tm=81.5 + 0.41%(G+C)-log(Na+). Low stringency hybridizations are performed at conditions approximately 40°C below Tm, and high stringency hybridizations are performed at conditions approximately 10°C below Tm. Variants preferably have at least 75% nucleotide identity to wild-type sequence in the RTase motif region, preferably at least 80%, 85%, and most preferably at least 90% nucleotide identity.

As used herein, a "promoter" refers to a nucleotide sequence that contains elements that direct the transcription of a linked gene. At minimum, a promoter contains an RNA polymerase binding site. More typically, in eukaryotes, promoter sequences contain binding sites for other transcriptional factors that control the rate and timing of gene expression. Such sites include TATA box, CAAT box, POU box, AP 1 binding site, and the like. Promoter regions may also contain enhancer elements. When a promoter is linked to a gene so as to enable transcription of the gene, it is "operatively linked".

An "isolated nucleic acid molecule" refers to a polynucleotide molecule in the form of a separate fragment or as a component of a larger nucleic acid construct, that has been separated from its source cell (including the chromosome it normally resides in) at least once in a substantially pure form. Nucleic acid molecules may be comprised of a wide variety of nucleotides, including DNA, RNA, nucleotide analogues, or some combination of these.

### I. TELOMERASE, TELOMERASE GENES AND GENE PRODUCTS

As noted above, the invention provides compositions relating to vertebrate telomerase genes and gene products, and methods for the use of the genes and gene products. Given the disclosure provided herein, a telomerase gene can be isolated from a variety of cell types that express telomerase, including immortalized or transformed cells. As exemplified herein, a cDNA and variants encoding telomerase from human cells are identified, isolated, and characterized. Telomerase protein is then readily produced by host cells transfected with an expression vector encoding telomerase.

### A. Isolation of telomerase gene

As described herein, the invention provides genes encoding telomerase. Within one embodiment of the invention, a gene encoding human telomerase can be identified by amplification of a cDNA library using a primer pair designed from an EST sequence. The EST sequence GenBank Accession No. AA281296, is identified by sequence identity and similarity to a *Euplotes aediculatus* telomerase gene (GenBank accession no. U95964; Lingner et al., *Science 276*: 561, 1997). Sequence comparisons between the *Euplotes* telomerase gene and the EST show approximately 38% amino acid identity and 59% amino acid similarity.

Telomerase genes may be isolated from genomic DNA or cDNA. Genomic DNA is preferred when the promoter region or other flanking regions are desired. Genomic DNA libraries constructed in chromosomal vectors, such as YACs (yeast artificial chromosomes), bacteriophage vectors, such as λEMBL3, λgt10, cosmids, or plasmids, and cDNA libraries constructed in bacteriophage vectors (*e.g.,*λ ZAPII), plasmids, or others, are suitable for screening. Such libraries may be constructed using methods and techniques known in the art (see Sambrook et al., *Molecular Cloning: A Laboratory Manual,* Cold Spring Harbor Press, 1989) or purchased from commercial sources (*e.g.,* Clontech, Palo Alto, CA). The DNA may be isolated from vertebrate cells, such as human cells, mouse cells, other rodent or primatic cells, avian cells, and the like.

Within one embodiment, the telomerase gene is isolated by amplification using cDNA library DNA as templates. Using the reported EST sequence, human telomerase may be isolated. Briefly, sets of amplification primers are designed based upon the EST nucleotide sequence. Examples of such primers are presented in Table 2 (see also Example 1). Amplification of cDNA libraries made from cells with high telomerase activity is preferred. The primers described herein amplify a fragment that has a length predicted from the EST sequence from a LIM1215 cDNA library. LIM1215 is a human colon cancer cell line. Confirmation of the nature of the fragment is obtained by DNA sequence analysis.

DNA fragments encompassing additional sequence are amplified in reactions using a primer that hybridizes to vector sequence in conjunction with one of the EST primers. By using vector primers from either side of the cloning site in combination with the EST primers, a 1.6 kb fragment derived from the 3' region of h-TEL (human telomerase) and a 0.7 kb fragment derived from the 5' region are isolated. These fragments are verified as containing telomerase coding sequence by amplification with a pair of primers internal to the EST sequence. The two fragments are cloned into pBluescript and subjected to DNA sequence analysis. Additional DNA sequence is obtained by C-RACE and amplification procedures to obtain the 5' end of a cDNA as well as by hybridization and isolation of clones from the cDNA library.

The compiled DNA sequence and predicted amino acid sequence of a reference human telomerase are presented in Figure 1. As shown, the coding region of the reference telomerase is 3396 bases long and has an approximately 620 base long 3' untranslated region. The predicted amino acid sequence is 1132 amino acids long and may be delineated into four major domains: N-terminal, basic, reverse transcriptase (RT) and C-terminal. Furthermore, human telomerase contains regions of homology to other telomerases (*e.g.*, from *Euplotes* and *S. pombe*) and reverse transcriptases. These motifs are identified herein and in Kilian et al. (*Human Molecular Genetics, 12:* 2011-2019, 1997) as domains 1, 2, A, B, C, and D, in Nakamura et al., (*Science, 277:* 955-959) as domains 1, 2, A, B', C, D, and E, and in Meyerson et al. (*Cell, 90:* 785-795, 1997) as motifs 1-6. Regardless of the name used, these motifs encompass amino acids 621-626 (motif 1) and 631-634 (motif 2), 708-720 (motif A), 827-839 (motif B), 863-871 (motif C), and 895-902 (motif D). Because the boundaries of these motifs are based on similarity and identity with other telomerases, the functional boundary of each motif may be different.

In addition, variants of the reference telomerase sequence are obtained by amplifications, which are described herein. Their DNA and predicted amino acid sequences are presented in Figure 11 and discussed in further detail below. Briefly, some of these variants encode truncated proteins and others have different C-terminal sequences. These variants likely result from alternative RNA splicing because telomerase appears to be a single copy gene in humans (see Example 2).

Alternatively, other methods may be used to obtain a nucleic acid molecule that encodes telomerase. For example, a nucleic acid molecule encoding telomerase may be obtained from an expression library by screening with an antibody or antibodies reactive to telomerase (*see,* Sambrook et al. *Molecular Cloning: A Laboratory Manual,* 2nd Ed., Cold Spring Harbor Laboratory Press, NY, 1987; Ausubel et al. *Current Protocols in Molecular Biology,* Greene Publishing Associates and Wiley-Interscience, NY, 1995). In another embodiment, nucleic acid molecules encoding telomerase may be isolated by hybridization screening of cDNA or genomic libraries. Oligonucleotides for hybridization screening may be designed based on the DNA sequence of human telomerase presented herein. Oligonucleotides for screening are typically at least 11 bases long and more usually at least 20 or 25 bases long. In one embodiment, the oligonucleotide is 20-30 bases long. Such an oligonucleotide may be synthesized in an automated fashion. To facilitate detection, the oligonucleotide may be conveniently labeled, generally at the 5' end, with a reporter molecule, such as a radionuclide, (*e.g.*, ³²P), enzymatic label, protein label, fluorescent label, or biotin. A library is generally plated as colonies or phage, depending upon the vector, and the recombinant DNA is transferred to nylon or nitrocellulose membranes. Hybridization conditions are tailored to the length and GC content of the oligonucleotide. Following denaturation, neutralization, and fixation of the DNA to the membrane, membranes are hybridized with labeled probe. Suitable hybridization conditions may be found in Sambrook et al., *supra,* Ausubel et al., *supra,* and furthermore hybridization solutions may contain additives such as tetramethylammonium chloride or other chaotropic reagents or hybotropic reagents to increase specificity of hybridization (see for example, PCT/US97/17413). Following hybridization, suitable detection methods reveal hybridizing colonies or phage that are then isolated and propagated. Candidate clones or amplified fragments may be verified as containing telomerase DNA by any of various means. For example, the candidate clones may be hybridized with a second, non-overlapping probe or subjected to DNA sequence analysis. In these ways, clones containing a telomerase gene or gene fragment, which are suitable for use in the present invention, are isolated.

Telomerase DNA may also be obtained by amplification of cDNA or genomic DNA. Oligonucleotide primers for amplification of a full-length cDNA are preferably derived from sequences at the 5' and 3' ends of the coding region. Amplification of genomic sequences will use primers that span intronic sequences and may use conditions that favor long amplification products (see Promega catalogue). Briefly, oligonucleotides used as amplification primers preferably do not have self-complementary sequences nor have complementary sequences at their 3' end (to prevent primer-dimer formation). Preferably, the primers have a GC content of about 50% and contain restriction sites to facilitate cloning. Generally, primers are between 15 and 50 nucleotides long, and more usually between 20 and 35 nucleotides long. The primers are annealed to cDNA or genomic DNA and sufficient amplification cycles are performed to yield a detectable product, preferably one that is readily visualized by gel electrophoresis and staining. The amplified fragment is purified and inserted into a vector (*e.g.*, a viral, phagemid or plasmid vector, such as λgt10 or pBS(M13+)) and propagated.

Telomerase genes from a multitude of species can be isolated using the compositions provided herein. For closely related species, the human sequence or portion thereof may be utilized as a probe on a genomic or cDNA library. For example, a fragment of the telomerase gene that encompasses the catalytic site (approximately corresponding to amino acids 605-915 of Figure 1) may be labeled and used as a probe on a library constructed from mouse, primate, rat, dog, or other vertebrate, warm-blooded, or mammalian species. An initial hybridization at normal stringency may yield clones or fragments encoding telomerase. If no hybridization is observed, relaxed (low) stringency hybridizations may be pursued. Guidelines for varying the stringency of the hybridization may be acquired from Sambrook et al., *supra,* and other well-known sources. Such probes may also be used on libraries from evolutionarily diverse species, such as *Drosophila,* altliough hybridization conditions will typically be more relaxed.

Other methods may alternatively be used to isolate telomerase genes from non-human species. These methods include, but are not limited to, amplification using primers derived from conserved areas (*e.g*., RTase motifs), amplification using degenerate primers from various regions of telomerase including the RTase region, antibody probing of expression libraries, telomerase RNA probing of expression libraries, and the like. A gene sequence is identified as a telomerase by amino acid similarity and / or nucleic acid identity. Generally, amino acid similarity, which allows for conservative differences, is preferred to identify a telomerase. From diverse species, amino acid similarity is generally at least 30% and preferably at least 40% or at least 50%. Nucleic acid identity may be lower and thus difficult to assess. Several readily available computer analysis programs, such as BLASTN and BLASTP, are useful to determine relatedness of genes and gene products. Candidate telomerase genes are examined for enzyme activity by one of the functional assays described herein or other equivalent assays.

### B. Variant telomerase genes

Variants (including alleles) of the telomerase nucleic acid or amino acid sequence provided herein may be readily isolated from natural variants (e.g., polymorphisms, splice variants, mutants), synthesized, or constructed. Depending upon the intended use, mutants may be constructed to exhibit altered or deficient telomerase function. Particularly useful telomerase genes encode a protein lacking enzyme activity but that has a dominant negative phenotype. The telomerase variants, moreover, may lack one or more of known telomerase activities, including reverse transcriptase activity, nucleolytic activity, telomere binding activity, dNTP binding activity, and telomerase RNA (hTR) binding activity.

One skilled in the art recognizes that many methods have been developed for generating mutants (*see, generally,* Sambrook et al., *supra;* Ausubel et al., *supra*). Briefly, preferred methods for generating a few nucleotide substitutions utilize an oligonucleotide that spans the base or bases to be mutated and contains the mutated base or bases. The oligonucleotide is hybridized to complementary single stranded nucleic acid and second strand synthesis is primed from the oligonucleotide. Similarly, deletions and/or insertions may be constructed by any of a variety of known methods. For example, the gene can be digested with restriction enzymes and religated such that some sequence is deleted or ligated with an isolated fragment having cohesive ends so that an insertion or large substitution is made. In another embodiment, variants are generated by "exon shuffling" (see U.S. Patent No. 5,605,793). Variant sequences may also be generated by "molecular evolution" techniques (see U. S. Patent No. 5,723,323). Other means to generate variant sequences may be found, for example, in Sambrook et al. (*supra*) and Ausubel et al. (*supra*). Verification of variant sequences is typically accomplished by restriction enzyme mapping, sequence analysis, or probe hybridization, although other methods may be used. The double-stranded nucleic acid is transformed into host cells, typically *E. coli,* but alternatively, other prokaryotes, yeast, or larger eukaryotes may be used. Standard screening protocols, such as nucleic acid hybridization, amplification, and DNA sequence analysis, will identify mutant sequences.

In preferred embodiments, variant telomerases are inactive with respect to enzyme activity and impart a dominant negative phenotype to a host cell. Regardless of the actual mechanism, when a dominant negative telomerase is expressed in a cell, the native active telomerase is rendered inactive. In the catalytic domain, RTase motifs share conserved aspartic acid residues. Human telomerase also contains these critical residues: Asp 712, Asp 718, Asp 868, and Asp 869. Mutation of one or more of these Asp residues to a non-conservative amino acid (*e.g*., alanine) will likely destroy enzymatic activity and or affect telomere shortening. For each of these mutants, dominant negativity is assayed. Preferred mutants are dominant negative and induce a senescence phenotype in certain embodiments. Other dominant negative variants may be generated by deletion of one or more of the RTase motifs or alteration of the region involved in DNA priming (such as motif E), binding site for the RNA component, the template binding site, the metal ion binding site (such as motif C), and the like.

In other embodiments, the nucleic acid molecule encoding telomerase may be fused to another nucleic acid molecule. As will be appreciated, the fusion partner gene may contribute, within certain embodiments, a coding region. Thus, it may be desirable to use only the catalytic site of telomerase (*e.g.*, amino acids 609-915), individual RTase motifs (described above), any of the splicing variant telomerases described herein, the telomerasc RNA binding site and the like. The choice of the fusion partner depends in part upon the desired application. The fusion partner may be used to alter specificity of the telomerase, provide a reporter function, provide a tag sequence for identification or purification protocols, and the like. The reporter or tag can be any protein that allows convenient and sensitive measurement or facilitates isolation of the gene product and does not interfere with the function of the telomerase. For reporter function, β-glucuronidase (U.S. Patent No: 5,268,463), green fluorescent protein and β-galactosidase are readily available as DNA sequences. A peptide tag is a short sequence, usually derived from a native protein, which is recognized by an antibody or other molecule. Peptide tags include FLAG®, Glu-Glu tag (Chiron Corp., Emeryville, CA) KT3 tag (Chiron Corp.), T7 gene 10 tag (Invitrogen, La Jolla, CA), T7 major capsid protein tag (Novagen, Madison, WI), His₆ (hexa-His), and HSV tag (Novagen). Besides tags, other types of proteins or peptides, such as glutathione-S-transferase may be used.

### C. Fragments and oligonucleotide derived from telomerase genes

In addition, portions or fragments of telomerase gene may be isolated or constructed for use in the present invention. For example, restriction fragments can be isolated by well-known techniques from template DNA, *e.g.,* plasmid DNA, and DNA fragments, including restriction fragments, can be generated by amplification. Furthermore, oligonucleotides can be synthesized or isolated from recombinant DNA molecules. One skilled in the art will appreciated that other methods are available to obtain DNA or RNA molecules having at least a portion of a telomerase sequence. Moreover, for particular applications, these nucleic acids may be labeled by techniques known in the art with a radiolabel (*e.g.,* ³²P, ³³P,³⁵S, ¹²⁵I, ¹³¹I, ³H, ¹⁴C), fluorescent label (*e.g*., FITC, Cy5, RITC, Texas Red), chemiluminescent label, enzyme, biotin and the like.

Methods for obtaining fragments are well-known in the art. Portions that are particularly useful within the context of this invention contain the catalytic site, individual RTase motifs, the putative intronic sequences (see Figure 10), and the like. Oligonucleotides are generally synthesized by automated fashion; methods and apparatus for synthesis are readily available (*e.g.*, Applied Biosystems Inc, CA). Oligonucleotides may contain non-naturally occurring nucleotides, such as nucleotide analogues, a modified backbone (*e.g*., peptide backbone), nucleotide derivatives (*e.g*., biotinylated nucleotide), and the like. As used herein, oligonucleotides refers to a nucleic acid sequence of at least about 7 nucleotides and generally not longer than about 100 nucleotides. Usually, oligonucleotides are between about 10 and about 50 bases, more often between about 18 and about 35 nucleotides long. Oligonucleotides can be single-stranded or in some cases double-stranded. As used herein, portions of a nucleic acid refer to a polynucleotide that contains less than the entire parental nucleic acid sequence. For example, a portion of telomerase coding sequence contains less than a full-length telomerase sequence. A 'portion' is generally at least about seven nucleotides, and may be as many as 10, 20, 25 or more nnucleotides in length. A fragment refers to a polynucleotide molecule of any length and can encompass an oligonucleotide, although more usually, but not to be limiting, the term oligonucleotide is used to denote short polynucleotides and the term fragment is used to denote longer polynucleotides.

Oligonucleotides for use as primers for amplification and probes for hybridization screening may be designed based on the DNA sequence of human telomerase presented herein. Oligonucleotide primers for amplification of a full-length cDNA are preferably derived from sequences at the 5' and 3' ends. Primers for amplification of specific regions are chosen to generate products of an easily detectable size. In preferred embodiments, primers are chosen that flank the sequences subject to alternative RNA splicing. In preferred embodiments, one set of primers is chosen such that both the product that spans spliced-in sequence as well as the product that spans spliced-out sequence are suitable sizes to be detected under the same reaction conditions. In other embodiments, two sets of primers are used to detect the alternative spliced RNAs. For example, one set of primers flanks the splice junction in order to detect a spliced-out product. The second set of primers may be derived very close to the junction (such that a spliced-out amplification product is the same size or barely larger than a primer-dimer length) or one or more of the set may be derived from the spliced-in sequence (such that the spliced-out RNA would not yield any product). An amplified

Amplification primers preferably do not have self-complementary sequences nor have complementary sequences at their 3' end (to prevent primer-dimer formation). Preferably, the primers have a GC content of about 50% and may contain restriction sites to facilitate cloning. Amplification primers usually are at least 15 bases and usually are not longer than 50 bases, although in some circumstances and conditions shorter or longer lengths can be used. More usually, primers are from 17 to 40 bases long, 17 to 35 bases long, or 20 to 30 bases long. The primers are annealed to cDNA or genomic DNA and sufficient amplification cycles, generally 20-40 cycles, are performed to yield a product readily visualized by gel electrophoresis and staining or by hybridization. The amplified fragment can be purified and inserted into a vector, such as λgt10 or pBS(M13+), and propagated, isolated and subjected to DNA sequence analysis, subjected to hybridization, or the like.

An oligonucleotide hybridization probe suitable for screening genomic, cDNA or other types (*e.g.*, mutant telomerase sequences) of libraries, probing southern, northern, or northwestern blots, amplification products, and the like may be designed based on the sequences provided herein. Oligonucleotides for hybridization are typically at least 11 bases long, generally less than 100 bases long, and preferably at least 15 bases long, at least 20 bases long, at least 25 bases long, and preferably 20-70, 25-50, or 30-40 bases long. To facilitate detection, the oligonucleotide may be conveniently labeled, generally at the 5' end, with a reporter molecule, such as a radionuclide, (*e.g.*, ³²P), enzymatic label, protein label, fluorescent label, or biotin. (see Ausubel et al., and Sambrook et al., *supra*). A library is generally plated as colonies or phage, depending upon the vector, and the recombinant DNA is transferred to nylon or nitrocellulose membranes. Following denaturation, neutralization, and fixation of the DNA to the membrane, membranes are hybridized with labeled probe, and washed. Suitable detection methods reveal hybridizing colonies or phage that are then isolated and propagated. Methods for transferring nucleic acids to membranes and performing hybridizations are well known. In certain embodiments, additives to hybridization solution, such as a chaotrope (*e.g.*, tetramethylammonium chloride) or a hybotrope (*e.g.*, ammonium trichloroacetate; see PCT/ US97/17413) are added to increase sensitivity and specificity of hybridization. A probe specifically hybridizes to a nucleic acid if it remains detectably annealed after washing under conditions equivalent to hybridization conditions (expressed herein as the number of degrees less than Tm).

### D. Splicing variants of human telomerase

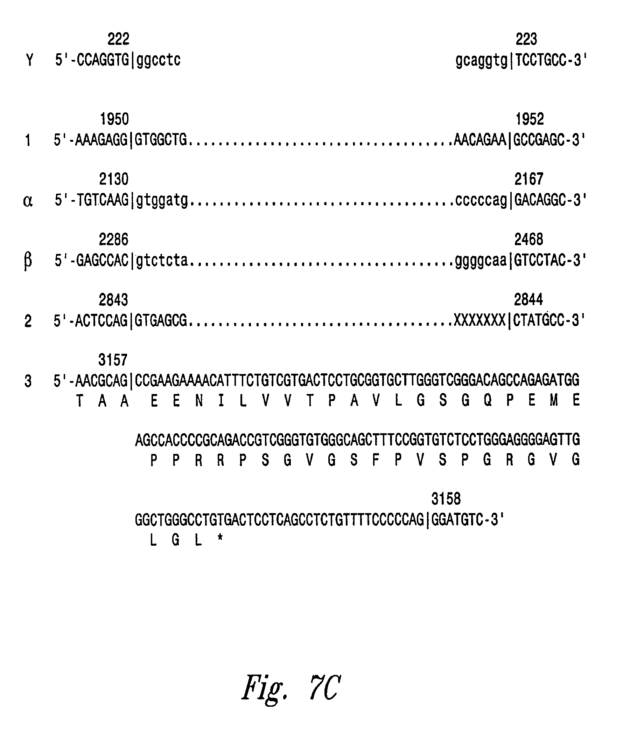

In addition to the reference telomerase DNA and protein sequences presented in Figures 1, several RNA splice variants are observed. Although some of the variants may reflect incompletely processed mRNA, it is noteworthy that such variants are abundant in an RNA sample (LIM1215) preselected for polyadenylated mRNA. These findings, together with their clustering in the RT domain, suggest that the insertion variants more likely reflect regulation of hT1 protein expression. For example, variants in which exons are deleted (see α, β, Fig. 7) are likely alternative mature coding for variant proteins. Additional evidence in support of alternative proteins comes from sequence analysis of cDNA clones identified in a LIM1215 cDNA library that contained both deletions and insertions compared to the reference sequence.

At least seven different putative introns appear to be retained in mRNAs (see Figure 7, which displays 6 of the 7 introns). The introns may be independently retained, thus, a particular mRNA may have none, any one, two, etc. up to seven introns. The maximum number of different mRNAs resulting from seven independently spliced introns is 2⁷, or 128 different mRNAs. DNA sequences of these introns are presented in Figure 10. The 5' most intron, called sequence "X", is an unknown length, and only a partial sequence is presented.

The reference telomerase sequence (Figure 1) includes intron α and intron β. In the following discussion, the effect of presence/absence and location of each intron is presented on the basis that it is the only alteration. It will be appreciated that a particular intron may alter the sequence of the translated product, regardless of whether other introns are spliced in or out. For example, the presence of intron 1 results in a frameshift and truncated protein, regardless of whether introns α, β, 2 or 3 are spliced in or out.

The presence of intron "X" results in a truncated protein that contains approximately 600 N-terminal amino acids and lacks all of the RTase motifs. The presence of intron "Y" at base 222 results in a frameshifted protein that terminates within three codons past the intron. As the Y intron is very GC rich, approximately 78%, which is difficult to sequence, it is possible that intron Y causes an insertion of about 35 amino acids and not a frameshift.

Intron 1 at nucleotide 1950 is 38 bp and its presence in mRNA causes a frame-shift and ultimate translation of a truncated protein (stop codon at nt 1973). This truncated protein contains only RTase domains 1 and 2.

Intron α, located from bases 2131-2166 is frequently observed spliced out of telomerase mRNA. A protein translated from such an RNA is deleted for 12 amino acids, removing RTase motif A. This motif appears to be critical for RT function; a single amino acid mutation within this domain in the yeast EST2 protein results in a protein that functions as a dominant negative and results in cellular senescence and telomere shortening.

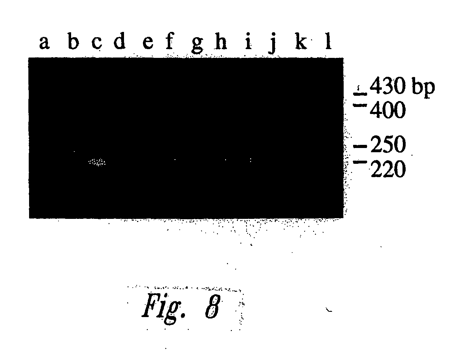

Another of the variant sequences, the β-exon deletion at base 2286-2468, encodes a truncated protein, due to a reading frameshift at base 2287, which is joined to base 2469, and subsequently a termination codon at base 2605. This variant protein has RTase domains 1, 2, A, B, and part of C, but lacks another motif; in addition to the RTase domain motifs, another sequence motif (AVRIRGKS) identified in the β insert of hT1 matches a P-loop motif consensus AXXXXGK(S) (Saraste et al., *Trends Biochem. Sci. 15,* 430-434, 1990). This motif is found in a large number of protein families including a number of kinases, bacterial dnaA, recA, recF, mutS and ATP-binding helicases (Devereaux et al., *Nucleic Acids Res., 12*, 387-395, 1984). The P-loop is thus present only in a subpopulation of the h-TEL mRNAs in most RNA samples analyzed and completely absent from several tumor samples (Figure 8).

Intron 2 at base 2843 contains an in-frame termination codon, resulting in a truncated protein that has the entire RTase domain region, but lacks the C-terminus. As the C-terminus may play a regulatory role, protein activity will likely be affected. When intron 3 is retained, a smaller protein is also produced because the intron contains an in-frame stop codon. Thus, the protein has an altered C-terminal sequence. What activity such proteins might have is currently unknown. The crystal structure of the HIV-1 reverse transcriptase demonstrates that a short form of the protein (p51) that lacks the RNAase domain is inhibited by the C-terminal 'connection' folding into the catalytic cleft. If hT1 is assumed to adopt a similar structure to HIV-RT, then C-terminal hT1 protein variants may reflect a similar mechanism of regulation.

In addition to variants that lack the reference C-terminal domain, a variant with intron 3 at base 2157 expresses an alternative C-terminal domain. Furthermore, the coding region donated by intron 3 has a potential SH3 binding site, SGQPEMEPPRRPSGCVG, which matches the consensus c-Abl SH3 binding peptide (PXXXXPXXP) found in proteins such as ataxia telangiectasia mutated (ATM). A second example of this motif is found within the N-tenninal end of the hT1 protein in the peptide HAGPPSTSRPPRPWDTP. Other alternative C-terminal domains are found in telomerase cDNAs; the EST12462 (GenBank Accession No. AA299878) has about 50 bases of identical sequence up to base 2157 and then diverges from the reference telomerase sequence as well as intron 3. This new sequence has an internal stop codon in 50 bases that would result in a truncated C-terminus.

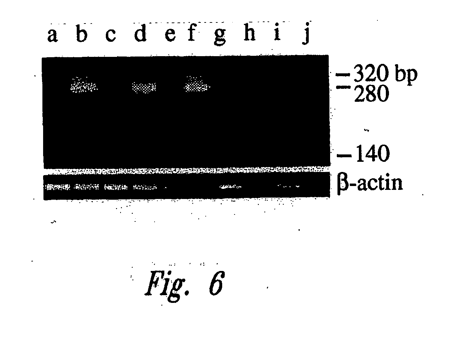

The variant detected in one ALT cell line (Fig. 6, lane i) opens up the possibility that the basic domain of hT1 may contribute to the ALT mechanism in at least some ALT cell lines. Interestingly, this ALT cell line expresses the hTR gene. One possible mechanism of ALT could involve dysregulated telomerase components that are inactive in the TRAP assay.

The following table summarizes the splice variants and resulting proteins. For simplicity, only a single variant is listed for each resulting protein. Furthermore, as noted above, the presence of the Y intron appears to cause a frameshift resulting in a truncated protein, but may cause an insertion. Thus, each reading frame of the Y intron is presented and the table is constructed as if the insertion does not cause a truncated protein. An independent assortment of these known introns would lead to 128 different mRNA sequences. The DNA and amino acid sequences for the variants in Table 1 are presented in Figure 11.

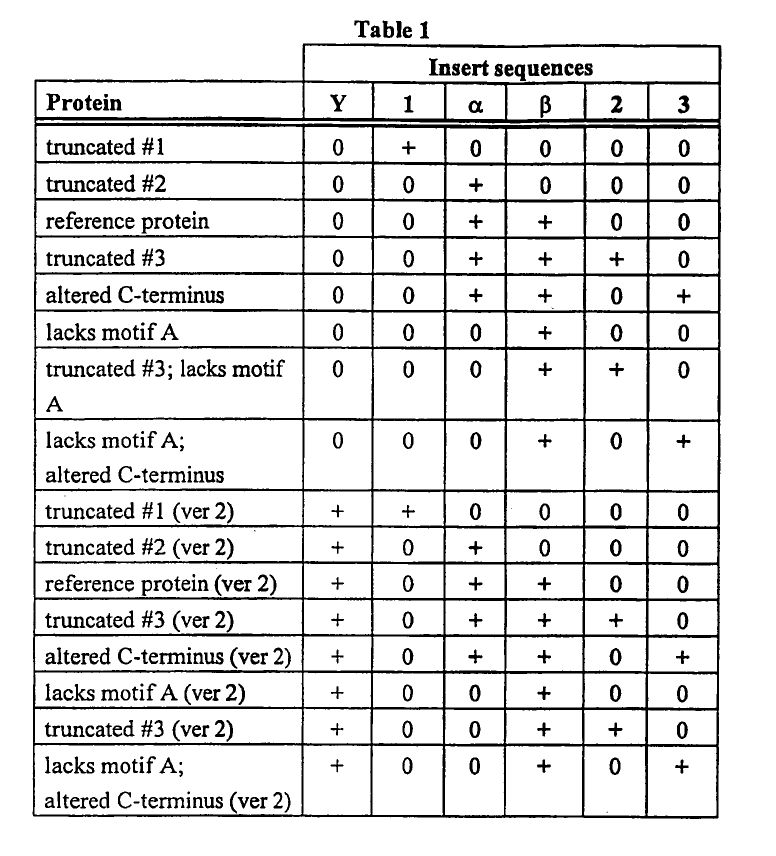

### E. Vectors, host cells and means of expressing and producing protein

Telomerase protein may be expressed in a variety of host organisms. In one embodiment, telomerase is produced in bacteria, such as *E. coli,* for which many expression vectors have been developed and are readily available. Other suitable host organisms include other bacterial species, and eukaryotes, such as yeast (*e.g., Saccharomyces cerevisiae*), mammalian cells (*e.g.,* CHO and COS-7), and insect cells (*e.g.,* Sf9).

A DNA sequence encoding telomerase, a portion thereof, a variant, fusion protein or the like, is introduced into an expression vector appropriate for the host. In certain embodiments, telomerase is inserted into a vector such that a fusion protein is produced. The telomerase sequence is derived from an existing fragment, cDNA clone, or synthesized. A preferred means of synthesis is amplification of the gene from cDNA using a set of primers that flank the coding region or the desired portion of the protein. As discussed above, the telomerase sequence may contain alternative codons for each amino acid with multiple codons. The alternative codons can be chosen as "optimal" for the host species. Restriction sites are typically incorporated into the primer sequences and are chosen with regard to the cloning site of the vector. If necessary, translational initiation and termination codons can be engineered into the primer sequences.

At minimum, the vector must contain a promoter sequence. Other regulatory sequences may be included. Such sequences include a transcription termination signal sequence, secretion signal sequence, origin of replication, selectable marker, and the like. The regulatory sequences are operationally associated with one another to allow transcription or translation.

The plasmids used herein for expression of telomerase include a promoter designed for expression of the proteins in a host cell (*e.g.*, bacterial). Suitable promoters are widely available and are well known in the art. Inducible or constitutive promoters are preferred. Such promoters for expression in bacteria include promoters from the T7 phage and other phages, such as T3, T5, and SP6, and the *trp, Ipp,* and *lac* operons. Hybrid promoters (*see,* U.S. Patent No. 4,551,433), such as *tac* and *trc,* may also be used. Promoters for expression in eukaryotic cells include the P10 or polyhedron gene promoter of baculovirus/insect cell expression systems (*see, e.g.,* U.S. Patent Nos. 5,243,041, 5,242,687, 5,266,317, 4,745,051, and 5,169,784), MMTV LTR, CMV IE promoter, RSV LTR, SV40, metallothionein promoter (*see, e.g.,* U.S. Patent No. 4,870,009) and other inducible promoters. For expression of the proteins, a promoter is inserted in operative linkage with the coding region for the telomerase protein.

The promoter controlling transcription of the telomerase may itself be controlled by a repressor. In some systems, the promoter can be derepressed by altering the physiological conditions of the cell, for example, by the addition of a molecule that competitively binds the repressor, or by altering the temperature of the growth media. Preferred repressor proteins include, but are not limited to, the *E. coli* lacI repressor, which is responsive to IPTG induction, the temperature sensitive λcI857 repressor, and the like. The *E. coli* lacI repressor is preferred.

In other preferred embodiments, the vector also includes a transcription terminator sequence, which has either a sequence that provides a signal that terminates transcription by the polymerase that recognizes the selected promoter and/or a signal sequence for polyadenylation.

Preferably, the vector is capable of replication in the host cells. Thus, when the host cell is a bacterium, the vector preferably contains a bacterial origin of replication. Preferred bacterial origins of replication include the f1-ori and col E1 origins of replication, especially the ori derived from pUC plasmids. In yeast, ARS or CEN sequences can be used to assure replication. A well-used system in mammalian cells is SV40 ori.

The plasmids also preferably include at least one selectable marker that is functional in the host. A selectable marker gene includes any gene that confers a phenotype on the host that allows transformed cells to be identified and selectively grown. Suitable selectable marker genes for bacterial hosts include the ampicillin resistance gene (Amp^{r}), tetracycline resistance gene (Tc^{r}) and the kanamycin resistance gene (Kan^{r}). The kanamycin resistance gene is presently preferred. Suitable markers for eukaryotes usually require a complementary deficiency in the host (*e.g*., thymidine kinase (tk) in tk- hosts). However, drug markers are also available (*e.g.*, G418 resistance and hygromycin resistance).

The sequence of nucleotides encoding the telomerase may also include a secretion signal, whereby the resulting peptide is a synthesized as precursor protein and is subsequently processed and secreted. The resulting processed protein may be recovered from periplasmic space or fermentation medium. Secretion signals suitable for use are widely available and are well known in the art (von Heijne, *J. Mol. Biol. 184*:99-105, 1985). Prokaryotic and eukaryotic secretion signals that are functional in *E. coli* (or other host) may be employed. The presently preferred secretion signals include, but are not limited to, those encoded by the following *E. coli* genes: pelB (Lei et al., *J. Bacteriol. 169*:4379,1987), phoA, ompA, ompT, ompF, ompC, beta-lactamase, and alkaline phosphatase.

One skilled in the art appreciates that there are a wide variety of suitable vectors for expression in bacterial cells and which are readily obtainable. Vectors such as the pET series (Novagen, Madison, WI), the tac and trc series (Pharmacia, Uppsala, Sweden), pTTQ18 (Amersham International plc, England), pACYC 177, pGEX series, and the like are suitable for expression of a telomerase. Baculovirus vectors, such as pBlueBac (*see, e.g.*, U.S. Patent Nos. 5,278,050, 5,244,805, 5,243,041, 5,242,687, 5,266,317, 4,745,051, and 5,169,784; available from Invitrogen, San Diego) may be used for expression of the telomerase in insect cells, such as *Spodoptera frugiperda* sf9 cells (*see,* U.S. Patent No. 4,745,051). The choice of a host for the expression of a telomerase is dictated in part by the vector. Commercially available vectors are paired with suitable hosts.

A wide variety of suitable vectors for expression in eukaryotic cells are available. Such vectors include pCMVLacI, pXT1 (Stratagene Cloning Systems, La Jolla, CA); pCDNA series, pREP series, pEBVHis (Invitrogen, Carlsbad, CA). In certain embodiments, telomerase gene is cloned into a gene targeting vector, such as pMC1neo, a pOG series vector (Stragene).

Telomerase protein is isolated by standard methods, such as affinity chromatography, size exclusion chromatography, metal ion chromatography, ionic exchange chromatography, HPLC, and other known protein isolation methods. (*see generally* Ausubel et al., *supra;* Sambrook et al., *supra*). An isolated purified protein gives a single band on SDS-PAGE when stained with Coomassie blue.

In one embodiment, the telomerase protein is expressed as a hexa-his fusion protein and isolated by metal-containing chromatography, such as nickel-coupled beads. Briefly, a sequence encoding His₆ is linked to a DNA sequence encoding a telomerase. Although the His₆ sequence can be positioned anywhere in the molecule, preferably it is linked at the 3' end immediately preceding the termination codon. The His-hTI fusion may be constructed by any of a variety of methods. A convenient method is amplification of the TEL gene using a downstream primer that contains the codons for His₆.

### F. Peptides and proteins of telomerase

In one aspect of the present invention, peptides having telomerase sequence are provided. Peptides may be used as immunogens to raise antibodies, as inhibitors or enhancers of telomerase function, in assays described herein and the like. Peptides are generally five to 100 amino acids long, and more usually 10 to 50 amino acids. Peptides are readily chemically synthesized in an automated fashion (PerkinElmer ABI Peptide Synthesizer) or may be obtained commercially. Peptides may be further purified by a variety of methods, including high-performance liquid chromatography. Furthermore, peptides and proteins may contain amino acids other than the 20 naturally occurring amino acids or may contain derivatives and modification of the amino acids.

Peptides of particular interest within the context of this invention have the sequence of the intron sequences (Figure 10), the RTase motifs, and the like. In certain embodiments, telomerase proteins have the amino acid sequences presented in Figures 1 or 11, or a portion thereof which is at least 8 amino acids in length (and may be 10, 15, 20 or more amino acids in length). In other embodiments, the protein has one or more amino acid substitutions, additions, deletions. In yet other embodiments, the protein has an amino acid sequence determined by a nucleic acid sequence that hybridizes under normal stringency conditions to the complement of any of the sequences in Figure 11. As indicated above, variants of telomerase include allelic variants.

### II. TELOMERASE ASSAYS

A variety of assays are available to determine telomerase activity and expression. Such assays include *in vitro* assays that measure the ability of telomerase to extend a telomeric DNA substrate, nucleolytic activity, primer (telomere) binding activity, dNTP binding activity, telomerase RNA (hTR) binding activity, *in vivo* gain-of-function assays, *in vivo* loss-of function assays, in situ hybridization, RNase probe protection, Northern analysis, amplification of cDNA, antibody staining, and the like.

### A. Assays for catalytic activity

Various assays for catalytic activity are described in U.S. Patent Nos. 5,629,154; 5,639,613; 5,645,986 among others. In one conventional assay for telomerase activity, a single-stranded DNA primer having the sequence of the host telomere (*e.g*., [TTAGGG]ₙ) and the telomerase enzyme are used (see Shay et al., *Methods in Molecular Genetics 5*:263, 1994; Greider and Blackburn, *Cell 43*:405, 1985; Morin, *Cell 59*:521, 1989; U.S. Patent No. 5,629,154). A preferred assay incorporates a detergent-based extraction with an amplification-based assay. This assay, called TRAP (telomeric repeats amplification protocol), has improved sensitivity (Kim et al., *Science 266*: 2011, 1994). Briefly, in TRAP, telomerase synthesizes extension products, which then serve as templates for amplification. The telomerase products are amplified with a primer derived from a non-telomeric region of the oligonucleotide and a primer derived from the telomeric region. When the amplification products are analyzed, such as by gel electrophoresis, a ladder of products is observed when telomerase activity is present. Permutations of this assay have been described (Krupp et al., *Nucl. Acids Res. 25:* 919, 1997; Savoysky et al., *Nucl. Acids Res. 24*: 1175, 1996). As well, other telomerase assays are available (Faraoni et al., *J. Chemother 8*: 394, 1996, describing *an in vitro* chemosensitivity assay; Tatematsu et al., *Oncogene 13:* 2265, 1996, describing a "stretch PCR assay"; Lin and Zakian, *Cell 81:* 1127, 1995, describing an *in vitro* assay for *Saccharomyces*)*.*

In addition, catalytic or other activities may be measured by an *in vitro* reconstitution system (see Examples). Briefly, the assays, such as those described herein, are performed using purified telomerase protein that is produced by recombinant meant and other necessary components, such as the telomerase RNA component, other proteins such as described in WO 98/14593.

### B. Assays for other activities

Nucleolytic activity may be assessed by protocols described for example in Collins and Grieder, *Genes and Development 7*: 1364, 1993). The nucleolytic activity is excision of a nucleotide (G from the telomeric repeat TTAGG) from the 3' end of a nucleotide sequence that is positioned at the 5' boundary of the DNA template. Briefly, the activity can be measured by a reaction that uses a nucleic acid template with a 3' nucleotide that is blocking, i.e., cannot serve as a primer for a polymerase, unless removed by nucleolytic activity.

Telomere binding activity and assays are described in for example Harrington et al., *J. Biol. Chem. 270:* 8893, 1995. In general, any assay such as a gel-shift assay, that detects protein-nucleic acid interactions may be used. DNTP and RNA binding activity assays are described in Morin, *Eur. J. Cancer 33:* 750 for example.

### C. Gain and loss of function

*In vivo* gain-of-function assays may be performed by transfecting an expression vector encoding telomerase into cells that have no or little detectable endogenous activity. Activity is then measured by an *in vitro* assay, such as those described herein. Another gain of function assay can be performed in tumor cells or other cells expressing telomerase or reverse transcriptase. A telomerase gene is transfected into the cells, expressed at high levels, and these cells are treated with inhibitors of reverse transcriptase. Telomerase activity is then observed as decreased sensitivity to such inhibitors. Furthermore, rescue of function in the yeast telomerase mutant EST2 may be measured.

Loss of function may be measured in cells expressing high levels of telomerase activity, such as LIM 1215 cells or other tumor cells. In this assay, antisense oligonucleotide molecules are introduced into the cells, generally in an expression vector. Telomerase gene is verified by diminished telomerase activity. In another assay, antibodies to telomerase that inhibit function can be used to demonstrate a functional molecule.

### D. Expression of telomerase

Expression of telomerase in various cells may be assayed by standard assays using the sequences provided herein. For example, in situ hybridization with radioactive or fluorescent-labeled probes (fragments or oligonucleotides) may be used on tissue sections or fixed cells. Alternatively, RNA may be isolated from the cells and used in Northern, RNase probe protection assays, and the like. Probes for particular regions and probes that are variant specific will generate expression profiles of the various telomerase transcripts.

In a preferred embodiment, telomerase expression is assayed by amplification. Primer pairs for telomerase, including primer pairs for particular variants, are used to amplify cDNA synthesized from cellular RNA. The cDNA may be synthesized from either total RNA or poly(A)+ RNA. Methods and protocols for RNA isolation are well known. The cDNA may be initiated by an oligo(dT) primer, random primers (*e.g.,* dN₆), telomerase specific primer, and the like. The choice of a primer will depend at least in part on the quantity of RNA and the purpose of the assay. Amplification primers are designed to amplify any one of, particular combinations, or all of the variants present in vertebrate cells. Conditions for amplification are chosen to be commensurate with the primer length, base content, length of amplified product and the like. Various amplification systems are available (see Lee et al., *Nucleic Acid Amplification Technologies,* BioTechniques Books, Eaton Publishing, Natick, MA, 1997; Larrick, *The PCR Technique: Quantitative PCR,* BioTechniques Books, Eaton Publishing, Natick, MA, 1997).

Other assays for measuring expression qualitatively and quantitatively are well known. RNase probe protection and Northern analysis are amenable when the amount of telomerase mRNA is sufficient. When very few cells are available, a single cell analysis is desirable, or when the fraction of telomerase RNA in the sample is very low, an amplification protocol is preferred. RNase probe protection, in particular, is well suited for detecting splice variants, mutations, as well as quantitating these RNAs.

As discussed above, in preferred embodiments, expression of the various RNA species is monitored. The different species may be assayed by any method which distinguishes one of the species over the others. Thus, length determination by Northern, RNase probe protection, cloning and amplification are some of the available methods. In preferred embodiments, RNase probe protection and amplification are used. For RNase probe protection, the probe will generally be a fragment derived from the junction of the reference sequence and the intron sequence or derived from the sequence surrounding the intron insertion site. For example, a fragment of the reference telomerase that spans nucleotide 1950-1951 (*e.g.*, nucleotides 1910-1980) will protect the reference sequence as a 71 base fragment, but will protect a telomerase with intron 1 as two fragments of 41 and 30 bases. In contrast, a fragment that contains nucleotides 1910-1950 and 30 bases of intron 1 will protect an intron 1 variant as a 71 base fragment and the reference telomerase as a 41 base fragment Fragments for RNase probe protection are chosen usually in the range of 30 to 400 bases and are positioned to yield readily distinguishable protection products.

Another method that can be used to distinguish variants is amplification. Amplification primer design and strategy are described above. Briefly, primers that will individually amplify each spliced-in or spliced-out variant are preferred. Multiple reactions can be performed to identify variants with more than one spice-in or splice-out event.

Methods that measure telomerase protein are also useful within the context of the present invention. By way of example, antibodies to telomerase may be used to stain tissue sections or permeabilized cells. Antibodies may also be used to detect protein by immunoprecipitation, Western blot and the like. Furthermore, subcellular localization of telomerase and telomerase variants may be determined using the antibodies described herein.

### E. Antibodies to telomerase

Antibodies to the telomerase proteins, fragments, or peptides discussed herein may readily be prepared. Such antibodies may specifically recognize wild type telomerase protein and not a mutant (or variant) protein, mutant (or variant) telomerase protein and not wild type protein, or equally recognize both the mutant (or variant) and wild-type forms. Antibodies may be used for isolation of the protein, inhibiting (antagonist) activity of the protein, or enhancing (agonist) activity of the protein. As well, assays for small molecules that interact with telomerase will be facilitated by the development of antibodies.

Within the context of the present invention, antibodies are understood to include monoclonal antibodies, polyclonal antibodies, anti-idiotypic antibodies, antibody fragments (*e.g*., Fab, and F(ab')₂, F_{V} variable regions, or complementarity determining regions). Antibodies are generally accepted as specific against telomerase protein if they bind with a K_{d} of greater than or equal to 10⁻⁷M, preferably greater than of equal to 10⁻⁸M. The affinity of a monoclonal antibody or binding partner can be readily determined by one of ordinary skill in the art (*see* Scatchard, *Ann. N. Y. Acad. Sci. 51*:660-672, 1949).

Briefly, a polyclonal antibody preparation may be readily generated in a variety of warm-blooded animals such as rabbits, mice, or rats. Typically, an animal is immunized with telomerase protein or peptide thereof, which is preferably conjugated to a carrier protein, such as keyhole limpet hemocyanin. Routes of administration include intraperitoneal, intramuscular, intraocular, or subcutaneous injections, usually in an adjuvant (e.g., Freund's complete or incomplete adjuvant). Particularly preferred polyclonal antisera demonstrate binding in an assay that is at least three times greater than background.

Monoclonal antibodies may also be readily generated from hybridoma cell lines using conventional techniques (*see* U.S. Patent Nos. RE 32,011, 4,902,614, 4,543,439, and 4,411,993; *see also Antibodies: A Laboratory Manual,* Harlow and Lane (eds.), Cold Spring Harbor Laboratory Press, 1988). Briefly, within one embodiment, a subject animal such as a rat or mouse is injected with telomerase or a portion thereof. The protein may be administered as an emulsion in an adjuvant such as Freund's complete or incomplete adjuvant in order to increase the immune response. Between one and three weeks after the initial immunization the animal is generally boosted and may tested for reactivity to the protein utilizing well-known assays. The spleen and/or lymph nodes are harvested and immortalized. Various immortalization techniques, such as mediated by Epstein-Barr virus or fusion to produce a hybridoma, may be used. In a preferred embodiment, immortalization occurs by fusion with a suitable myeloma cell line to create a hybridoma that secretes monoclonal antibody. Suitable myeloma lines include, for example, NS-1 (ATCC No. TIB 18), and P3X63 - Ag 8.653 (ATCC No. CRL 1580). The preferred fusion partners do not express endogenous antibody genes. Following fusion, the cells are cultured in medium containing a reagent that selectively allows for the growth of fused spleen and myeloma cells such as HAT (hypoxanthine, aminopterin, and thymidine). After about seven days, the hybridomas may be screened for the presence of antibodies that are reactive against a telomerase protein. A wide variety of assays may be utilized, including for example countercurrent immuno-electrophoresis, radioimmunoassays, radioimmunoprecipitations, enzyme-linked immuno-sorbent assays (ELISA), dot blot assays, western blots, immunoprecipitation, inhibition or competition assays, and sandwich assays (*see* U.S. Patent Nos. 4,376,110 and 4,486,530; *see also Antibodies: A Laboratory Manual,* Harlow and Lane (eds.), Cold Spring Harbor Laboratory Press, 1988).

Other techniques may also be utilized to construct monoclonal antibodies (*see* Huse et al., *Science 246*:1275-1281, 1989; Sastry et al., *Proc. Natl. Acad Sci. USA 86*:5728-5732, 1989; Alting-Mees et al., *Strategies in Molecular Biology 3*:1-9, 1990; describing recombinant techniques). Briefly, mRNA is isolated from a B cell population and utilized to create heavy and light chain immunoglobulin cDNA expression libraries in suitable vectors, such as λImmunoZap(H) and λImmunoZap(L). These vectors may be screened individually or co-expressed to form Fab fragments or antibodies (*see* Huse et al., *supra;* Sastry et al., *supra*). Positive plaques may subsequently be converted to a non-lytic plasmid that allows high level expression of monoclonal antibody fragments from *E. coli.*

Similarly, portions or fragments, such as Fab and Fv fragments, of antibodies may also be constructed utilizing conventional enzymatic digestion or recombinant DNA techniques to yield isolated variable regions of an antibody. Within one embodiment, the genes which encode the variable region from a hybridoma producing a monoclonal antibody of interest are amplified using nucleotide primers for the variable region. These primers may be synthesized by one of ordinary skill in the art, or may be purchased from commercially available sources (*e.g.*, Stratacyte, La Jolla, CA) Amplification products are inserted into vectors such as ImmunoZAP™ H or ImmunoZAP™ L (Stratacyte), which are then introduced into *E. coli,* yeast, or mammalian-based systems for expression. Utilizing these techniques, large amounts of a single-chain protein containing a fusion of the V_{H} and V_{L} domains may be produced (*see* Bird et al., *Science 242*:423-426, 1988). In addition, techniques may be utilized to change a "murine" antibody to a "human" antibody, without altering the binding specificity of the antibody.

Once suitable antibodies have been obtained, they may be isolated or purified by many techniques well known to those of ordinary skill in the art (*see Antibodies: A Laboratory Manual,* Harlow and Lane (eds.), Cold Spring Harbor Laboratory Press, 1988). Suitable techniques include peptide or protein affinity columns, HPLC or RP-HPLC, purification on protein A or protein G columns, or any combination of these techniques.

### F. Proteins that interact with telomerase

Proteins that directly interact with telomerase can be detected by an assay such as a yeast 2-hybrid binding system. Briefly, in a two-hybrid system, a fusion of a DNA-binding domain-telomerase protein (*e.g.*, GAL4-telomerase fusion) is constructed and transfected into a cell containing a GAL4 binding site linked to a selectable marker gene. The whole telomerase protein or subregions of telomerase may be used. A library of cDNAs fused to the GAL4 activation domain is also constructed and co-transfected. When the cDNA in the cDNA-GAL4 activation domain fusion encodes a protein that interacts with telomerase, the selectable marker is expressed. Cells containing the cDNA are then grown, the construct isolated and characterized. Other assays may also be used to identify interacting proteins. Such assays include ELISA, Western blotting, co-immunoprecipitations and the like.

### III. INHIBITORS AND ENHANCERS OF TELOMERASE ACTIVITY

Candidate inhibitors and enhancers (collectively referred to as "effectors") may be isolated or procured from a variety of sources, such as bacteria, fungi, plants, parasites, libraries of chemicals (*e.g.*, combinatorial libraries), random peptides or the like. Effectors may also be peptides or variant peptides of telomerase, variants of telomerase, antisense nucleic acids, antibodies to telomerase, inhibitors of promoter activity of telomerase, and the like. Inhibitors and enhancers may be also be rationally designed, based on the protein structure determined from X-ray crystallography (see, Livnah et al., *Science 273*:464, 1996). In certain preferred embodiments, the inhibitor targets a specific telomerase, such as a variant.

An inhibitor may act by preventing binding of telomerase to other components of the ribonucleoprotein complex or to the telomere, by causing dissociation of the bound proteins, or by other mechanism. An inhibitor may act directly or indirectly. In preferred embodiments, inhibitors interfere in the binding of the telomerase protein to either the telomerase RNA or to the telomeres. In other preferred embodiments, the inhibitors are small molecules. In a most preferred embodiment, the inhibitors cause a cell to cease replication. Inhibitors should have a minimum of side effects and are preferably non-toxic. Inhibitors that can penetrate cells are preferred.

In other preferred embodiments, an effector is a protein or peptide of telomerase that acts in a dominant negative fashion (see, Ball et al., *Current Biology 7*:71, 1997; *Current Biology 6*:84, 1996). For example, a peptide of telomerase that competitively inhibits the binding of telomerase to telomeres will disrupt the lengthening of telomeres. Generally, these peptides have native sequence, but variants may have increased activity (see, Ball et al., *supra*). Variants may be constructed by the methods described herein. Other peptides may bind telomerase and inhibit one or more of its activities, but do not have telomerase amino acid sequence. Such peptides may be identified by the assays described herein. The proteins or peptides may also increase telomerase activity. For effective inhibition, peptide inhibitors are preferably expressed from vectors transfected or infected into host cells, but may also be introduced by other means, such as liposome-mediated fusion, and the like. Eukaryotic vectors are well known and readily available. Vectors include plasmids, viral-based vectors, and the like.

In another preferred embodiment, the inhibitor is a ribozyme. "Ribozyme" refers to a nucleic acid molecule which is capable of cleaving a telomerase nucleic acid sequence. Ribozymes may be composed of DNA, RNA, nucleic acid analogues, or any combination of these (*e.g.*, DNA/RNA hybrids). A "ribozyme gene" refers to a nucleic acid molecule which, when transcribed into RNA, yields the ribozyme, and a "ribozyme vector" refers to an assembly that is capable of transcribing a ribozyme gene of interest, and may be composed of either DNA or RNA. Within certain embodiments of the invention, the vector may include one or more restriction site(s) and selectable marker(s). Furthermore, depending on the choice of vector and host cell, additional elements such as an origin of replication, polyadenylation site, and enhancers may be included in the vectors described herein.

As noted above, the present invention also provides ribozymes having the ability to inhibit expression of the telomerase gene. Briefly, a wide variety of ribozymes may be generated for use within the present invention, including for example, hairpin ribozymes (*see e.g.,* Hampel et al., *Nucl. Acids Res. 18*:299-304, 1990, EPO 360,257, and U.S. Patent No. 5,254,678), hammerhead ribozymes (*see e.g.,* Rossi, J.J. et al., *Pharmac. Ther. 50*:245-254, 1991; Forster and Symons, *Cell 48*:211-220, 1987; Haseloff and Gerlach, *Nature 328*:596-600, 1988; Walbot and Bruening, *Nature 334*:196, 1988; Haseloff and Gerlach, *Nature 334*:585, 1988; Haseloff et al., U.S. Patent No. 5,254,678), hepatitis delta virus ribozymes (see, e.g, Perrotta and Been, *Biochem. 31*:16, 1992), Group I intron ribozymes such as those based upon the *Tetrahymena* ribosomal RNA (see, e.g, Cech et al., U.S. Patent No. 4,987,071) RNase P ribozymes (see, e.g, Takada et al., *Cell 35*:849, 1983); as well as a variety of other nucleic acid structures with the capability to cleave a desired or selected target sequence (*see e.g.,* WO 95/29241, and WO 95/31551). Within certain embodiments of the invention, the ribozymes may be altered from their traditional structure in order to include tetraloops or other structures that increase stability (*see, e.g.,* Anderson et al., *Nucl. Acids Res. 22*:1096-1100, 1994; Cheong et al., *Nature 346*:680-682, 1990), or which make the ribozyme resistant to RNase or endonuclease activity (*see e.g.,* Rossi et al., *Pharmac. Ther. 50*:245-254, 1991).

Within one embodiment of the invention, hairpin and hammerhead ribozymes are provided with the capability of cleaving telomerase nucleic acid sequences. Briefly, hairpin ribozymes are generated so that they recognize the target sequence N₃XN*GUC(N_{>6}), wherein N is G, U, C, or A, X is G, C, or U, and * is the cleavage site. Similarly, hammerhead ribozymes are generated so that they recognize the sequence NUX, wherein N is G, U, C, or A. The additional nucleotides of the hammerhead ribozyme or hairpin ribozyme is determined by the target flanking nucleotides and the hammerhead consensus sequence (*see* Ruffner et al., *Biochemistry 29*:10695-10702, 1990). The preparation and use of certain ribozymes is described in Cech et al. (U.S. Patent No. 4,987,071). The ribozymes are preferably expressed from a vector introduced into the host cells.

Ribozymes of the present invention, as well as DNA encoding such ribozymes can be readily generated utilizing published protocols (*e.g*., Promega, Madison Wis., Heidenreich et al., *J. FASEB 70*:90-6, 1993; Sproat, *Curr. Opin. Biotechnol. 4*:20-28, 1993). Alternatively, ribozymes may be generated from a DNA or cDNA molecule which encodes a ribozyme and which is operably linked to a RNA polymerase promoter (*e.g.*, SP6 or T7). An RNA ribozyme is generated upon transcription of the DNA or cDNA molecule.

In other preferred embodiments, inhibitors diminish promoter activity of telomerase. A eukaryotic promoter comprises sequences bound by RNA polymerase and other proteins participating in control of the transcription unit. Telomerase transcription appears to be highly regulated; the protein is expressed mainly in stem, embryonic, and cancer cells, and expressed at much lower levels, if at all, in most somatic cells. Thus, the promoter is a potential target for inhibitors. The inhibitors may disrupt or prevent binding of one or more of the factors that control transcription of telomerase, causing transcription to diminish or cease. The levels of transcription need only fall to a low enough level that at least one telomere becomes absent.

Another inhibitor of the present invention is antisense RNA or DNA to telomerase coding or non-coding sequence. Antisense nucleic acids directed to a particular mRNA molecule have been shown to inhibit protein expression of the encoded protein Based upon the telomerase sequences presented herein, an antisense sequence is designed and preferably inserted into a vector suitable for transfection into host cells and expression of the antisense. The antisense may bind to any part of the hTI RNA. In certain embodiments, the antisense is designed to bind specifically to one or more variants. Specific binding means that under physiological conditions, the antisense binds to RNAs that have the complementary sequence, but not other RNAs. Because telomerase RNAs that contain any particular intron' sequence may be a heterogeneous group of variants due to independent assortment of splice variants, more than one species of RNA may be bound and inactivated. The antisense polynucleotides herein are at least 7 nucleotides long and generally not longer than 100 to 200 bases, and are more typically at least 10 to 50 bases long. Considerations for design of antisense molecules and means for introduction into cells are found in U.S. Patent Nos. 5,681,747; 5,734,033; 5,767,102; 5,756,476; 5,749,847; 5,747,470; 5,744,362; 5,716,846).

In addition, enhancers of telomerase activity or expression are desirable in certain circumstances. At times, increasing the proliferation potential of cells will have a therapeutic effect. For example, organ regeneration or differentiation after injury or diseases, nerve cell or brain cell growth following injury, proliferation of hematopoietic stem cells used in bone marrow transplantation or other organ stem cells, and the like may be limiting and thus benefit from an enhancer of telomerase. Enhancers may stabilize endogenous protein, increase transcription or translation, or act through other mechanisms. As is apparent to one skilled in the art, many of the guidelines presented above apply to the design of enhancers as well.

Screening assays for inhibitors and enhancers will vary according to the type of inhibitor and nature of the activity that is being inhibited. Assays include the TRAP assay or variation, a non-amplification based polymerase assay, yeast two-hybrid, release of repression in yeast transfected with a vertebrate telomerase, and the like. For screening compounds that interact with the promoter for telomerase, a reporter gene driven assay is convenient.

### IV. USES FOR TELOMERASE

Nucleotide sequence for telomerase and telomerase protein are used in a variety of contexts in this invention. In preferred embodiments, the compositions of the present invention are used either as diagnostic reagents or as therapeutics.

### A. Diagnostics

Expression of mRNA encoding telomerase and/or protein may be used for detection of dividing cells, especially tumor cells and stem cells. Detection methods include antibody staining or tagged telomerase binding compounds for detection of protein, nucleic acid hybridization in situ for mRNA, hybridization on DNA "chips", Northern analysis, RNase probe protection, amplification by PCR or other method, ligase-mediated amplification and the like. Furthermore, expression of RNA splice variants may be assayed conveniently by amplification, RNase probe protection, other disclosed methods and the like. In particular, oligonucleotide primers surrounding the site of frequent splice variants, such as the primers described herein (*e.g*., Htel Intron T and HT 2482R) may be used to detect splice variants in various cell types. As shown in the examples, various tumor cell types exhibit different RNA splice variations. Correlation of the splice variant pattern with tumor stage, metastasis potential and the like may be determined. As such, assays for the particular variants may be used as a diagnostic. Cells with increased telomerase activity, such as cancer cells or hyperproliferative cells, may be identified by assaying qualitatively or quantitatively by any of the assays described herein. Typically, telomerase activity or expression will be compared between suspect cells and normal counterpart cells from the same or different individual. Increased activity indicative of a tumor or excessive proliferation is established by direct comparison or by detecting activity in cells otherwise known to be absent in telomerase activity or expression. In addition, monitoring cancer progression or response to therapy can be performed using the assays described herein and comparing activity or expression over a time course.

The variant detected in one ALT cell line, which expresses telomerase, suggests that the basic domain of hT1 may contribute to the ALT mechanism in at least some ALT cell lines. One possible mechanism of ALT could involve dysregulated telomerase components that are inactive in the TRAP assay. Thus, identification of the variants may be useful for following tumorigenesis.

Alternative mRNA splicing is a common mechanism for regulating gene expression in higher eukaryotes and there are many examples of tissue-specific, development-specific and sex-specific alterations in splicing events. Importantly, 15% of mutations linked to disease states in mammals affect splicing patterns (Horowitz and Krainer *Trends Genet., 10,* 100-106, 1994). Changes in cell physiology can also induce altered splicing patterns. Indeed, tumorigenesis itself has been suggested to enhance the expression of mRNA spliced variants by compromising the alternative splicing mechanisms. Although other, novel minor alternatively spliced hT1 variants may play a role in tumor development, the altered relative expression levels of the major transcripts found in various tumors compared to normal cells, and in post-crisis cell lines compared to limited life-span pre-crisis cells, are likely to play a major role in the establishment and progression of cancers. In addition, the existence of the alternative spliced variants of hT1 that are seen in both testis and colonic crypt, as well as tumor cell lines, suggests complex regulation of this gene in normal development.

Expression of the major hT1 products is found in most tumors and in all telomerase-positive immortalized cell lines. Transcriptional control of hT1 may therefore be a major aspect of the regulation of telomerase activity, in addition to other functions. For example, telomerase may be involved in the healing of chromosome breaks in addition to its role in maintaining telomere length in the germline. The composition of telomerase may vary according to these functional roles.

Therefore, the intron sequences may be especially useful for diagnostic applications. For example, detection and identification of diseases, such as cancer, aging, wound healing, neuronal regeneration, regenerative cells (*e.g.*, stem cells), may be important preludes to determining effective therapy. In this regard, detection of wound healing can facilitate development and identification of an ameliorative compound. Currently, wound healing assays are expensive and time consuming, whereas an amplification or hybridization-based assay would be quick and cost effective. In any of these applications, detection may be quantitative or qualitative. In a qualitative assay, a particular amplification primer pair or hybridization probe for one of the variant sequences (*e.g.*, introns that are variably spliced) can be used to detect the presence or absence of the variant sequence.

Probes useful in the context of the present invention include nucleic acid molecules that hybridize to the sequences presented in Figure 10 or to their complements. Probes for hybridization are generally at least 24 bases, but may range from 12 to full-length sequence. The probes may comprise additional sequence that does not hybridize to hT1 DNA or RNA. Probes are generally DNA, but may be RNA, PNA, or derivatives thereof. Hybridization conditions will be chosen appropriate for the length of the probe and method of hybridization (*e.g*., on nylon support, on silicon-based chip). Conditions are well known in the art. One of the sequences in Figure 10 is a genomic sequence, not found in telomerase mRNA. A probe derived from this sequence may be used to detect genomic DNA in RNA preparations and amplification reactions. Hybridization probes may be labeled with a radiolabel, chemiluminescent label, or any of the myriad other known labels.

Hybridization can be performed on mRNA preparations, cDNA preparations, affixed to a solid support, in solution, or in situ tissues, and the like. One type of hybridization analysis is annealing to oligonucleotides immobilized on a solid substrate, such as a functionalized glass slide or silicon chip. Such chips may be commercially procured or made according to methods and procedures set out in e.g., PCT/US94/12282; U.S. Patent No. 5,405,783; U.S. Patent No. 5,412,087; U.S. Patent No. 5,424,186; U.S. Patent No. 5,436,327; U.S. Patent No. 5,429,807; U.S. Patent No. 5,510,270; WO 95/35505; U.S. Patent No. 5,474,796. Oligonucleotides are generally arranged in an array form, such that the position of each oligonucleotide sequence can be determined.

For amplification assays, primer pairs that either flank the introns or require the presence of the intron for amplification are desirable. Many such primer pairs are disclosed herein. Others may be designed from the sequences presented herein. Generally, the primer pairs are designed to only allow amplification of a single intron, however, in some circumstances detection of multiple introns in the same RNA preparation may be preferred.

Other diagnostic assays, such as in situ hybridization, RNase protection, and the like may be used alternatively or in addition to the assays discussed above. The principles that guide these assays are provided by the present invention, while the techniques are well known.

Transgenic mice and mice that are null mutants (*e.g.*, "knockout mice") may be constructed to facilitate testing of candidate inhibitors. The telomerase gene is preferably under control of a tissue-specific promoter for transgenic mice vector constructs. Mice that overexpress telomerase can be used as a model system for testing inhibitors. In these mice, cells overexpressing telomerase are expected to be continuously proliferating. Administration of candidate inhibitors is followed by observation and measurement of cell growth. Inhibitors that slow or diminish growth are candidate therapeutic agents.

Telomerase may also be transfected into cells to immortalize various cell types. Transient immortalization may be achieved by non-stable transfection of an expression vector containing telomerase. Alternatively, proliferation of stable transformants of telomerase gene under control of an inducible promoter can be turned on and off by the addition and absence of the inducer. Similarly, the presence and absence of an inhibitor of telomerase activity may be used to selectively immortalize cells. Expression of part of all of the protein in yeast may act as a dominant negative, as many human proteins interact with components of a complex in yeast, but do so imperfectly and therefore unproductively. As such, these genes act as dominant negatives. Thus, the yeast will eventually senesce. Such cells may be used in screens for inhibitory drugs, which will allow growth of yeast past the time of senescence.

Purified telomerase protein, reference variant protein, or fragments, may be used in assays to screen for inhibitory drugs. These assays will typically be *performed in vitro* and utilize any of the methods described above or that are known in the art. The protein may also be crystallized and subjected to X-ray analysis to determine its 3-dimensional structure.

### B. Therapeutics

The compositions and methods disclosed herein may also be used as therapeutics in the treatment of diseases and disorders to effect any of the telomerase activities in a cell. Treatment means any amelioration of the disease or disorder, such as alleviating symptoms of the disease or disorder, reduction of tumor cell mass and the like. For example, inhibitors of enzyme activity may be used to restrict proliferation of cells.

Many diseases and disorders are tightly associated with proliferation and proliferative potential. One of the most apparent diseases involving unwanted proliferation is cancer. The methods and compositions described herein may be used to treat cancers, such as melanomas, other skin cancers, neuroblastomas, breast carcinomas, colon carcinomas, leukemias, lymphomas, osteosarcomas, and the like. Other diseases and disorders amenable for treatment within the context of the present invention include those of excessive cell proliferation (increased proliferation rate over normal counterpart cells from the same or different individual) such as smooth muscle cell hyperplasias, skin growths, and the like. Yet other diseases and disorders would benefit from increased telomerase activity. Enhancers of telomerase may be used to stimulate stem cell proliferation and possibly differentiation. As such, expansion of hematopoietic stem cells could be administered in the bone marrow transplant context. As well, many tissues have stem cells. Proliferation of these cells may be beneficial for wound healing, hair growth, treatment of diseases, such as Wilm's tumor, and the like.

Certain of the inhibitors or enhancers may be administered by way of an expression vector. Many techniques for introduction of nucleic acids into cells are known. Such methods include retroviral vectors and subsequent retrovirus infection, adenovirals or adeno-associated viral vectors and subsequent infection, complexes of nucleic acid with a condensing agent (*e.g.*, poly-lysine), these complexes or viral vectors may be targeted to particular cell types by way of an incorporated ligand. Many ligands specific for tumor cells and other cells are well known in the art.

As noted above, within certain aspects of the present invention, nucleic acids encoding ribozymes, antisense, dominant-negative telomerases, portions of telomerase and the like may be utilized to inhibit telomerase activity by introducing a functional gene to a cell of interest. This may be accomplished by either delivering a synthesized gene to the cell or by delivery of DNA or cDNA capable of *in vivo* transcription of the gene product. More specifically, in order to produce products *in vivo,* a nucleic acid sequence coding for the product is placed under the control of a eukaryotic promoter (*e.g*., a pol III promoter, CMV or SV40 promoter). Where it is desired to more specifically control transcription, the gene may be placed under the control of a tissue or cell specific promoter (*e.g.,* to target cells in the liver), or an inducible promoter.

A wide variety of vectors may be utilized within the context of the present invention, including for example, plasmids, viruses, retrotransposons and cosmids. Representative examples include adenoviral vectors (*e.g.*, WO 94/26914, WO 93/9191; Yei et al., *Gene Therapy 1*:192-200, 1994; Kolls et al., *PNAS 91*(1):215-219, 1994; Kass-Eisler et al., *PNAS 90*(24):11498-502, 1993; Guzman et al., *Circulation 88*(6):2838-48, 1993; Guzman et al., *Cir. Res. 73*(6):1202-1207, 1993; Zabner et al., *Cell 75*(2):207-216, 1993; Li et al., *Hum Gene Ther. 4*(4):403-409, 1993; Caillaud et al., *Eur. J. Neurosci. 5*(10):1287-1291, 1993), adeno-associated type 1 ("AAV-1") or adeno-associated type 2 ("AAV-2") vectors (*see* WO 95/13365; Flotte et al., *PNAS 90*(22):10613-10617, 1993), hepatitis delta vectors, live, attenuated delta viruses and herpes viral vectors (*e.g.*, U.S. Patent No. 5,288,641), as well as vectors which are disclosed within U.S. Patent No. 5,166,320. Other representative vectors include retroviral vectors (*e.g.*, EP 0 415 731; WO 90/07936; WO 91/02805; WO 94/03622; WO 93/25698; WO 93/25234; U.S. Patent No. 5,219,740; WO 93/11230; WO 93/10218. For methods and other compositions, see U.S. Patent Nos. 5,756,264; 5,741,486; 5,733,761; 5,707,618; 5,702,384; 5,656,465; 5,547,932; 5,529,774; 5,672,510; 5,399,346, and 5,712,378.)

Within certain aspects of the invention, nucleic acid molecules may be introduced into a host cell utilizing a vehicle, or by various physical methods. Representative examples of such methods include transformation using calcium phosphate precipitation (Dubensky et al., *PNAS 81*:7529-7533, 1984), direct microinjection of such nucleic acid molecules into intact target cells (Acsadi et al., *Nature 352*:815-818, 1991), and electroporation whereby cells suspended in a conducting solution are subjected to an intense electric field in order to transiently polarize the membrane, allowing entry of the nucleic acid molecules. Other procedures include the use of nucleic acid molecules linked to an inactive adenovirus (Cotton et al., *PNAS 89*:6094, 1990), lipofection (Felgner et al., *Proc. Natl. Acad Sci. USA 84*:7413-7417, 1989), microprojectile bombardment (Williams et al., *PNAS 88*:2726-2730, 1991), polycation compounds such as polylysine, receptor specific ligands, liposomes entrapping the nucleic acid molecules, spheroplast fusion whereby *E. coli* containing the nucleic acid molecules are stripped of their outer cell walls and fused to animal cells using polyethylene glycol, viral transduction, (Cline et al., *Pharmac. Ther. 29*:69, 1985; and Friedmann et al., *Science 244*:1275, 1989), and DNA ligand (Wu et al, *J. of Biol. Chem. 264*:16985-16987, 1989), as well as psoralen inactivated viruses such as Sendai or Adenovirus. In one embodiment, the nucleic acid molecule is introduced into the host cell using a liposome.

Administration of effectors will generally follow established protocols. The compounds of the present invention may be administered either alone, or as a pharmaceutical composition. Briefly, pharmaceutical compositions of the present invention may comprise one or more of the inhibitors or enhancers as described herein, in combination with one or more pharmaceutically or physiologically acceptable carriers, diluents or excipients. Such compositions may comprise buffers such as neutral buffered saline, phosphate buffered saline and the like, carbohydrates such as glucose, mannose, sucrose or dextrans, mannitol, proteins, polypeptides or amino acids such as glycine, antioxidants, chelating agents such as EDTA or glutathione, adjuvants (*e.g.,* aluminum hydroxide) and preservatives. In addition, pharmaceutical compositions of the present invention may also contain one or more additional active ingredients. Effectors may be further coupled with a targeting moiety that binds a cell surface receptor specific to the proliferating cells.

Compositions of the present invention may be formulated for the manner of administration indicated, including for example, for oral, nasal, venous, intracranial, intraperitoneal, subcutaneous, or intramuscular administration. Within other embodiments of the invention, the compositions described herein may be administered as part of a sustained release implant. Within yet other embodiments, compositions of the present invention may be formulized as a lyophilizate, utilizing appropriate excipients which provide stability as a lyophilizate, and subsequent to rehydration.

As noted above, pharmaceutical compositions also are provided by this invention. These compositions contain any of the above described ribozymes, DNA molecules, proteins, chemicals, vectors, or host cells, along with a pharmaceutically or physiologically acceptable carrier, excipients or diluents. Generally, such carriers should be nontoxic to recipients at the dosages and concentrations employed. Ordinarily, the preparation of such compositions entails combining the therapeutic agent with buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with nonspecific serum albumin are exemplary appropriate diluents.

In addition, the pharmaceutical compositions of the present invention may be prepared as medicaments for administration by a variety of different routes, including for example intraarticularly, intracranially, intradermally, intrahepatically, intramuscularly, intraocularly, intraperitoneally, intrathecally, intravenously, subcutaneously or even directly into a tumor. In addition, pharmaceutical compositions of the present invention may be placed within containers, along with packaging material which provides instructions regarding the use of such pharmaceutical compositions. Generally, such instructions will include a tangible expression describing the reagent concentration, as well as within certain embodiments, relative amounts of excipient ingredients or diluents (e.g., water, saline or PBS) which may be necessary to reconstitute the pharmaceutical composition. Pharmaceutical compositions are useful for both diagnostic or therapeutic purposes.

Pharmaceutical compositions of the present invention may be administered in a manner appropriate to the disease to be treated (or prevented). The quantity and frequency of administration will be determined by such factors as the condition of the patient, and the type and severity of the patient's disease. Dosages may be determined most accurately during clinical trials. Patients may be monitored for therapeutic effectiveness by appropriate technology, including signs of clinical exacerbation, imaging and the like.

The following examples are offered by way of illustration, and not by way of limitation.

### EXAMPLES

### EXAMPLE 1

### IDENTIFICATION AND ISOLATION OF THE HUMAN TELOMERASE GENE

A human telomerase gene is identified in a cDNA library constructed from a cancer cell line. The cDNA is subjected to DNA sequence analysis (Kilian et al., *supra*).

An EST sequence, GenBank Accession No. AA281296, is identified as partial telomerase gene sequence by a BLAST search against the *Euplotes* telomerase sequence, GenBank Accession No. U95964 (p=3.2 x 10⁻⁶). Amino acid sequence identity between the two sequences is approximately 38% and amino acid sequence similarity is approximately 60%.

To obtain longer clones of hT1, a number of cDNA libraries prepared from tumor cells are screened by amplification using primers from within the EST sequence. Primers HT1553F and HT1920R, based on the EST sequence, are used to amplify an approximately 350 bp fragment in a variety of cDNA libraries. The amplification reaction is performed under "hot start" conditions. Amplification cycles are 4 min at 95°C; 1 min at 80°C; 30 cycles of 30 sec at 94°C, 30 sec at 55°C, 1 min at 72°C°; and 5 min at 72°C. An amplified product of the expected size (~350 bp) is detected in only 3 of the 12 libraries screened. No fragment is detectable in a testis cDNA library, somatic cell libraries, and a variety of cancer cell cDNA libraries. However, an abundant 350 bp fragment is detected in a cDNA library from LIM 1215 cells, a colon cancer cell line. In this library, and in several others, an additional fragment of around 170 bp was amplified.

Two approaches are followed to obtain longer clones from the LIM1215 library: screening plaques with a ³²P-labeled EST probe and amplification on library DNA. A single positive plaque, designated 53.2, with a 1.9 kb insert is obtained by hybridization of the library with the EST probe. DNA sequence analysis of this clone demonstrates that it extends both 5' and 3' of the EST sequence, but did not contain a single open reading frame (ORF). A fragment obtained from amplification analysis of the library is similar in sequence to the 53.2 fragment but also contains two additional sequences of 36bp and >300bp. Both insertions demonstrate characteristics of splice acceptor and donor sequences at their boundaries relative to the 53.2 sequence and may represent unspliced introns. Amplification using primers T7 and HT1553F, yields an approximately 1.6 kb fragment; and using primers T3 and HT1893R, yields an approximately 0.7 kb fragment. Each of these fragments support amplification of a 320 bp fragment using primers HTEL1553F and HT1893R.

Longer clones may also be obtained by amplification of mRNA samples. Reverse transcriptase PCR (RT-PCR) on LIM1215 mRNA identifies a number of additional PCR products, including one with a 182 bp insertion relative to 53.2 that results in a single open reading frame (ORF). cDNA is synthesized from RNAs isolated from normal and tumor tissues. RT-PCR followed by nested amplification is performed using the Titan RT-PCR system (Boehringer-Mannheim). Amplification conditions are as follows: 95°C for 2 min, two cycles of 94°C for 30 sec, 65°C for 30 sec and 68°C for 3 min, 2 cycles of 94°C for 30 sec, 63°C for 30 sec, 68°C for 3 min, 34 cycles of 94°C for 30 sec, 60°C for 30 sec and 68°C for 3 min. RT-PCR products are diluted 100 fold, and 1 µl is used for nested amplification using *Taq* polymerase with buffer Q (Qiagen). Amplification conditions are as above, except that the final step is 14 cycles. For normal tissues and tumors, amplification products are resolved by electrophoresis in 1.5% agarose gel, transferred to Zetaprobe membrane and probed with radiolabeled oligonucleotide HT1691F.

The DNA sequence is also extended 5' and 3' using a combination of cRACE and 3' RACE, respectively, on L1M1215 mRNA to give a fragment of 3871 bp designated hT1 (Figure 1). Two rounds of cRACE are carried out to extend the sequence of hT1 and map the transcription initiation site. 500 ng LIM1215 polyA+ RNA is used as the template. First strand cDNA synthesis is primed using the HT1576R primer. The first round of amplification on the ligation product (using the XL-PCR system) employs the HT1157R and HT1262F primers. Amplification products are purified using Qiagen columns, and further amplified using primers HT1114R and HT1553F. A resulting 1.4 kb band is subjected to DNA sequence analysis, and a new set of primers are designed based on this sequence. For the second round of cRACE, the first strand cDNA is primed with the HT220R primer. The first round of amplification utilizes the HT0142R and HT0141F primers. Products are purified as above and amplified using HT0093 and HT0163F primers. A product of 100 bp is observed and subjected to sequence analysis in two independent experiments to define the 5' end of the hT1 transcript. The 5' end of the transcript is also obtained by amplification using primer HtelFulcodT 5'-AGGAGATCTCGCGATGCCGCGCGCTC-3' and HtelFulcodB 5'-TCCACGCGTCCTGCCCGGGTG-3' on LIM1215 RNA. The resulting amplified product was digested with Mlu I and Bgl II and ligated to the remaining telomerase cDNA sequence.

The 3'-most sequences of the transcript are obtained by two rounds of amplification (XL-PCR system) using EBHT18 in both rounds as the reverse primer, and HT2761 F and HT3114F as the forward primers in the first and second rounds, respectively.

The size of hT1 accords well with the size estimated from the Northern blot (see below) for the most abundant RNA species in LIM1215 RNA. Approximately 3.9 kb of DNA sequence is presented in Figure 1. The sequence found in the EST is located from nucleotides 1624-2012. The predicted amino acid sequence of the largest open reading frame is also presented in Figure 1. As presented, the protein is 1132 amino acids.

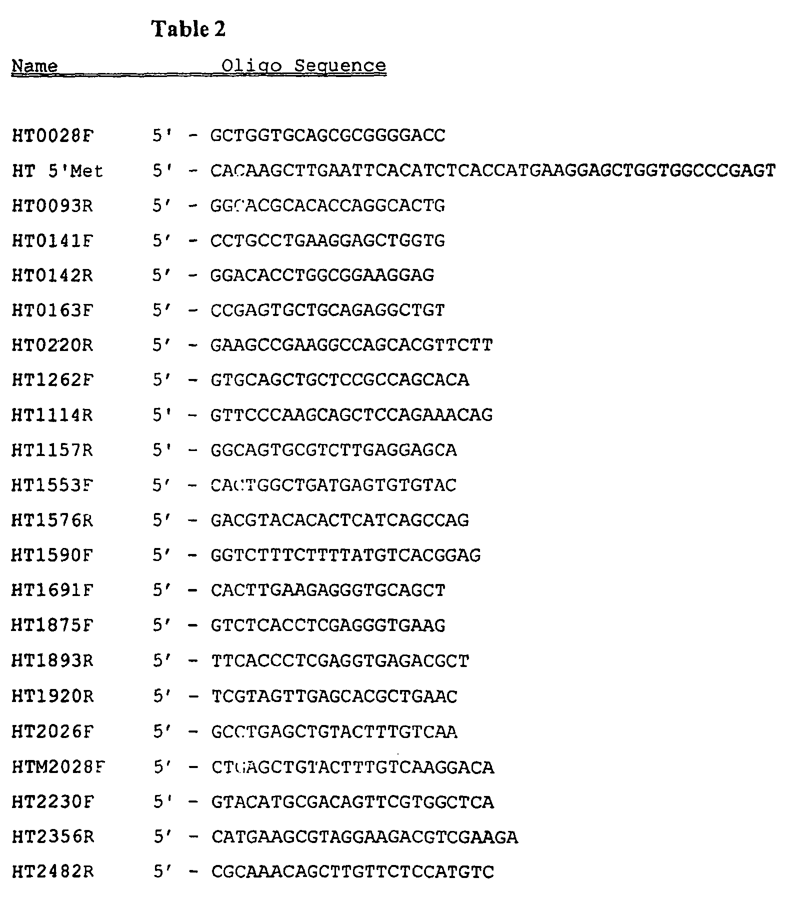

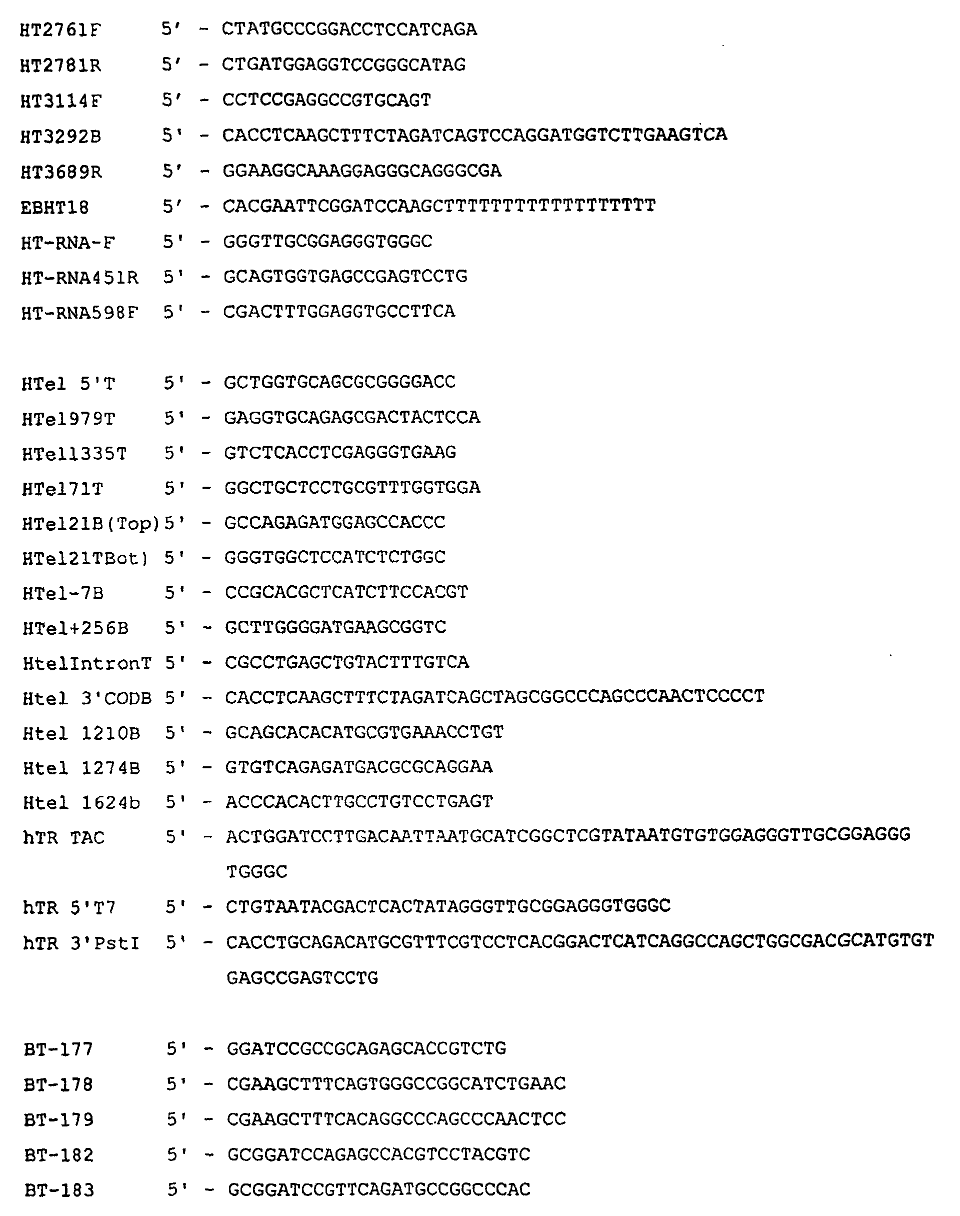

### EXAMPLE 2

### HT1 SEQUENCE AND ALIGNMENT WITH OTHER TELOMERASES

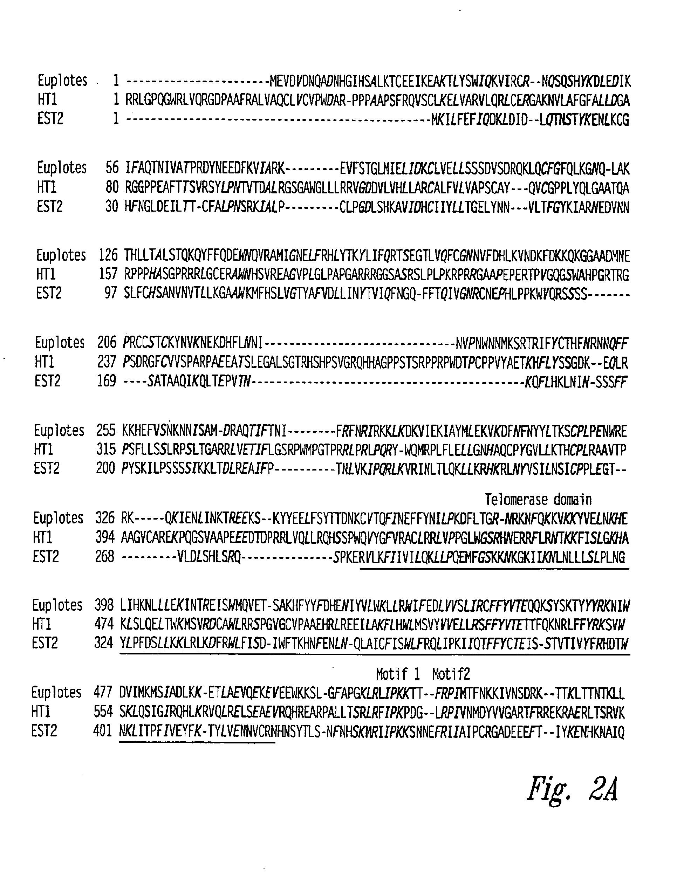

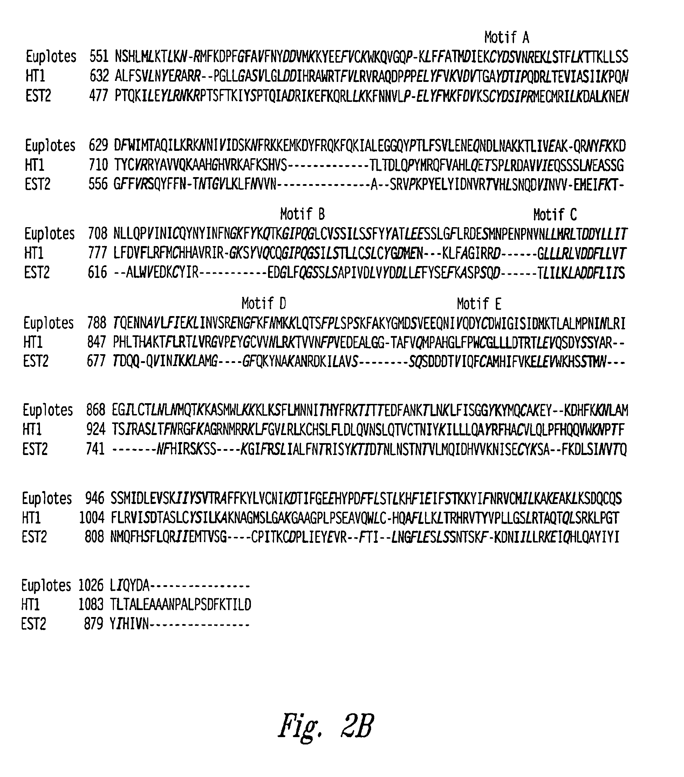

Multiple sequence alignment demonstrates that the predicted hT1 protein is co-linear with the *Euplotes* and *S. cerevisae* telomerase catalytic subunits over their entire lengths (Figure 2). Although the overall homology between the three proteins is relatively low (approximately 40% similarity in all pairwise combinations) the overall structure of the protein seems to be well conserved. Four major domains: N-terminal, basic, reverse transcriptase (RT) and C-terminal are present in all three proteins. The highest area of sequence similarity is within the RT domain. Notably, all the motifs characteristic of the *Euplotes* RT domain are present and all amino acid residues implicated in RT catalysis are conserved in the hT1 sequence (Lingner et al., *Science 276*: 561-567, 1997).

Recently, protein phosphatase 2A treatment of human breast cancer cell extracts has been shown to inhibit telomerase activity (Li et al., *J. Biol. Chem. 272:* 16729-16732, 1997). It is not known whether this effect is direct, but it raises the possibility of regulation of telomerase activity by protein phosphorylation. The predicted hT1 protein does contain numerous potential phosphorylation sites, including 11 SP or TP dipeptides, which are potential sites for cell cycle dependent kinases.

### EXAMPLE 3

### CHARACTERIZATION OF TELOMERASE GENE

Northern analysis and Southern analysis are performed to determine the size of the telomerase transcript and whether telomerase gene is amplified in tumors cells.

For Northern analysis, polyA mRNA is isolated from LIM 1215 cells and from CCD fibroblasts. CCD is a primary human fibroblast cell line. Briefly cells are lysed by homogenization in a buffered solution (0.1 M NaCl, 10 mM Tris, pH 7.4, 1 mM EDTA) containing detergent (0.1% SDS) and 200 µg/ml of proteinase K. SDS is added to the lysate to a final concentration of 0.5%, and the lysate is incubated at 60°C for 1 hr and 37°C for 20 min. The lysate is then incubated for 1 hr with a slurry oligo dT-cellulose that has been pre-cycled in 0.1 M NaOH and equilibrated in 0.5 M NaCl, 10 mM Tris pH 7.4, 1 mM EDTA, and 0.1% SDS. The resin is collected by centrifugation, batch washed in the equilibration buffer, and loaded into a column. The mRNA is eluted with warmed (37°C) buffer (10 mM Tris pH 7.4, 0.1 mM EDTA) and ethanol precipitated.

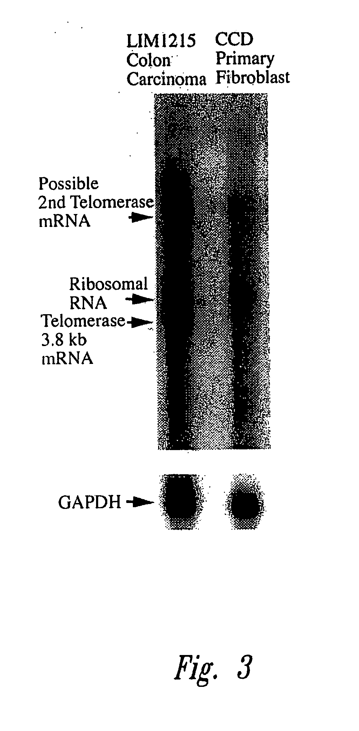

Approximately 3 µg of polyadenylated RNA is electrophoresed in a 0,.85% formaldehyde-agarose gel (see Sampbrook et al., *supra*) and transferred overnight to Genescreen plus (Bio-Rad, CA). The membrane is hybridized with a ³²P-labeled telomerase-specific probe (390 bp insert corresponding to the EST sequence). After washing the blot at high stringency, a prominent ~3.8 kb band is observed in mRNA from LIM 1215, but not in mRNA from CCD fibroblasts (Figure 3). Subsequent hybridization of the same membrane with a probe for glyceraldehyde 6-phosphate dehydrogenate demonstrated an equivalently strong band in both mRNAs, indicating that each lane contained a similar amount of high quality RNA. The presence of larger transcripts (especially a ~ 8 kb heterodispersed band) is also visible only in LIM1215 RNA (Fig. 10, upper panel.). These findings provide an indication of additional hTl-specific mRNA and also that hT1 may be preferentially expressed in tumor versus normal cells.

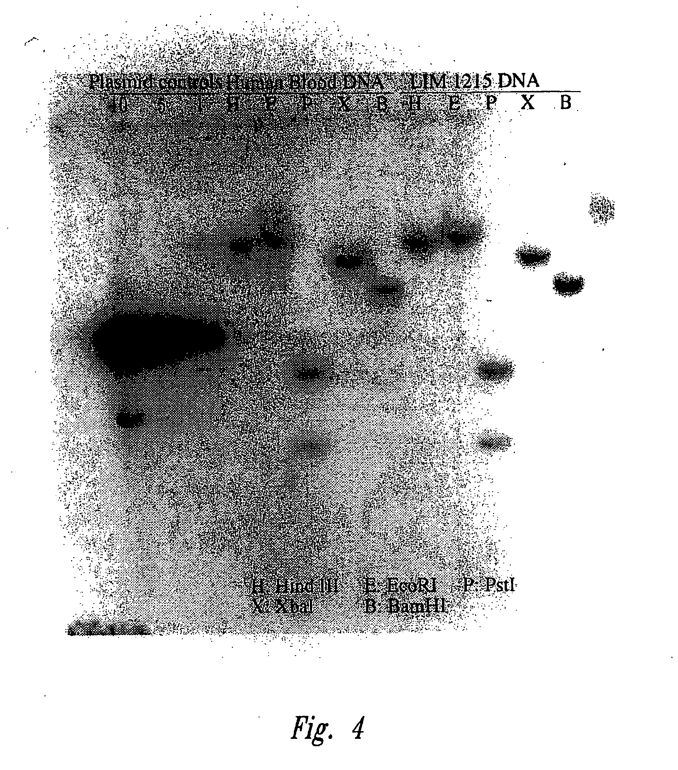

For Southern analysis, DNA is isolated from human peripheral blood mononuclear cells and LIM 1215. Approximately 10 µg of DNAs are digested with *Hind* III, *Xba I, Eco* RI, *Bam*HI, and *Pst*I, electrophoresed in a 1% agarose gel, and transferred to a nylon membrane. For controls, plasmid DNA containing human telomerase is titrated to approximately the equivalent of 10 copies, 5 copies, and 1 copy per 10 µg genomic DNA and electrophoresed on the same gel. A 390 bp fragment of telomerase gene (containing the EST sequence) is ³²P-labeled and hybridized under normal stringency conditions. The filter is washed in 2X SSC, 0.1% SDS at 55°C. A scanned phosphor image is presented in Figure 4. As shown, the telomerase gene does not appear to be amplified or rearranged in LIM1215 as there is not significant difference in the pattern or intensity of hybridization when comparing LIM 1215 to PBMC DNA. Moreover, telomerase appears to be a single copy gene, as all digestions except *Pst* I yielded a single band.

### EXAMPLE 4

### HT1 EXPRESSION PATTERNS

Although telomerase activity has been widely associated with tumor cells and the germline, it has only recently been recognized that certain normal mammalian tissues express low levels of telomerase activity. hT1 expression is not detected in primary fibroblast RNA, and amplification of several commercially available cDNA libraries from lung, heart, liver, pancreas, hippocampus, fetal brain, and testis using primers for the EST region, did not reveal any products.

However, the expression of hT1 in normal tissues that have previously been shown to have telomerase activity (colon, testis and peripheral blood lymphocytes) are examined, as well as a number of melanoma and breast cancer samples. RNA is isolated from normal human colon, testis and circulating lymphocytes, and from tissue sections of tumor samples, and subjected to RT-PCR analysis. Amplification products from cDNA are easily distinguished from products resulting from contaminating genomic DNA, as a product of ~300 bp is observed using cDNA as a template and a product of 2.7 kb is observed using genomic DNA as a template. hT1 transcripts are detected in both colon and testis, in the majority of tumor samples, and very weakly in the lymphocyte RNA (Figure 5, upper panel). Interestingly, two of the breast cancer samples are negative for hT1 expression, despite containing comparable amounts of RNA to the other samples, as judged by amplification of β-actin as a positive control (Figure 5, lower panel).

Acquisition of telomerase activity appears to be an important aspect of the immortalization process. The expression of hT1 in a number of matched pairs of pre-crisis cell cultures and post-crisis cell lines is determined using RT-PCR followed by amplification from nested primers (Figure 6, upper panel). These cell lines are telomerase negative (pre-crisis cell line) and positive (post-crisis cell lines), respectively, using the TRAP assay (Bryan et al., *EMBO J. 14:* 4240-4248, 1995). In two matched pairs, BFT-3B and BET-3K, hT1 is detected only in the post-crisis cell lines (compare lanes a and b, lanes e and f). While the post-crisis line (lanes d, f) in the BFT-3K set shows an abundant hT1 band, a fragment of the same size is also weakly present in the pre-crisis (lanes c, e) culture sample. In addition, two of the three post-crisis cell lines demonstrate the presence of an additional unexpected fragment of 320 bp, and this product is also observed when colon and testis mRNA are analyzed on high resolution gels.

Three immortalized telomerase-negative (ALT) cell lines are also analyzed for hT1 expression (Figure 6, lanes g, h, i). Two of the lines appear negative for hT1 expression, but in one line (IIICF-T/B1), a product of approximately 320 bp is again amplified (Figure 6, lane i), similar to the post-crisis, colon and testis samples. DNA sequence analysis of the 320 bp product from the line IIICF-TB1 (ALT) reveals the presence of a 38 bp insertion, relative to the expected product. The possibility that this is an amplification from genomic DNA rather than mRNA is ruled out by performing amplification with the same primers but using genomic DNA as the template. Under these conditions, a 2.7 kb fragment is amplified and its authenticity confirmed by partial sequence analysis.

### EXAMPLE 5

### IDENTIFICATION OF ALTERNATIVE SPLICING PATTERNS OF TELOMERASE MRNA

DNA sequence analysis of clones from the LIM1215 cDNA library and the RT-PCR data presented above for the pre-crisis and post-crisis cultures indicated that there is a number of different sequence variants within the hT1 transcript. To systematically survey for variants, RT-PCR is performed using primer pairs covering the whole sequence. No variants are observed in the N-terminal and the basic domains, but several variants are observed in the RT domain and, to a lesser extent, the C-terminal domain. Most notably, there are several RNA variants between RT Motif A and RT Motif B (Figure 7A).

Samples of mRNA are prepared from several different tumors using conventional protocols. The tumors are: (1) SLL lung carcinoma, (2) Lymphoma C, (3) Lung carcinoma, (4) Medullablastoma A, (5) Lymphoma B, (6) Lymphoma E, (7) Tumor sample 47D, (8) Pheochromocystoma, (9) Lymphoma F, (10) Glioma, and (11) Lymphoma G. The mRNAs from these samples are first reverse transcribed to cDNAs and then amplified using primers HT1875F and HT2781R, followed by amplification with nested primers HT2026F and HT2482R. Four different amplified products are observed in Figure 8: 220 bp (band 1), 250 bp (band 2), 400 bp (band 3) and 430 bp (band 4). Strikingly, there is considerable variation among the tumor samples tested both in the total number of amplified products and in the quantitative distribution among the products.

Three of these products are isolated from a number of tumor tissues and subjected to DNA sequence analysis. One of them, a 220 bp fragment, is equivalent to the 53.2 cDNA from the LIM1215 library. The fragment of the ~250 bp (band 2) contains a 36 bp in-frame insertion, the same insertion that was identified in an amplified product from a LIM1215 cDNA library. As the RT-PCR product had the same sequence as the product from the cDNA library, it is apparent that the 36 bp insertion is not an artifact generated during library construction. The largest product (band 4) contains a 182 bp insertion (the same as the larger product amplified earlier from LIM1215 RNA) compared to the 250 bp amplicon. Unambiguous sequence for the 400 bp band (band 3) is not obtained. Based on its size, it may contain the 182 bp insert but missing the 36 bp insertion present in bands 2 and 4 and absent from band 1.

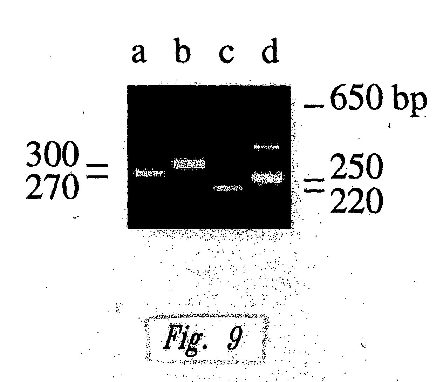

To test the hypothesis that such a transcript exists, a primer, HTM2028F, is designed such that amplification ensues only when the 36 bp fragment was missing. Amplification using HTM2028F and HT2026F primers in combination with HT2356R demonstrate that transcripts containing the 182 bp fragment but missing the 36 bp fragment are present in LIM1215 RNA (Figure 9, lanes a and b). The same top strand primers (HTM2028F and HT2026F) in combination with HT2482R primer amplify a number of products from LIM1215 RNA (Figure 9, lanes c and d), most of which represent bands 1- 4 as determined by direct sequence analysis of PCR products. An amplified fragment of 650 bp using HTM2028F and HT2482R primers represents another, not yet fully characterized, alternatively spliced telomerase variant in the RT-MotifA/RT Motif B region. For clarity of presentation, the protein sequence giving the best match with *Euplotes* and *S. cerevisiae* proteins is presented in Figure 1 as the reference sequence.

Specifically, there are at least seven inserts or introns that can be present (or absent) from telomerase RNA. (1) The 5'-most sequence (Y) is located between bases 222 and 223. (2) the insert (X) is located between bases 1766 and 1767. A partial sequence is determined and is presented in Figure 10. Termination codons are present in all three reading frames. Thus, a truncated protein without any of the Rtase motifs would be produced. (2) A sequence, indicated as "1" in Figure 7, is located between bases 1950 and 1951. This intronic sequence is 38 bp (Figure 10) and appears to be present in ALT and most tumor lines. The presence of this sequence adds 13 amino acids and shifts the reading frame, such that a termination codon (TGA) is in frame at nucleotide 1973. (3) A sequence, indicated as "α" in Figure 7, is located between bases 2130 and 2167. This sequence is 36 bp (Figure 10) and its absence removes RTase motif "A" but does not alter the reading frame. (4) A sequence, indicated as "β" in Figure 7 is present between bases 2286 and 2469. The insert is 182 bases (Figure 10) and its absence causes a reading frame-shift and a termination codon in RTase motif 5 at nucleotide 2604. (5) The sequence "2" in Figure 7 is present between bases 2823 and 2824. Its length is undetermined; its partial sequence is presented in Figure 10. The presence of this insert causes a truncated telomerase protein, as the first codon of the insert is a termination codon. (6) The sequence "3" is a 159 bp insert (Figure 10) between bases 3157 and 3158. Its presence leads to a telomerase protein with an altered COOH-terminus. The insert contains a stop codon. Moreover, sequence "3" has a putative binding site for the SH3 domain of c*-abl* (PXXXXPXXP; PEMEPPRRP).

The transcript that most closely aligns with *Euplotes* and yeast telomerases by amino acid similarity contains sequences A and B, and does not contain sequence C. The nucleotide and amino acid sequences of eight variants resulting from mRNAs comprising combinations of sequences A, B, and C are presented in Figure 8.

### EXAMPLE 6

### RECOMBINANT EXPRESSION OF HUMAN TELOMERASE

Human telomerase is cloned into bacterial expression vectors. The sequence shown in Figure 1 is amplified from LIM 1215 mRNA in two pieces and then ligated together.

For the amplification, first strand cDNA is synthesized and used in an amplification reaction (Titan system, Boehringer, IN) with a mixture of DNA polymerases, such that a proofreading thermostable enzyme (*e.g., rTth*)is used with *Taq* DNA polymerase. As much of the mRNA in LIM 1215 lacks sequence B (Figure 9), the amplification primers are designed such that one primer of each pair is within sequence B, on either side of the *Sac* I site at nucleotide 2271 (Figure 1). The 5' portion is first amplified from cDNA using HT2356R and HT0028F primers (cycle conditions: 70°C, 2 min; then added primer sequences equilibrated to 50°C; 50°C, 30 min; 95°C, 2 min; 2 cycles of 94°C, 30 sec; 65°C, 30 sec; 3 cycles of 94°C, 30 sec; 63°C, 30 sec; 68° C 3 min; 32 cycles of 94°C, 30 sec; 60°C, 30 sec; 68°C, 3 min). The extreme 5' portion of the telomerase gene is then ligated in *Eco* RI/ *Sac* I digested pTTQ18 (Amersham International plc, Buckinghamshire, England) and pBluescriptII KS+, and the sequence verified.

To obtain the 3' end, LIM 1215 cDNA is amplified using HT2230F and a HT3292B primer that is complementary to the sequence encoding the very C-terminus of telomerase. The amplification products are digested with *Hind* III and *Sac* I and inserted into pTTQ18 and pBluescript II KS+. The 5' and 3' ends are also cloned joined at the native *Sac* l site in pTTQ18 both as a Hexa-His fusion and a non-fusion protein.

The plasmid pTTQ18-Htel is transfected into bacterial cells (*e.g.,* BL21(DE3)). Over expression of the protein is accomplished upon induction with IPTG. The bacteria are collected by centrifugation and lysed in lysis buffer (20 mM NaPO₄, pH 7.0, 5 mM EDTA, 5 mM EGTA, 1 mM DTT, 0.5 µg/ml leupeptin, 1 µg/ml aprotinin, 0.7 µg/ml pepstatin). This mixture is evenly suspended via a Polytron homogenizer and the cells are broken open by agitation with glass beads or passage through a microfluidizer. The resulting lysate is centrifuged at 50,000 rpm for 45 min. The supernatant is diluted with 20 mM NaPO₄, 1 mM EDTA, pH 7.0 (buffer A). The diluted lysate supernatant is then loaded onto a SP-Sepharose or equivalent column, and a linear gradient of 0 to 30% SP Buffer B (1 M NaCl, 20 mM NaPO₄, 1 mM EDTA, pH 7.0) in Buffer A with a total of 6 column volumes is applied. Fractions containing telomerase are combined. Further purifications can be performed.

For hexa-His fusion proteins, the lysate is clarified by centrifugation and batch absorbed on a Ni-IDA-Sepharose column. The matrix is poured into a column and washed with buffer, typically either 50 mM Tris pH 7.6, 1 mM DTT; 50 mM MES pH 7.0, or IMAC buffer (for hexa-his fusions). The telomerase protein bound to the matrix is eluted in NaCl containing buffer.

### EXAMPLE 7

### RECOMBINANT EXPRESSION OF HUMAN TELOMERASE RNA COMPONENT

The human telomerase RNA component is first isolated by amplification from genomic DNA. The amplification primers are telRNA T and telRNA 598B (Figure 5). Amplification conditions are 95°C, 3 min; addition of polymerase; 80°C 2 min; 35 cycles of 94°C, 30 sec; 68°C, 2 min.

The amplified product is inserted into pBluescript after another amplification using hTR TAC (has a tac promoter sequence) and hTR 3'Pst (has a cis-acting ribozyme sequence) primers. The pBluescript insert is then isolated and ligated to pACYC 177.

### EXAMPLE 8

### EXPRESSION OF HUMAN TELOMERASE SUBREGIONS

The RTase domain of human telomerase is determined by sequence comparison with Moloney MuLV reverse transcriptase. The fingers/palm region of Moloney MuLV reverse transcriptase forms a stable unit for crystallization (Georgiadis et al., *Structure 3:* 879, 1995). A number of residues and motifs are conserved in the active site of both proteins. Primers are designed to amplify the RTase domain and the fingers/palm domain for insertion into an expression vector and subsequent protein isolation.

| Fragment ID | Primers | Amino acids |
|---|---|---|
| I | BT-177 / BT-178 | AAEH...→ ...VQMPAH |
| U | BT-177 / BT-179 | AAEH... → ...VGLGL |
| III | BT-182 /BT-179 | RATS... → ...VGLGL |
| IV | BT-183 / BT-179 | VQMPAH...→...VGLGL |

Fragment I encodes the "fingers and palm" domain that corresponds to MoMuLV. The C-terminal "thumb" and "connection" (see, Kohlstaedt et al., *Science 256*: 1783, 1992) are deleted. Fragment II encodes the telomerase reverse transcriptase domain, as well as the C-terminal "connection" domain. The N-terminus is chosen by size comparison with the MoMuLV RTase structure. Fragment III encodes the C-terminus of the protein. The RATS sequence is located within the RTase domain (palm region) of the protein. Fragment IV encodes the C-terminal region containing the "thumb" and "connection" domains and may function as a regulatory element. The connection domain in HIV-1 is able to block the catalytic cleft of HIV RTase in the absence of the RNase domain (Kohlstaedt et al, *supra*). In an analogous fashion, the C-terminal region may be useful as a regulatory (inhibitory) fragment. Moreover, sequence C has a putative binding site for the SH3 domain of c*-abl* (PXXXXPXXP; PEMEPPRRP, see variant 2 sequence of Figure 8). c*-abl* protein interacts directly with the ATM (ataxia telangiectasia) protein (Shafman et al., *Nature 389:* 520, 1997), a protein apparently involved in cell-cycle control, meiotic recombination, telomer length monitoring and DNA damage response. Binding of c*-abl* protein may be assessed in standard protein-protein interaction methods. As such, an interaction of telomerase and c*-abl* or other SH3-domain containing proteins (*e.g.*, erb2) and regulation by movement of the telomerase C-terminus in and out of the catalytic cleft may be controllable using the constructs and products described herein. In one instance, regulation may be mediated by phosphorylation/dephosphorylation reactions.

All primers have either a *Hind* III or a *Bam* HI site. The amplification reaction is performed in 1X *Pfu* buffer, 250 µM dNTPs, 100 ng each primer, clone 53.2 template DNA using the following cycling conditions: 94°C for 2 min; 25 cycles of either 55°C, 60°C or 65°C for 2 min, 72°C for 2 min, 94°C for 1 min; followed by 72° C for 10 min. Products of the predicted length are obtained (966 bp for BT-177/BT-178; 1479 bp for BT-177/BT-179; 824 bp for BT-182/BT-179; 529 bp for BT-183/BT-179). The amplified products are extracted with phenol:CHCL3 and precipitated with ethanol. The products are resuspended and digested with the appropriate enzyme that cleaves in the primer sequence.

The digested products are ligated to pBluescript that is digested with enzymes that leave compatible ends. The inserts are digested with *Hind* III and partially digested with *Bam* HI for ligation to pGEX. The plasmid is transfected in BL21(DE3) cells and selected on ampicillin plates. Colonies are picked and grown overnight in liquid broth. An aliquot is diluted in Terrific Broth with 100 µg/ml ampicillin. The cells are grown at 37°C and induced with 0.5 mM IPTG at approximately O.D. 0.8. Growth is continued for 5 hours. Cells are collected by centrifugation and may be processed immediately or frozen at -70°C until needed.

Protein is purified from lysed cells. Cell pellets are lysed by vortexing in 50 mM Tris pH 8.0, 10 mM 2-ME, 1 mg/ml lysozyme, 0.5% Triton X-100, 1 µg/ml pepstatin, 10 µg/ml leupeptin, 10 µg/ml aprotinin, 0.5 mM PMSF, and 2 mM EDTA and a freeze/thaw cycle. Lysates are clarified by centrifugation. Supernatant is added to a 50% slurry of GSH-Sepharose, rotated at 4°C for 2 hr. The matrix is washed twice with lysis buffer, followed by 50 mM Tris, pH 8.0, 10 mM 2-ME. For analysis by SDS-PAGE gel electrophoresis, sample buffer with 150 mM 2-ME is added and the samples boiled.

### EXAMPLE 9

### ISOLATION OF MURINE TELOMERASE GENE

The murine telomerase gene is isolated from genomic or cDNA library. A mouse genomic library is constructed in λFIX II vector from strain 129 DNA. The library is plated, and plaques are lifted onto nylon membranes. The membranes are hybridized with the insert from clone 53.1 (1.9 kb) under normal stringency conditions. Six hybridizing plaques are chosen for further analysis.

### EXAMPLE 10

### DEMONSTRATION OF TELOMERASE ACTIVITY USING HT-1 AND TELOMERASE VARIANTS

Full-length hT-1 sequence is cloned into an expression vector and the resulting protein is assayed for telomerase activity. Vector pRc/CMV2 (Invitrogen, Carlsbad, CA) is a eukaryotic expression vector that has a multi-cloning site positioned between a promoter, the RSV LTR, and a polyadenylation signal and transcription termination sequences from the bovine growth hormone gene. Telomerase sequence in which Leu49 codon was converted to a Met codon was inserted into pRc/CMV2. One clone, phTC51, is chosen for further study. The DNA sequence of the 5' junction was determined and confirmed the orientation of the insert. Subsequently, the sequence of the 3' junction was determined and showed a deletion of the polyA signal, but no deletion of telomerase coding sequence.

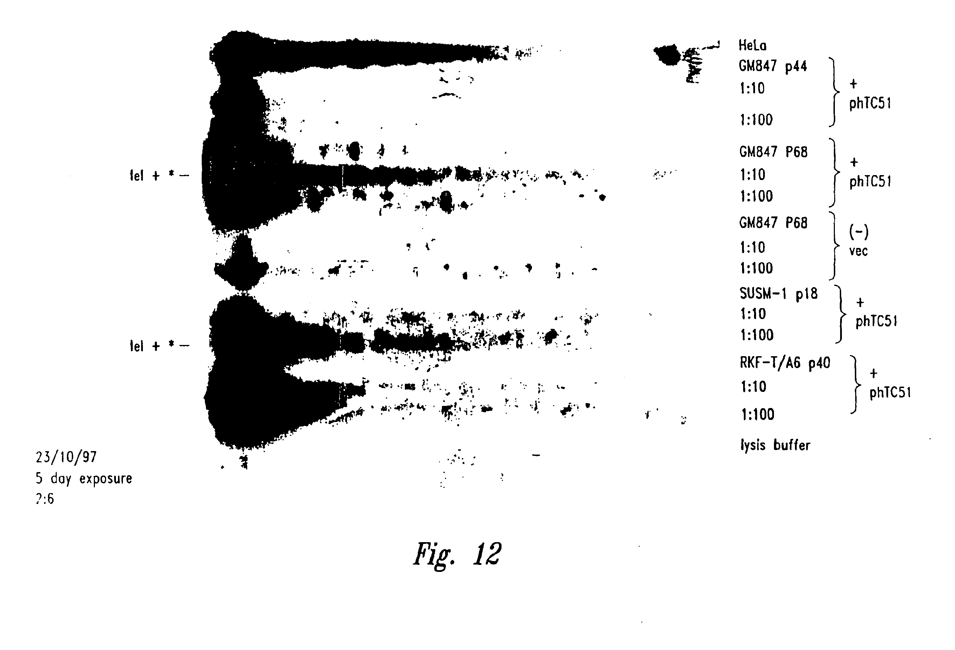

The clone is transfected into HeLa GM847 cells at passages 44 and 68, SUSM-1 cells at passage 18, and RKF-T/A6 cells at passage 40. Cell extracts are assayed for telomerase activity by the TRAP assay as described herein. As shown in Figure 12, a ladder of products indicative of telomerase activity is seen at the 1:100 dilution of extract from SUSM-1 cells and is not seen in control cells. A ladder is not readily detectable at the higher concentration of extract, which may be due to nuclease activity in the extract.

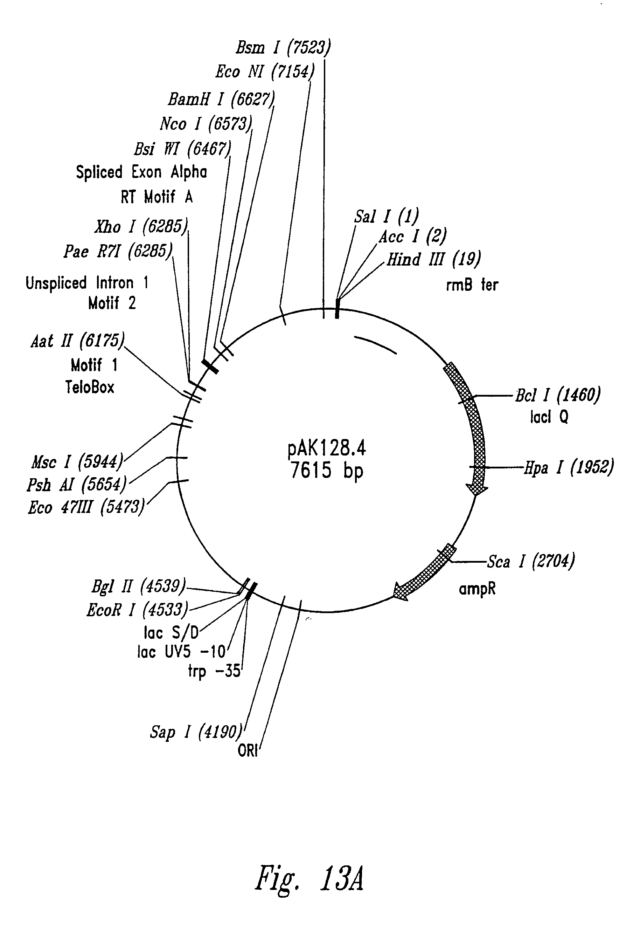

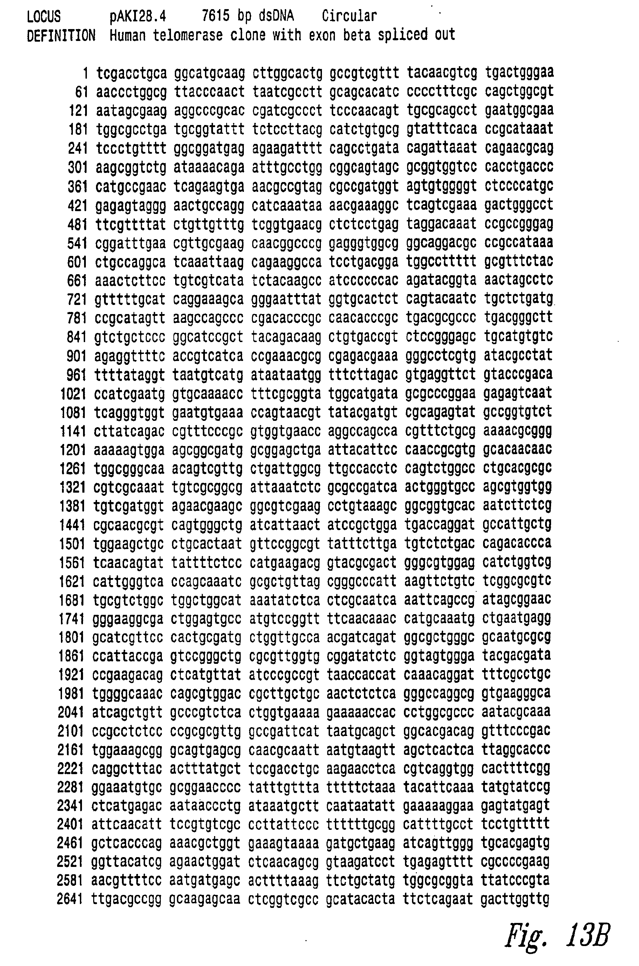

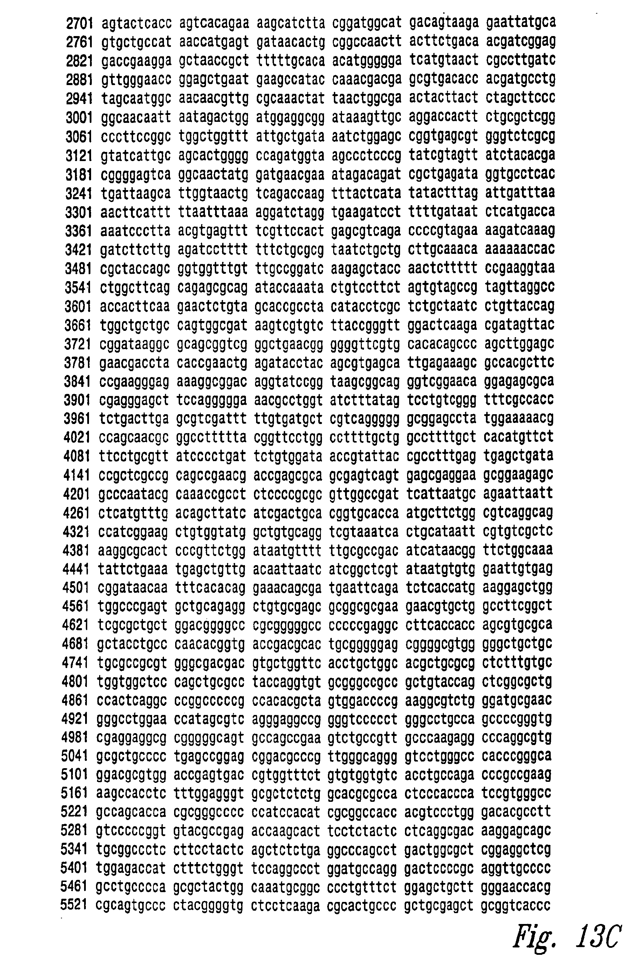

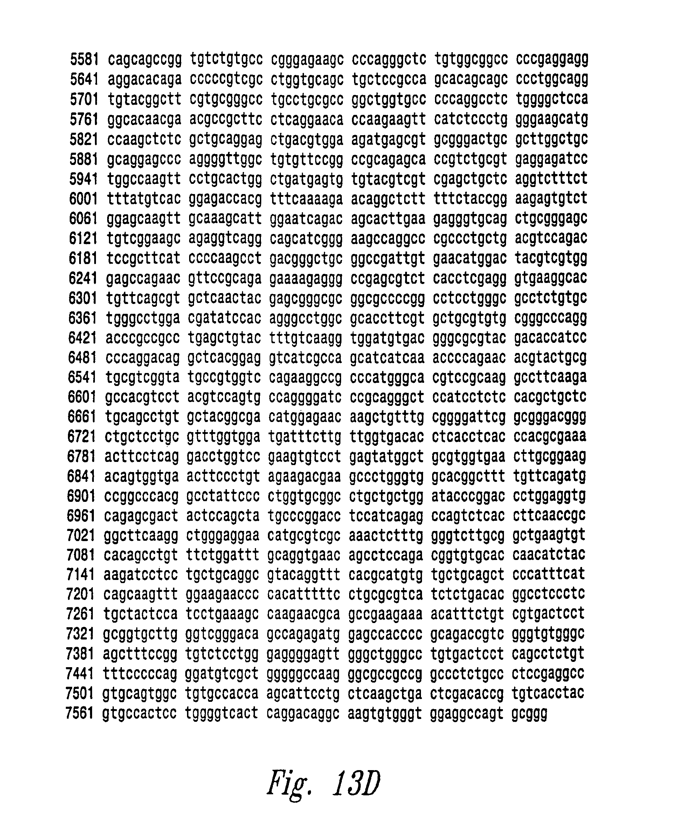

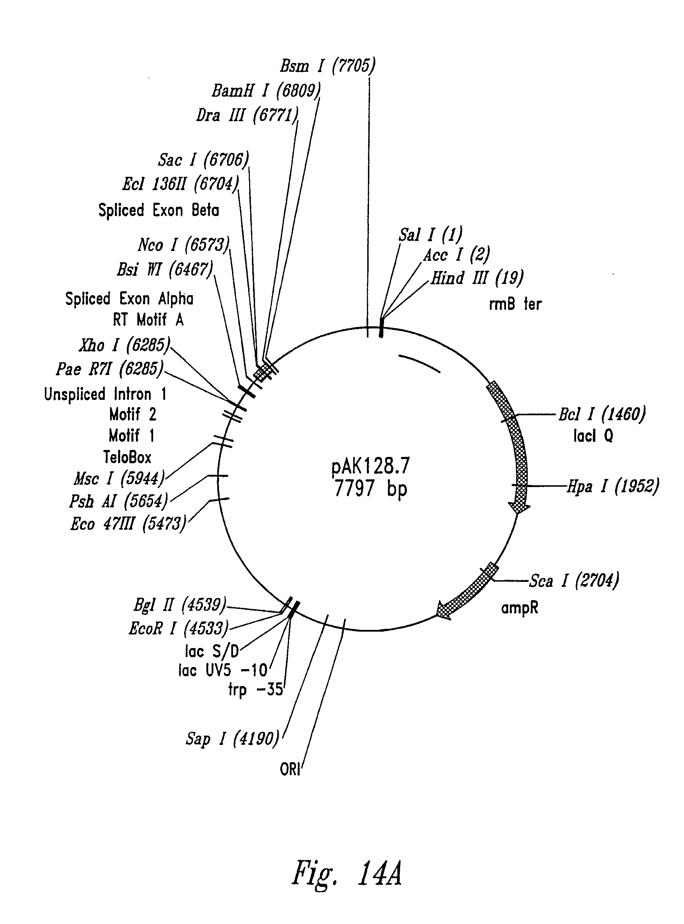

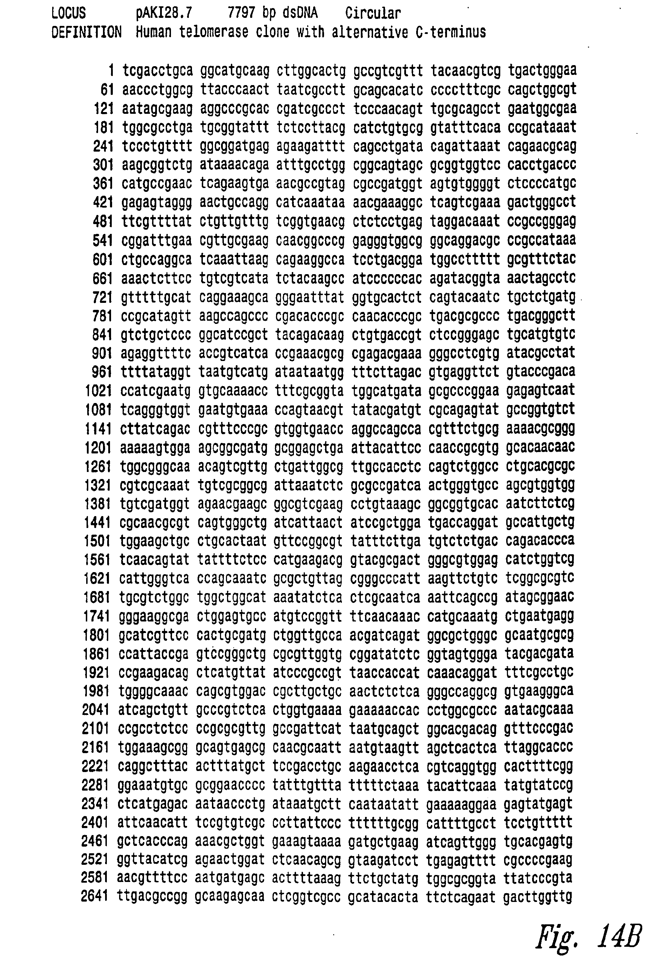

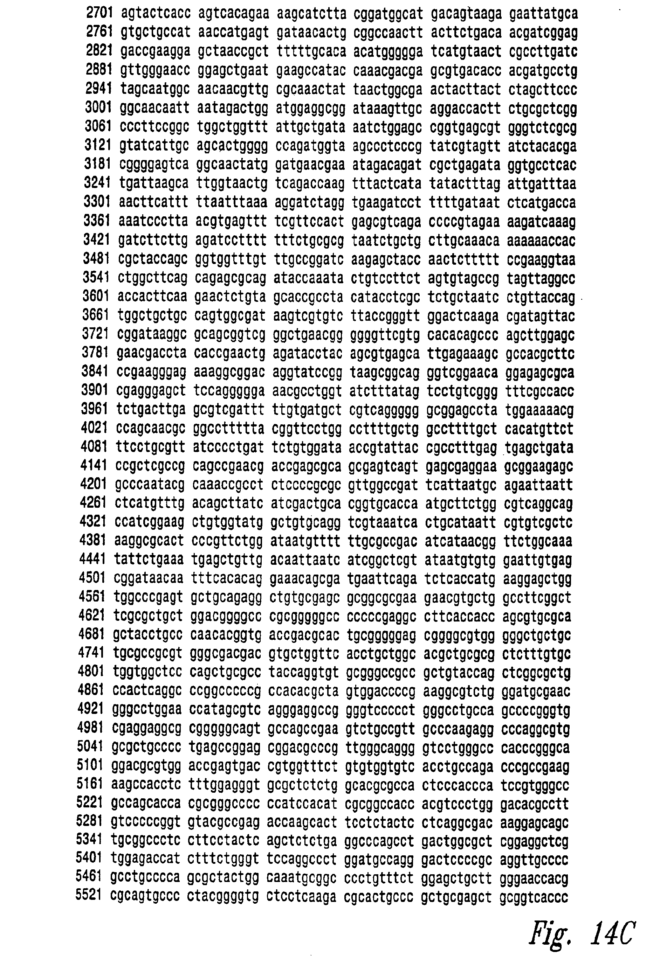

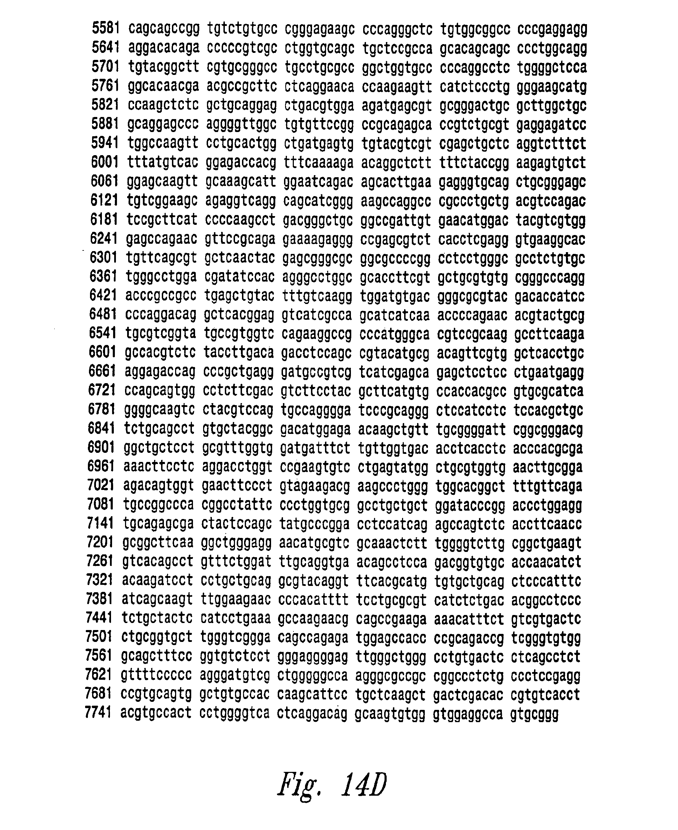

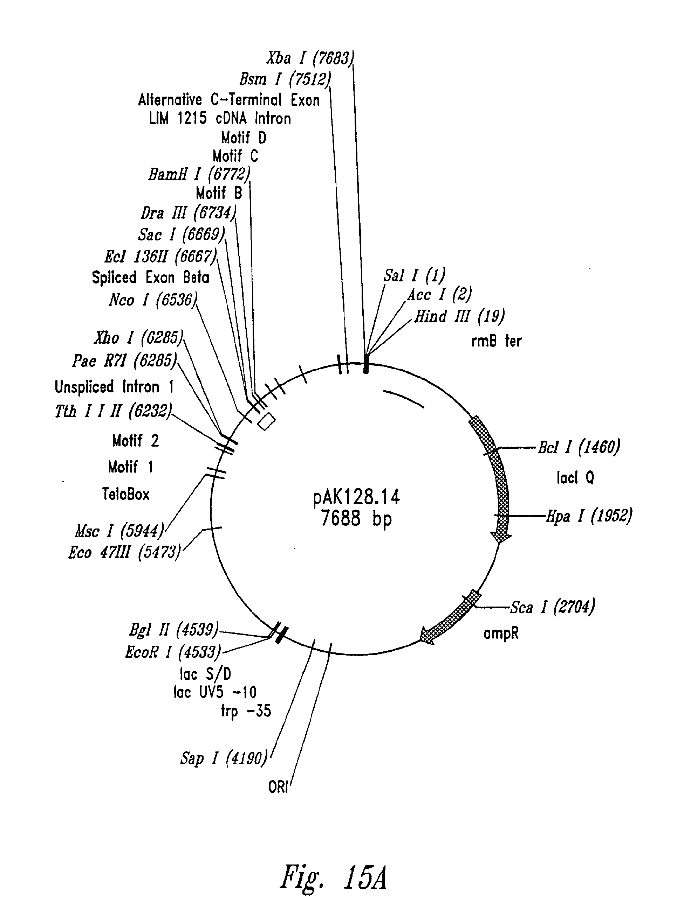

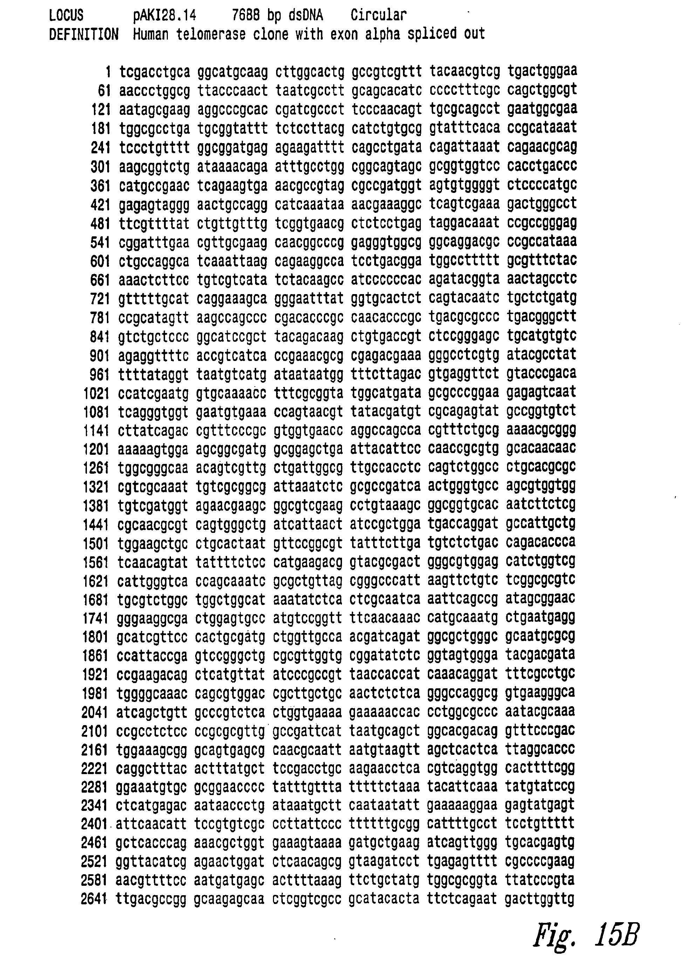

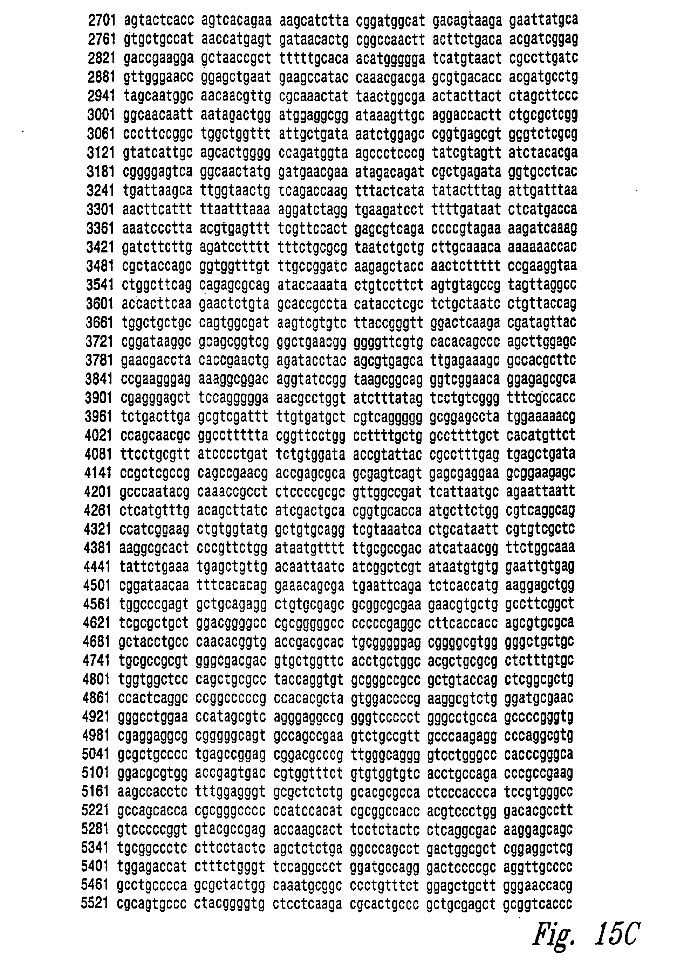

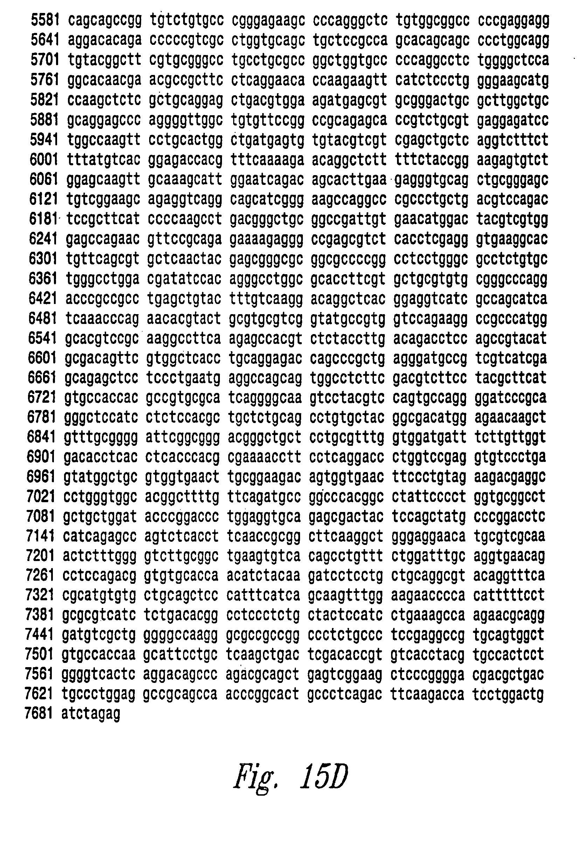

Three telomerase variants are constructed: pAKI.4 is telomerase with the beta region spliced out (Figure 13); pAKI.7 is telomerase with the alternative C-terminus insert 3 (Figure 14); and pAKI.14 is telomerase with the alpha region spliced out (Figure 15). The 5' end of the telomerase gene was inserted into each of these three vectors and the inserts moved to pCIneo expression vector. The variants, along with reference telomerase in pCIneo are transiently transfected into GM847 cells, which are ALT cells having no detectable telomerase activity but which express the RNA subunit. Cell extracts are tested in a TRAP assay. The reference telomerase exhibits activity, as well as the telomerase with insert 3 (pAKI.7 insert), but the other variants do not express activity.

From the foregoing it will be appreciated that, although specific embodiments of the invention have been described herein for purposes of illustration, various modifications may be made without deviating from the spirit and scope of the invention. Accordingly, the invention is not limited except as by the appended claims.

## Claims

1. An isolated nucleic acid molecule encoding vertebrate telomerase.

2. The isolated nucleic acid molecule according to claim 1 wherein said vertebrate is a human.

3. The nucleic acid molecule of claim 1, wherein the nucleic acid molecule comprises the sequence presented in Figure 1, or hybridizes under normal stringency conditions to the complement of the sequence presented in Figure 1, provided that the nucleic acid molecule is not EST AA281296.

4. The nucleic acid molecule of claim 1, wherein the nucleic acid molecule encodes the amino acid sequence presented in Figure 1 or 11, or variant thereof.

5. An isolated nucleic acid molecule encoding any of the amino acid sequences presented in Figure 11, or hybridizes under normal stringency conditions to the complement of the sequences thereof, provided that the nucleic acid molecule is not EST AA281296.

6. An isolated nucleic acid molecule comprising any of the sequences presented in Figure 10, or hybridizes under normal stringency conditions to the complement of the sequences thereof.

7. An oligonucleotide comprising from 10 to 100 contiguous nucleotides from the sequence presented in Figure 1 or its complement.

8. An oligonucleotide comprising from 10 to 100 contiguous nucleotides from the sequences presented in Figure 10 or the complements thereof.

9. The oligonucleotide of either of claims 7 or 8, wherein the oligonucleotide is labeled.

10. The oligonucleotide of claim 9, wherein the label is a radiolabel, a chemiluminescent label, or biotin.

11. An expression vector, comprising a heterologous promoter operably linked to a nucleic acid molecule according any of claims 1-6.

12. The expression vector of claim 11, wherein the vector is selected from the group consisting of bacterial vectors, retroviral vectors, adenoviral vectors and yeast vectors.

13. A host cell containing a vector according to either claims 11 or 12.

14. The host cell of claim 13, wherein the cell is selected from the group consisting of human cell, monkey cell, mouse cell, rat cell, yeast cell and bacterial cell.

15. The host cell of claim 13, wherein the cell is a human cell.

16. An isolated protein comprising a vertebrate telomerase protein.

17. The protein of claim 16, wherein the vertebrate is a human.

18. The protein of claim 16, wherein the protein comprises the amino acid sequence presented in Figure 1 or 11, or variant thereof.

19. A portion of a vertebrate telomerase protein.

20. The portion of claim 19, wherein the amino acid sequence of the portion is presented in Figure 1.

21. The portion of claim 19, wherein the amino acid sequence of the portion is presented in Figure 11.

22. The portion of claim 19, wherein the portion is from 10 to 100 amino acids long.

23. An antibody that specifically binds to the protein according to either claim 16 or 19.

24. An antibody that specifically binds to a polypeptide encoded by a sequence selected from the group consisting of region 1, region α, region β, region 2 and region 3.

25. The antibody according to claim 24, wherein the antibody is a monoclonal antibody.

26. A hybridoma that produces an antibody according to claim 14.

27. A nucleic acid probe that is capable of specifically hybridizing to a nucleic acid molecule encoding a vertebrate telomerase under conditions of normal stringency, provided that the probe does not hybridize to nucleotides 1624-2012 presented in Figure 1.

28. The probe of claim 27, wherein the probe is from 12 to 200 nucleotides long.

29. The probe of claim 27, wherein the probe is from 20 to 50 nucleotides long.

30. The probe of claim 17, wherein the nucleic acid molecule has the sequence presented in Figure 1 or its complement thereof.

31. The probe of claim 17, wherein the nucleic acid molecule is labeled.

32. A pair of oligonucleotide primers capable of specifically amplifying all or a portion of a nucleic acid molecule encoding human telomerase.

33. The primers of claim 32, wherein the nucleic acid molecule comprises the sequence presented in Figure 1 or its complement.

34. The primers of claim 32, wherein the nucleic acid molecule comprises any of the sequences presented in Figure 11 or the complements thereof.

35. The primers of claim 32, wherein the pair of primers is capable of specifically amplifying sequence comprising all or a part of region 1, region α, region β, region 2, region 3 region X or region Y.

36. The primers of claim 35, wherein the primers flank nucleotide 222, 1950,2131-2166,2287-2468,2843, or 3157 as presented in Figure 1.

37. The primers of claim 36, wherein only one of each primer pair flanks nucleotide 222, 1950, 2131-2166, 2287-2468, 2843, or 3157 as presented in Figure 1 and the other primer of the pair has sequence corresponding to one of the sequences presented in Figure 10 or complements thereof.

38. A pair of oligoprimers capable of specifically amplifying genomic sequence presented in Figure 10, wherein the primers amplify more than nucleotides 1 to 38.

39. An oligonucleotide that hybridizes specifically to a nucleic acid sequence in region 1, region α, region β, region 2, region 3 region X or region Y.

40. The oligonucleotide of claim 39, wherein the oligonucleotide is from 15 to 36 bases.

41. A method of diagnosing cancer in a patient, comprising preparing tumor cDNA and amplifying the tumor cDNA using primers that specifically amplify human telomerase nucleic acid sequence, wherein the detection of telomerase nucleic acid sequences is indicative of a diagnosis of cancer.

42. The method of claim 41, further comprising comparing the amount of amplified telomerase sequence to a control, wherein increase telomerase nucleic acid sequences over the control is indicative of a diagnosis of cancer.

43. The method of claim 41, wherein the primers span region 1, region α, region β, region 2, region 3 region X or region Y, wherein the pattern of amplification is indicative of a diagnoses of cancer.

44. The method of claim 43, wherein the primers are Htel Intron T and Htel 723B.

45. The method of claim 44, wherein the primers are Htel335T and Hte11022B.

46. A method of determining a pattern of telomerase RNA expression in cells, comprising preparing cDNA from mRNA isolated from the cells, amplifying the cDNA using primers according to claim 35, therefrom determining the pattern of telomerase RNA expression.

47. The method of claim 46, further comprising detecting the amplified product by hybridization with an oligonucleotide having all or part of the sequence of region 1, region α, region β, region 2, region 3 region X or region Y.

48. A method of diagnosing cancer in a patient, comprising determining a pattern of telomerase RNA expression, comprising amplifying telomerase from cDNA synthesized from tumor RNA, and detecting the amplified product by hybridization with an oligonucleotide having all or part of the sequence of region 1, region α, region β, region 2, region 3 region X or region Y, therefrom determining the pattern of telomerase RNA expression, wherein the pattern is indicative of a diagnosis of cancer.

49. The method of claim 48, further comprising comparing the pattern to a pattern obtained from a reference cancer.

50. A non-human transgenic animal whose cells contain a vertebrate telomerase gene that is operably linked to a promoter effective for the expression of the gene.

51. The animal of claim 50, wherein the animal is a mouse.

52. The animal of claim 50, wherein the promoter is tissue-specific.

53. The animal of claim 50, wherein the telomerase gene is any of the nucleic acid sequences presented in Figure 11.

54. A mouse, whose cells have an endogenous telomerase gene disrupted by homologous recombination with a nonfunctional telomerase gene, wherein the mouse is unable to express endogenous telomerase .

55. An inhibitor of vertebrate telomerase activity, wherein the inhibitor binds to telomerase and is not a nucleoside analogue.

56. The inhibitor of claim 55, wherein the vertebrate is a human.

57. The inhibitor of claim 55, wherein the inhibitor is antisense nucleic acid complementary to human telomerase mRNA.

58. The inhibitor of claim 57, wherein the antisense is complementary to region α, region β, region 2, region 3 or region X.

59. The inhibitor of claim 55, wherein the inhibitor is a ribozyme.

60. A method of treating cancer, comprising administering to a patient a therapeutically effective amount of an inhibitor according to claim 55.

61. A nucleic acid molecule comprising the sequence selected from the set consisting of sequences selected from region 1, region α, region β, region 2 or region 3 as presented in Figure 10 and variants thereof.

62. A method of identifying an effector of telomerase activity comprising:
(a) adding a candidate effector to a mixture of telomerase protein, RNA component and template, wherein the telomerase protein is encoded by an isolated nucleic acid molecule according to claim 1;
(b) detecting telomerase activity; and
(c) comparing the amount of activity in step (b) to the amount of activity in a control mixture without candidate effector, therefrom identifying an effector.

63. The method of claim 62, wherein the effector is an inhibitor.

64. the method of claim 62, wherein the nucleic acid molecule encodes human telomerase.

65. An isolated nucleic acid molecule, wherein the nucleic acid molecule encodes one of the amino acid sequences presented in Figures 11A, 11B-C, 11D-E, 11J-K, 11L-M, 11N-O, 11P-Q, 11R-S, or 11T-U or encodes an altered amino acid sequence of the amino acid sequences presented in Figures 11A, 11B-C, 11D-E, 11J-K, 11L-M, 11N-O, 11P-Q, 11R-S or 11T-U wherein at least 90% of the amino acids have not been altered, and wherein the altered amino acid sequence binds telomerase RNA (hTR) or has telomerase activity.
